(19) Europäisches Patentamt
European Patent Office
Office européen des brevets

(11) EP 2 123 755 A1

(12) EUROPEAN PATENT APPLICATION
published in accordance with Art. 153(4) EPC

(43) Date of publication:
**25.11.2009 Bulletin 2009/48**

(21) Application number: **08710906.2**

(22) Date of filing: **07.02.2008**

(51) Int Cl.:
***C12N 15/09*** (2006.01) ***C12N 7/00*** (2006.01)
***C07K 14/08*** (2006.01) ***C07K 14/115*** (2006.01)

(86) International application number:
**PCT/JP2008/052016**

(87) International publication number:
**WO 2008/096811 (14.08.2008 Gazette 2008/33)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **07.02.2007 JP 2007027520**

(71) Applicant: **Dnavec Corporation**
**Tsukuba-shi, Ibaraki 305-0856 (JP)**

(72) Inventors:
- **YOSHIZAKI, Mariko**
**Tsukuba-shi**
**Ibaraki 305-0856 (JP)**
- **INOUE, Makoto**
**Tsukuba-shi**
**Ibaraki 305-0856 (JP)**
- **HASEGAWA, Mamoru**
**Tsukuba-shi**
**Ibaraki 305-0856 (JP)**

(74) Representative: **Vossius & Partner**
**Siebertstraße 4**
**81675 München (DE)**

# (54) ATTENUATED MINUS-STRANDED RNA VIRUS

(57) An objective of the present invention is to provide attenuated minus-strand RNA viruses. The present inventors discovered that the amino acid mutation at position 1214 (Y1214F) in the amino acid sequence of L protein of Sendai virus suppressed the viral genome replication activity and/or transcription activity. The inventors also found that the deletion of a particular gene from the viral genome could result in much less cytotoxicity and immune response than conventional. The inventors thus completed the present invention.

EP 2 123 755 A1

## Description

### Technical Field

The present invention relates to attenuated minus-strand RNA viruses.

### Background Art

The reverse genetics method developed in 1994 enabled *in vitro* production of viral molecules as infectious particles using cDNA of a virus carrying a minus-strand RNA genome. This technique has allowed arbitrary modification of viral cDNA. To date, several minus-strand non-segmented RNA viral vectors have been developed as gene transfer vectors (see Non-patent Document 1).

When viral vectors are applied to human, attenuation is an essential requirement. Methods for attenuating viruses are roughly divided into two types. The first method is to delete genes from the viral genome. For example, human metapneumovirus (HMPV) and respiratory syncytial virus (RSV) are causative viruses of respiratory diseases in infant patients. Children aged two or younger have very high risk of being infected with these viruses, and after infection they may develop severe bronchiolitis or pneumonia (see Non-patent Documents 2 and 3). Thus, there has been a need to develop pediatric vaccines. In this context, an HMPV vector lacking the SH and G genes, which are envelope genes, has been developed (see Non-patent Document 4). For RSV, which has similar constitutive genes, a live vaccine was also developed by deleting the SH gene(see Non-patent Document 5). Both of them were attenuated, and the proliferation of infectious particles was suppressed in the upper and lower trachea after administration of the vaccine vectors. Furthermore, since the purpose of these vectors was to induce immunity against the viruses *per se*, the administration of the vaccine vectors resulted in the production of neutralizing antibodies against them, and the protective effect against the wild type viruses. Regarding Sendai virus (SeV), there are reports on the deletion of the F gene (see Non-patent Document 6), M/F genes (see Non-patent Document 7), and M/F/HN genes (see Non-patent Document 8). The deletion of envelope-related genes has an advantage in that it renders the vectors non-transmissible. It is also effective in suppressing infectious SeV particles *in vivo* and weakening the elicitation of immune reaction. However, even when the vectors lack genes, deleted proteins are supplied to the vector particles in *trans* from production cells. The number of molecules is greatly reduced as compared to when the genes are on the genome and transcribed therefrom. Nevertheless, the method has its limitation in reducing the cytotoxicity and immune response.

The second method is identification of mutations that result in a phenotype showing reduced viral cytotoxicity. For example, in order to develop attenuated vaccines against parainfluenza virus, which also causes a human respiratory disease, the analysis of point mutations that reduce the activity of its RNA-dependent RNA polymerase (RdRp) has been greatly advanced, aiming at reducing all viral components in a balanced manner (see Non-patent Documents 9 to 11). Since the parainfluenza virus vector also has constitutive genes similar to those of RSV described above, many improved vaccines developed by utilizing the advantage that "point mutations can be transferred to related viruses", have been reported (see Non-patent Documents 12 and 13).

RNA viruses have a high mutation rate: the nucleotide substitution rate per cycle of their genome replication is as high as $10^{-5}$ to $10^{-3}$. Spontaneous generation of attenuated viruses is often observed during passaging viruses in *in vitro*-cultured cell systems or such. For example, there are some reports of identifying such attenuated viruses derived from human immunodeficiency virus (see Non-patent Documents 14 and 15), hepatitis A virus (see Non-patent Document 16), and Japanese encephalitis virus (see Non-patent Document 17) and proposing to utilize these as vaccine strains. Regarding Sendai virus, a number of persistent infectious viral strains have so far been identified and analyzed for their mutation sites and characteristics (see Non-patent Documents 18 to 24) (http://br.expasy.org/uniprot/P06447).

[Non-patent Document 1] Bukreyev, A., Skiadopoulos, M. H., Murphy, B. R., and Collins, P. L. (2006) Nonsegmented negative-strand viruses as vaccine vectors. J Virol 80, p.10293-10306.

[Non-patent Document 2] Bastien, N., Ward, D., Van Caeseele, P., Brandt, K., Lee, S. H., McNabb, G., Klisko, B., Chan, E., and Li, Y (2003) Human metapneumovirus infection in the Canadian population. J Clin Microbiol 41, p. 4642-4646.

[Non-patent Document 3] Boivin, G., Abed, Y, Pelletier, G., Ruel, L., Moisan, D., Cote, S., Peret, T. C., Erdman, D. D., and Anderson, L. J. (2002) Virological features and clinical manifestations associated with human metapneumovirus: a new paramyxovirus responsible for acute respiratory-tract infections in all age groups. J Infect Dis 186, p.1330-1334.

[Non-patent Document 4] Biacchesi, S., Skiadopoulos, M. H., Yang, L., Lamirande, E. W., Tran, K. C., Murphy, B. R., Collins, P. L., and Buchholz, U. J. (2004) Recombinant human Metapneumovirus lacking the small hydrophobic SH and/or attachment G glycoprotein: deletion of G yields a promising vaccine candidate. J Virol 78, p12877-12887.

[Non-patent Document 5] Whitehead, S. S., Bukreyev, A., Teng, M. N., Firestone, C. Y, St Claire, M., Elkins, W. R.,

Collins, P. L., and Murphy, B. R. (1999) Recombinant respiratory syncytial virus bearing a deletion of either the NS2 or SH gene is attenuated in chimpanzees. J Virol 73, p.3438-3442.

[Non-patent Document 6] Li, H. O., Zhu, Y F., Asakawa, M., Kuma, H., Hirata, T., Ueda, Y, Lee, Y S., Fukumura, M.. Iida, A., Kato, A., et al. (2000) A cytoplasmic RNA vector derived from nontransmissible Sendai virus with efficient gene transfer and expression. J Virol 74, p.6564-6569.

[Non-patent Document 7] Inoue, M., Tokusumi, Y, Ban, H., Shirakura, M., Kanaya, T., Yoshizaki, M., Hironaka, T., Nagai, Y, Iida, A., and Hasegawa, M. (2004) Recombinant Sendai virus vectors deleted in both the matrix and the fusion genes: efficient gene transfer with preferable properties. J Gene Med 6, p.1069-1081.

[Non-patent Document 8] Yoshizaki, M., Hironaka, T., Iwasaki, H., Ban, H., Tokusumi, Y, Iida, A., Nagai, Y, Hasegawa, M., and Inoue, M. (2006) Naked Sendai virus vector lacking all of the envelope-related genes: reduced cytopathogenicity and immunogenicity. J Gene Med 8, p.1151-1159.

[Non-patent Document 9] Skiadopoulos, M. H., Surman, S., Tatem, J. M., Paschalis, M., Wu, S. L., Udem, S. A., Durbin, A. P., Collins, P. L., and Murphy, B. R. (1999) Identification of mutations contributing to the temperature-sensitive, cold-adapted, and attenuation phenotypes of the live-attenuated cold-passage 45 (cp45) human parainfluenza virus 3 candidate vaccine. J Virol 73, p.1374-1381.

[Non-patent Document 10] Haller, A. A., MacPhail, M., Mitiku, M., and Tang, R. S. (2001) A single amino acid substitution in the viral polymerase creates a temperature-sensitive and attenuated recombinant bovine parainfluenza virus type 3. Virology 288, p.342-350.

[Non-patent Document 11] McAuliffe, J. M., Surman, S. R., Newman, J. T., Riggs, J. M., Collins, P. L., Murphy, B. R., and Skiadopoulos, M. H. (2004) Codon substitution mutations at two positions in the L polymerase protein of human parainfluenza virus type 1 yield viruses with a spectrum of attenuation in vivo and increased phenotypic stability in vitro. J Virol 78, p.2029-2036.

[Non-patent Document 12] Bartlett, E. J., Amaro-Carambot, E., Surman, S. R., Newman, J. T., Collins, P. L., Murphy, B. R., and Skiadopoulos, M. H. (2005) Human parainfluenza virus type I (HPIV1) vaccine candidates designed by reverse genetics are attenuated and efficacious in African green monkeys. Vaccine 23, p.4631-4646.

[Non-patent Document 13] Newman, J. T., Riggs, J. M., Surman, S. R., McAuliffe, J. M., Mulaikal, T. A., Collins, P. L., Murphy, B. R., and Skiadopoulos, M. H. (2004) Generation of recombinant human parainfluenza virus type 1 vaccine candidates by importation of temperature-sensitive and attenuating mutations from heterologous paramyxoviruses. J Virol 78, p.2017-2028.

[Non-patent Document 14] Fujita, K., Silver, J., and Peden, K. (1992) Changes in both gp120 and gp41 can account for increased growth potential and expanded host range of human immunodeficiency virus type 1. J Virol 66, p. 4445-4451.

[Non-patent Document 15] Sawyer, L. S., Wrin, M. T., Crawford-Miksza, L., Potts, B., Wu, Y, Weber, P. A., Alfonso, R. D., and Hanson, C. V. (1994) Neutralization sensitivity of human immunodeficiency virus type 1 is determined in part by the cell in which the virus is propagated. J Virol 68, p.1342-1349.

[Non-patent Document 16] Graff, J., Kasang, C., Normann, A., Pfisterer-Hunt, M., Feinstone, S. M., and Flehmig, B. (1994) Mutational events in consecutive passages of hepatitis A virus strain GBM during cell culture adaptation. Virology 204, p.60-68.

[Non-patent Document 17] Cao, J. X., Ni, H., Wills, M. R., Campbell, G. A., Sil, B. K., Ryman, K. D., Kitchen, I., and Barrett, A. D. (1995) Passage of Japanese encephalitis virus in HeLa cells results in attenuation of virulence in mice. J Gen Virol 76 (Pt 11), p.2757-2764.

[Non-patent Document 18] Nagata, I., Kimura, Y., Ito, Y., and Tanaka, T. (1972) Temperature-sensitive phenomenon of viral maturation observed in BHK cells persistently infected with HVJ. Virology 49, p.453-461.

[Non-patent Document 19] Yoshida, T., Nagai, Y., Maeno, K., Iinuma, M., Hamaguchi, M., Matsumoto, T., Nagayoshi, S., and Hoshino, M. (1979) Studies on the role of M protein in virus assembly using a ts mutant of HVJ (Sendai virus). Virology 92, p.139-154.

[Non-patent Document 20] Itoh, M., Isegawa, Y, Hotta, H., and Homma, M. (1997) Isolation of an avirulent mutant of Sendai virus with two amino acid mutations from a highly virulent field strain through adaptation to LLC-MK2 cells. J Gen Virol 78 (Pt 12), p.3207-3215.

[Non-patent Document 21] Adachi, A., Kanda, T., and Shibuta, H. (1980) Isolation and characterization of temperature-sensitive mutants of Sendai virus. Microbiol Immunol 24, p.1053-1068.

[Non-patent Document 22] Kanda, T., and Shibuta, H. (1982) Restricted viral RNA synthesis in establishment of persistent infection in Vero cells with a Sendai virus mutant. Microbiol Immunol 26, p.1045-1055.

[Non-patent Document 23] Nishio, M., Tsurudome, M., Ito, M., Kawano, M., Komada, H., and Ito, Y (2003) Characterization of Sendai virus persistently infected L929 cells and Sendai virus pi strain: recombinant Sendai viruses having Mpi protein shows lower cytotoxicity and are incapable of establishing persistent infection. Virology 314, p. 110-124.

[Non-patent Document 24] Nishio, M., Nagata, A., Tsurudome, M., Ito, M., Kawano, M., Komada, H., and Ito, Y (2004) Recombinant Sendai viruses with L1618V mutation in their L polymerase protein establish persistent infection, but not temperature sensitivity. Virology 329, p.289-301.

Disclosure of the Invention

[Problems to be Solved by the Invention]

An objective of the present invention is to provide attenuated minus-strand RNA viruses.

[Means for Solving the Problems]

The present inventors conducted dedicated studies to achieve the objective described above.

To develop most effective attenuated vectors, the present inventors considered utilizing mutations that significantly reduce the activity of Large (L) protein, as well as a gene deletion method that makes a virus non-transmissible through suppressing the production of particular viral components.

Thus, in the present invention, mutant Sendai virus (SeV) strains with markedly suppressed transcription/replication activity were cloned using as an indicator the persistent infectivity in an *in vitro* cultured cell system, and L gene mutations that significantly reduced the cytotoxicity were identified in the *in vitro* cultured cell system.

Cells infected with the M (matrix) gene-deficient SeV vector (SeV/ΔM) release few virus-like particles (VLPs; secondary particles) because the M gene has been removed from the genome of the vector. Accordingly, the use of the SeV/ΔM vector prevents reinfection with daughter vector particles released from the infected cells. Furthermore, the present inventors predicted that since SeV RNA accumulated in the cytoplasm, SeV or cells were likely to permit introduction of some mutations to avoid such accumulation. Utilizing this property of the vector, LLC-MK2 cells were infected with SeV/ΔM vector carrying the EGFP (enhanced green fluorescent protein) gene (SeV/ΔM-GFP) at a MOI of 3, and 74 clones of persistently infected cell lines were obtained. Daughter vectors were able to be harvested from the infected cytoplasm of 60 clones, and their HA activity was compared with that of the parental vector (SeV/ΔM-GFP). The result showed that 59 clones exhibited HA activity comparable to that of the parental vector. Meanwhile, one clone (clone #37) exhibited no detectable HA activity when cultured at 37°C. When cultured at 32°C, this clone showed HA activity almost equal to that of the parental vector. Since many of previously identified attenuated viruses have been reported to be temperature sensitive, this clone may also be temperature sensitive. Further characteristic analysis of this clone #37 revealed that the expression level of the EGFP gene was reduced and the cytotoxicity was also attenuated significantly. This finding suggested that clone #37 was a mutant strain that showed both temperature sensitivity and retardation of transcription/replication rate.

The nucleotide sequence of the whole genome of identified SeV/ΔM-GFP-clone#37 was determined by a modified Sanger method using dideoxy nucleotides. Each of the Sendai virus genes, NP, P, F, HN, and L, was compared with that of Z strain. The result showed that tyrosine at amino acid position 1214 in the L gene (6687 nucleotides; 2228 amino acids) was substituted with phenylalanine and that methionine at amino acid position 1602 in the L gene was substituted with leucine.

The L gene encodes a multifunctional enzyme that functions as RdRp. The activity of L includes capping enzyme activity, transcription activity, and replication activity. L localizes in the cytoplasm while binding to RNP of SeV. L is a giant protein of 200 kDa, 6684 bp, and has been reported to have six domains (Chandrika, R. et al., (1995) Virology 213, p. 352-363; Cortese, C. K. et al., (2000) Virology 277, p. 387-396; Feller, J. A. et al., (2000) Virology 269, p. 426-439; Horikami, S. M. and Moyer, S. A. (1995) Virology 211, p. 577-582; Smallwood, S. et al., (1999) Virology 262, p. 375-383; Smallwood, S. et al., (2002) Virology 304, p. 135-145; Smallwood, S. et al., (2002) Virology 304, p. 235-245). The amino acid mutation at position 1214 of the L gene (tyrosine to phenylalanine; Y1214F) is located in domain V.

Mutations can be classified into: (1) point mutation, (2) inversion, or (3) translocation. The most frequent type is point mutation, which includes substitution, deletion, and insertion. Deletion or insertion results in frameshift, leading to dysfunction of gene products. Replacements of amino acids caused by substitution are referred to as missense mutations. Meanwhile, mutations caused by substitution at the third codon position and resulting in any of the three types of stop codons (TAA, TAG, and TGA) are referred to as nonsense mutations. The mutations detected were only Y1214F and M1602L. No other point mutations adding or deleting a nucleotide were detected.

The codon of Y1214F was altered from tyrosine (TaT) to phenylalanine (TtT). In general, transversion (alteration that substitutes a pyrimidine nucleotide with a purine nucleotide or *vice versa*) is less frequent than transition (alteration of a pyrimidine or purine nucleotide to a different pyrimidine or purine nucleotide, respectively). Yoshitake *et al.* (Yoshitake, J. et al., (2004) J Virol 78, p8709-8719) infected CV-1 cells with Sendai virus carrying the EGFP gene (SeV-GFP) and examined the mutation rate after passage. The result showed that the transition from A to G spontaneously occurred in the GFP gene during the passage. Actually, of the 60 clones identified in the present invention, 17 clones had the same phenotype as the parental vector; however, point mutations were found in the F or HN gene and most of them were A-to-G or G-to-A transition at the first or second nucleotide of a codon.

Meanwhile, in order to clarify which of the two mutations Y1214F and M1602L in the L gene of clone #37 contributes to its altered phenotypes such as reduced expression of a carried gene, attenuated cytotoxicity, and temperature sensitivity, either or both of the two mutations were introduced by site-directed mutagenesis into the L gene of the F gene-deficient SeV vector (SeV/ΔF), which is nontransmissible and whose practical application to gene therapy or gene vaccine has been considered, to prepare three SeV vectors: SeV/ΔF-GFP-1214, SeV/ΔF-GFP-1602, and SeV/ΔF-GFP-1214-1602. Of the identified mutations, M1602L was predicted to make greater contribution in view of recent findings. Nishio, *et al*. (Nishio, M. et al., (2004) Virology 329, p. 289-301) reported that the mutation in which leucine at amino acid position 1618 in the L gene of Sendai virus was substituted with valine resulted in persistent infection and in attenuated cytotoxicity. They also reported that the virus became temperature sensitive when the 1169th amino acid was threonine, in addition to the point mutation at position 1618.

However, the vector with M1602L (SeV/ΔF-GFP-1602) exhibited the same cytotoxicity as the vector with the wild type L gene, and only 30% decrease in the mRNA transcription level. Although the genome replication activity differed with temperatures, it led to no phenotypic change. This result suggested that the point mutation necessary for the phenotypic change was Y1214F.

Meanwhile, the vectors with Y1214F (SeV/ΔF-GFP-1214 and SeV/ΔF-GFP-1214-1602) exhibited almost no cytotoxicity associated with vector infection, and showed persistent infectivity (Figs. 12 and 13). This finding was also proven by the result of real-time PCR for cDNA converted from cytoplasmic RNA purified from vector-infected cells (Fig. 10). The activity of mRNA transcription for SeV/ΔF-GFP-1214 was reduced to 13% at 32°C and 1.5% at 37°C as compared to SeV/ΔF-GFP. As a result, only seven copies of genome were present in each cell even 20 hours after infection. Furthermore, it was also found that the mutations identified in SeV/ΔM-GFP clone#37 were also functional when introduced into another deficient Sendai virus vector (SeV/ΔF) and its phenotype was attributed to Y1214F.

Mutation identification in genetics often finds substitution of Y with F. Tyrosine and phenylalanine are structurally analogous, with only a difference of hydroxyl group. In the field of signal transduction, there is a method of testing by substituting tyrosine, a substrate of tyrosine kinase, with phenylalanine (Yurchak, L. K. et al., (1996) J Biol Chem 271, p. 12549-12554). Assume that the hydroxyl group of tyrosine residue at position 1214 may interact with other amino acids or peptides. In that case, when the hydroxyl group involved in this interaction is eliminated by the substitution with phenylalanine, it would affect the tertiary structure of viral RdRp. As a result, it would reduce the activity of the polymerase for the transcription of L gene and the replication of L gene. This is a possible reason. The reason for the temperature sensitivity is presumed to be that the tertiary structure of L can be maintained at 32°C with tyrosine lacking the hydroxyl group (namely, phenylalanine). If there is a regulatory mechanism to elevate the activity of L by tyrosine kinase, the substitution ofY with F is presumed to have a great impact. To date, however, there is no such a report.

McAuliffe *et al*. (McAuliffe, J. M. et al. (2004) J Virol 78, p. 2029-2036) analyzed the stability of a tyrosine-to-histidine mutation at position 942 in the amino acids encoded by the L gene during cell passages, using recombinant human parainfluenza virus 1 (rHPIV 1). The result suggested that the mutation easily reverted to the original nucleotide (from cAC to tAC) after six passages. Based on this report, it is recommended that when point mutation is used for attenuating HPIV and RSV, missense mutation resulting from two or more nucleotide substitutions should be used (Murphy, B. R., and Collins, P. L. (2002) J Clin Invest 110, p. 21-27). However, the mutation identified in the present invention, Y1214F, was stable even after 12 passages, although it is a single-nucleotide substitution.

RNA-dependent RNA polymerase (RdRp) is a multifunctional protein. RdRp controls the proliferation of the virus as a factor controlling the enzymatic activity. Sue A. Moyer *et al*. have proposed to divide the L protein into six domains in terms of functions (Chandrika, R. et al. (1995) Virology 213, p. 352-363; Cortese, C. K. et al. (2000) Virology 277, p. 387-396; Feller, J. A. et al. (2000) Virology 269, p. 426-439; Horikami, S. M., and Moyer, S. A. (1995) Virology 211, p. 577-582; Smallwood, S. et al. (1999) Virology 262, p. 375-383; Smallwood, S. et al. (2002) Virology 304, p. 135-145; Smallwood, S. et al. (2002) Virology 304, p. 235-245). Characteristics and previously reported speculations on each domain are as follows:

domain I - composed of hydrophobic residues (Smallwood, S. et al. (1999) Virology 262, p. 375-383).
domain II - containing a putative RNA-binding motif (Malur, A. G. et al. (2002) Gene Expr 10, p. 93-100; Schnell, M. J., and Conzelmann, K. K. (1995) Virology 214, p. 522-530).
domain III - containing a well-conserved amino acid sequence, VQGDNQ, and predicted to be an RNA polymerase active portion and RNA-binding motif.
domain VI - containing six invariable proline residues as well as a conserved amino acid sequence, RNIGDP. This amino acid sequence is a purine-binding domain.
domain V - histidine and cysteine residues are conserved, and involved in metal binding;
domain VI - involved in purine nucleotide binding, and has a methylase activity (Ferron, F. et al., (2002) Trends Biochem Sci 27, p. 222-224).
Y1214F is located within domain V (1129aa - 1378aa). To date, the function of domain V still remains unclear in many points (Cortese, C. K. et al., (2000) Virology 277, p. 387-396). The present inventors therefore compared and examined the nucleotide and amino acid sequences of RdRp of respiroviruses, rubulaviruses, and morbilliviruses based on the information registered in Genbank (Table 1).

GenBank Accession No.

**[0021]** YP_138518 SeV (Sendai virus); AAL89409 HPIV 1 (Human parainfluenza virus 1); P12577 HPIV 3 (Human parainfluenza virus 3); P12576 MeV (Measles virus); BAA12219 BPIV 3 (Bovine parainfluenza virus 3); AAK54670 CDV (Canine distemper virus); NP_054714 MuV (Mumps virus); P11205 NDV (Newcastle disease virus); YP_138518 SV5 (Simian parainfluenza virus 5)

Table 1

| Genus | Virus (strain) | L protein | | | |
|---|---|---|---|---|---|
| | | Amino acid residue | | | |
| Respirovirus | SeV (present invention) | F | (1214) | L | (1602) |
| | SeV (Z) | Y | (1214) | M | (1602) |
| | HPIV 1 (Washington1964) | Y | (1214) | M | (1602) |
| | HPIV 3 (NIH 47885) | Y | (1214) | M | (1602) |
| | BPIV 3 (910N) | Y | (1214) | D | (1602) |
| Morbillivirus | MeV (Edomonston) | Y | (1212) | D | (1602) |
| | CDV (Onderstepoort) | Y | (1212) | E | (1602) |
| Rubulavirus | MuV (Miyahara) | Y | (1222) | D | (1614) |
| | NDV (BEAUDETTE c/45) | Y | (1192) | E | (1602) |
| | SV 5 (W3A) | Y | (1216) | D | (1604) |

As shown in Table 1, both of the amino acid sequences Y1214 and M1602 were well conserved among the ten viruses. In particular, Y1214 was found to be highly conserved. This information suggests that the phenotype caused by the mutation of Y1214 into phenylalanine is common or general. In other words, it is strongly suggested that, as in the L protein of Sendai virus, the introduction of Y1214F or an equivalent mutation into RdRp of other viruses could attenuate the viruses or reduce their RdRp activity.

For influenza and parainfluenza, which are clinically important, attenuated viruses are used to develop live vaccines. There are reports on mutations that result in reduced RdRp activity, like the Y1214F mutation identified in Sendai virus (Skiadopoulos, M. H. et al., (1999) J Virol 73, p. 1374-1381; Haller, A. A. et al., (2001) Virology 288, p. 342-350; McAuliffe, J. M. et al., (2004) J Virol 78, p. 2029-2036). The Y1214F mutation identified using Sendai virus in the present invention suppressed the RdRp activity to about 1/10, and was also stable even after passages. One could expect that other viruses for developing vaccines, such as parainfluenza, could be effectively attenuated by introducing Y1214F into them.

When the LacZ gene is used as a carried gene, β-gal staining and quantification of expressed β-gal protein can be performed. Thus, LLC-MK2 cells were infected with a SeV vector, and the time-course dynamics of the SeV vector was assayed by mRNA and protein levels of LacZ (Figs. 14, 15, and 16). The time course after infection was divided into early infection period (0 to 10 hours), mid infection period (10 to 22 hours), and late infection period (22 to 32 hours). The SeV vector used was $SeV^{18+}LacZ/\Delta F$, an F gene-deficient vector which carries the LacZ gene as well as the wild type L gene.

The mRNA synthesis rate of wild type L of the Sendai virus at 37°C in the early infection period was calculated to be 1.5 nucleotide/sec. This value is comparable to the rate of mRNA extension of L (1.7 nucleotide/sec) in the early infection period determined by Gubbay *et al.* (Gubbay, O. et al., (2001) J Gen Virol 82, p. 2895-2903). In the mid infection period (10 to 22 hours), the transcriptional activity of L increased rapidly. In association with this, the LacZ protein was found to be accumulated in the cytoplasm. The genome replication also started in this period. As shown in Fig. 15, the mRNA was decreased in the late infection period (22 to 32 hours), when the cytotoxicity began to appear. This was due to cell lysis caused by the strong cytotoxicity of SeV For $SeV^{18+}LacZ/\Delta F$-1214, which has Y1214F mutation, the rate of transcription elongation of L in the early infection period (0 to 10 hours after infection) was 0.3 nucleotide/sec, which was one fifth of the rate for $SeV^{18+}LacZ/\Delta F$. Furthermore, the genome replication started 22 hours after infection. As the number of genome molecules is increased by replication, the number of mRNA molecules transcribed is increased, which results in an increase in the numbers of L and P transcription factors. Thus, the number of LacZ mRNA molecules was linearly increased in the mid infection period, which was under the above conditions.

To compare the expression level of the carried gene, the gene expression level of type 5 adenoviral vector (Ad 5) was used as a control. The result showed that the activity of Y1214F-L was equivalent to or greater than the expression level of β-galactosidase derived from the adenoviral vector. This implies that the expression level of a carried gene, which is an important factor in gene therapy, was sufficiently maintained even though the mutation of L significantly reduced the viral antigenicity.

The result of quantitation of LacZ mRNA accumulated in cells at 32°C showed that LacZ mRNA accumulation was detectable ten hours after infection. The transcription rate for $SeV^{18+}LacZ$ /ΔF-1214 was 40% of the activity for $SeV^{18+}LacZ$/ΔF. However, the transcription rate of Y1214F-L was well maintained even in the late infection period (22 to 32 hours after infection) so that the accumulation rate kept its linearity well. Meanwhile, the mRNA accumulation by wild type L in the late infection period tended to decrease, as in the case of 37°C. This was also deemed as a result of the cytotoxicity. The expression level of the carried gene was hardly detectable in the early infection period, while the intracellular accumulation was observed in the mid infection period. In the late infection period, the levels of LacZ mRNA and protein reached 80% of those of the vector having wild type L. The possible reason for this was that the cytotoxicity was suppressed and did not cause cell lysis even at 32°C. LacZ staining also revealed that the expression level of LacZ protein at 32°C, which is a temperature acceptable to the mutant, was comparable to that of the vector having wild type L (Fig. 16). When a Sendai virus vector lacking all envelope genes was introduced, the *in vivo* expression of the carried gene lasted several days and then rapidly abolished due to the cytotoxicity and strong immune response (Yoshizaki, M., Hironaka, T., Iwasaki, H., Ban, H., Tokusumi, Y., Iida, A., Nagai, Y, Hasegawa, M., and Inoue, M. (2006) Naked Sendai virus vector lacking all of the envelope-related genes: reduced cytopathogenicity and immunogenicity. J Gene Med 8, p. 1151-1159). This suggests that there is a limitation in the improvement based on deleting genes from the genome. Gene deletion can be expected to improve SeV to some extent. Furthermore, attenuation by the combination of gene deletion and regulation of L activity is promising. The strong toxicity of Sendai virus is also attenuated *in vivo.* Such viruses can be expected to be remarkably superior in expressing carried genes and avoiding immune responses.

The LacZ protein used was a recombinant β-galactosidase. The number of β-galactosidase molecule in the control was calculated from its molecular weight and mass. According to the calibration curve, the levels of the LacZ gene expression with the SeV vectors and the Ad 5 vector were determined based on the amounts of LacZ protein. The amount of LacZ was 6 pg/cell for the wild type L-SeV vector ($SeV^{18+}LacZ$/ΔF), 1.5 to 4 pg/cell for the L-SeV vector having Y1214F mutation ($SeV^{18+}LacZ$/ΔF-1214), and 0.3 pg/cell for the Ad vector. For example, when the immunization of mice to prepare a polyclonal or monoclonal antibody is considered as a model system, 5 to 50 μg of an antigen is required for the production of a polyclonal or monoclonal antibody in a mouse (Harlow, E., 1998, Antibodies, chapter 6, p. 152). Since the amount of antigen expressed with $SeV^{18+}LacZ$/ΔF-1214 is 1.5 to 4 pg/cell, a required amount of antigen could be provided in the body of the immunized mouse if the gene is introduced into 3 x $10^6$ cells. Furthermore, unlike a purified recombinant antigen which contains a denatured antigen, SeV enables immunization with an antigen that has been produced in cells in its native conformation. When a therapeutic gene product is supplied, SeV also enables to efficiently produce a protein having its native conformation. This value also serves as a guide to set a target value for the expression capability of a vector applied to human. It is suggested that the introduction of the point mutation Y1214F that results in reduction of the activity of L is promising at least in pre-clinical studies using mice where applications such as vaccination are envisioned.

Sendai virus vectors are characterized by the ability to allow high level gene expression (Tokusumi, T. et al., (2002) Virus Res 86, p. 33-38). In the development of attenuated virus vectors, there is a concern that alterations in the viral genome may reduce not only the cytotoxicity but also the expression level of carried genes. From the practical aspect, it is important to maximally reduce the level of SeV antigen and the amount of viral RNA that is targeted by the natural immune response of cells, while maintaining the minimum required gene expression level. When considering this aspect, although Y1214F reduced the activity of RdRp, it showed the expression of a carried gene that was equal to or more than that of adenovirus, and at the same time reduced the amounts of SeV antigen and viral RNA by lowering the activity of L. The present invention has demonstrated that the introduction of Y1214F mutation into the L gene, in combination with deletion of structural protein genes (for example, F gene deletion, M/F gene deletion, and M/F/HN gene deletion) from SeV vectors, enables to produce effective viral vectors not only in gene therapy and vaccine development but also in the development of expression vectors used in basic research. Furthermore, since the amino acid sequences of RdRp have high similarity, the point mutation Y1214F in L of Sendai virus, which is characterized herein, has generality and stability and hence is also applicable to the development of vaccines for parainfluenza virus and such.

Specifically, the present invention relates to attenuated minus-strand RNA viruses, and more specifically to:

[1] an attenuated minus-strand RNA virus, comprising a gene encoding a mutant L protein in which a wild-type amino acid at a position corresponding to position 1214 in the amino acid sequence of SEQ ID NO: 1 (wild type L protein of SeV) has been substituted with another;

[2] the minus-strand RNA virus of [1], wherein the attenuation is reduction in genome replication activity and/or transcription activity;

[3] the minus-strand RNA virus of [1] or [2], wherein the substitution is of tyrosine with phenylalanine;
[4] the minus-strand RNA virus of any one of [1] to [3], in which at least one or more of the genes encoding envelope-constituting proteins is deleted or inactivated;
[5] the minus-strand RNA virus of [4], wherein the deleted or inactivated gene is any one of, or a combination of two or more of, the genes encoding F, HN, and M proteins;
[6] the minus-strand RNA virus of any one of [1] to [5], which is a Paramyxoviridae virus;
[7] the minus-strand RNA virus of [6], wherein the Paramyxoviridae virus is Sendai virus;
[8] a viral vector comprising the minus-strand RNA virus of any one of [1] to [7];
[9] the viral vector of [8], comprising a foreign gene in an expressible manner;
[10] a method for attenuating a minus-strand RNA virus by introducing a mutation to substitute an amino acid at a position corresponding to position 1214 in the amino acid sequence of SEQ ID NO: 1, in a gene encoding L protein of the minus-strand RNA virus;
[11] the method of [10], wherein the attenuation is a decrease in the genome replication activity and/or transcription activity;
[12] the method of [10] or [11], which is performed to reduce the cytotoxicity to the cell introduced with the virus;
[13] the method of [10] or [11], which is performed to improve the persistency of foreign gene expression;
[14] the method of [10] or [11], which is performed to reduce immune response;
[15] the method of any one of [10] to [14], wherein the substitution is of tyrosine with phenylalanine;
[16] the method of any one of [10] to [15], in which at least one or more of the genes encoding envelope-constituting proteins in the minus-strand RNA virus are deleted or inactivated, or which comprises the step of deleting or inactivating at least one or more of the genes encoding envelope-constituting proteins in the minus-strand RNA virus;
[17] the method of [16], wherein the deleted or inactivated gene, or gene to be deleted or inactivated is any one of the genes encoding F, HN, and M proteins, or a combination of two or more of them;
[18] the method of any one of [10] to [17], wherein the minus-strand RNA virus is a Paramyxoviridae virus; and
[19] the method of [18], wherein the Paramyxoviridae virus is Sendai virus.
Furthermore, the present invention also relates to:
[20] a method for producing an attenuated minus-strand RNA virus, which comprises the step of introducing a mutation in the gene encoding L protein of the minus-strand RNA virus to substitute an amino acid at a position corresponding to position 1214 in the amino acid sequence of SEQ ID NO: 1;
[21] the method of [20], wherein the attenuation is a decrease in the genome replication activity and/or transcription activity;
[22] the method of any one of [20] or [21], wherein the substitution is of tyrosine with phenylalanine;
[23] the method of any one of [20] to [22], in which at least one or more of the genes encoding envelope-constituting proteins in the minus-strand RNA virus are deleted or inactivated, or which comprises the step of deleting or inactivating at least one or more of the genes encoding envelope-constituting proteins in the minus-strand RNA virus;
[24] the method of [23], wherein the deleted or inactivated gene, or gene to be deleted or inactivated is any one of the genes encoding F, HN, and M proteins, or a combination of two or more of them;
[25] the method of any one of [20] to [24], wherein the minus-strand RNA virus is a Paramyxoviridae virus; and
[26] the method of [25], wherein the Paramyxoviridae virus is Sendai virus.

Brief Description of the Drawings

Fig. 1 is a set of photographs showing the expression of GFP in LLC-MK2 cells after infection with the SeV/ΔM-GFP vector (MOI of 3).
Fig. 2 is a set of photographs showing the expression of GFP in CV-1 cells after infection with the SeV/ΔM-GFP vector (MOI of 3).
Fig. 3 is a set of diagrams showing the expression level of GFP in LLC-MK2 cells after infection with the SeV/ΔM-GFP vector (MOI of 1).
Fig. 4 is a set of diagrams showing the mutation sites possessed by cells persistently infected with the SeV/ ΔM-GFP vector.
Fig. 5 is a set of graphs showing the cytotoxicity for CV-1 cells after infection with SeV/ΔF-GFP, SeV/ΔF-GFP-1602, SeV/ΔF-GFP-1214, or SeV/ΔF-GFP-1214-1602 vector.
Fig. 6 is a set of photographs showing morphologies of CV-1 cells after infection with SeV/ΔF-GFP, SeV/ΔF-GFP-1602, SeV/ΔF-GFP-1214, or SeV/ΔF-GFP-1214-1602 vector.
Fig. 7 is a set of graphs showing the vector production ability in LLC-MK2/F7 cells after infection with SeV/ΔF-GFP, SeV/ΔF-GFP-1602, SeV/ΔF-GFP-1214, or SeV/ΔF-GFP-1214-1602 vector.

Fig. 8 is a set of photographs showing the expression of GFP in bone marrow-derived mesenchymal cells of C57BL/6 mice after infection with SeV/ΔF-GFP or SeV/ΔF-GFP-1214 vector (on day 7 after infection; MOI of 100).

Fig. 9 is a set of photographs showing the expression of GFP in mesenchymal cells derived from bone marrow of C57BL/6 mice after infection with SeV/ΔF-GFP or SeV/ΔF-GFP-1214 vector (on day 14 after infection; at a MOI of 100).

Fig. 10 is a set of graphs showing the expression levels of genome RNA, antigenomic RNA, and mRNA in LLC-MK2 cells after infection with SeV/ΔF-GFP(Y1214F), SeV/ΔF-GFP(M1602), or SeV/ΔF-GFP vector.

Fig. 11 is a graph showing the growth rate of CV-1 cells after infection with SeV/ΔF-GFP or SeV/ΔF-GFP-1214 vector.

Fig. 12 is a graph showing the growth rate of LLC-MK2 cells after infection with SeV/ΔF-GFP or SeV/ΔF-GFP-1214 vector.

Fig. 13 is a set of photographs showing the expression level of GFP in LLC-MK2 cells after five passages following infection with SeV/ΔF-GFP-1214 vector.

Fig. 14 is a set of graphs showing expression dynamics of the carried gene (LacZ) in LLC-MK2 cells after infection with $SeV^{18+}$LacZ/ΔF, $SeV^{18+}$LacZ/ΔF-1214, or Adeno-LacZ vector.

Fig.15(A) shows the copy numbers of LacZ mRNA and SeV genome in LLC-MK2 cells (cultured at 37°C) after infection with $SeV^{18+}$LacZ/ΔF or $SeV^{18+}$LacZ/ΔF-1214 vector. An enlarged graph is shown at the lower left for more details of the mRNA copy number between 1 to 10 hours after infection. (B) shows LacZ staining of LLC-MK2 cells (cultured at 37°C) after vector infection.

Fig. 16(A) shows the copy numbers of LacZ mRNA and SeV genome in LLC-MK2 cells (cultured at 32°C) after infection with $SeV^{18+}$LacZ/ΔF or $SeV^{18+}$LacZ/ΔF-1214 vector. An enlarged graph is shown at the lower left for more details of the mRNA copy number between 1 to 10 hours after infection. (B) shows LacZ staining of LLC-MK2 cells (cultured at 32°C) after vector infection.

## Best Mode for Carrying Out the Invention

The present invention provides attenuated minus-strand RNA viruses. Specifically, the present invention relates to attenuated minus-strand RNA viruses having a gene encoding a mutant L protein in which a wild-type amino acid at a position corresponding to position 1214 in the amino acid sequence of the wild-type L protein of Sendai virus is substituted with another (hereinafter may be referred to as "attenuated minus-strand RNA viruses").

The present inventors have for the first time discovered that Sendai virus become attenuated when an amino acid at position 1214 in the amino acid sequence of wild type L protein of Sendai virus is substituted as compared to the wild type. For example, as shown in Examples herein, it was demonstrated that the Sendai virus having Y1214F (SeV/ΔF-GFP-1214) had reduced mRNA transcription activity as compared to Sendai virus without the amino acid substitution, and that there were only seven copies of genome per cell even 20 hours after infection. A decrease in the number of genome molecules due to reduced genome replication will result in a decrease in the number of mRNA transcribed. Thus, in the present invention, the "attenuation" means reduction in the genome replication activity and/or transcription activity of the minus-strand RNA virus.

The amino acid sequence of wild type L protein of Sendai virus is shown in SEQ ID NO: 1, and the nucleotide sequence encoding the amino acid sequence is shown in SEQ ID NO: 2.

In the present invention, the "corresponding position" refers to a homologous position in the L protein, and specifically refers to an amino acid position that falls on the same position when aligned with the amino acid sequence of SEQ ID NO: 1. The L proteins of minus-strand RNA viruses are highly conserved and the positions corresponding to position 1214 of SEQ ID NO: 1 can be identified by aligning the respective amino acid sequences with known methods. The amino acid sequences can be easily aligned by, for example, using BLAST (Karlin S, Altschul SF, Proc. Natl. Acad. Sci. USA, 87: 2264-2268, 1990; Karlin S, Altschul SF, Proc. Natl. Acad Sci. USA, 90: 5873-5877, 99993; Altschul SF, et al., J. Mol. Biol., 215: 403, 1990), CLUSTAL W (Thompson JD, et al., Nucleic Acids Res 22:4673-4680, 1994), or the like. Examples of positions corresponding to position 1214 of SEQ ID NO: 1 are shown below together with GenBank accession numbers.

· position 1214 in the amino acid sequence of YP_138518 SeV (Sendai virus)
· position 1214 in the amino acid sequence of AAL89409 HPIV 1 (human parainfluenza virus 1)
· position 1214 in the amino acid sequence of P12577 HPIV 3 (human parainfluenza virus 3)
· position 1212 in the amino acid sequence of P12576 MeV (mink enteritis virus)
· position 1214 in the amino acid sequence of BAA12219 BPIV 3 (bovine parainfluenza virus 3)
· position 1212 in the amino acid sequence of AAK54670 CDV (canine distemper virus)
· position 1222 in the amino acid sequence of NP_054714 MuV (mumps virus)
· position 1192 in the amino acid sequence of P 11205 NDV (Newcastle disease virus)
· position 1216 in the amino acid sequence of YP_138518 SV5 (simian parainfluenza virus 5)

In the present invention, the "minus-strand RNA virus" refers to a virus that contains a minus strand (an antisense strand of a sense strand encoding viral proteins) RNA as its genome. The minus-strand RNA is also referred to as negative strand RNA. The minus-strand RNA virus used in the present invention particularly includes single-stranded minus-strand RNA viruses (also referred to as non-segmented minus-strand RNA viruses). The "single-stranded negative strand RNA virus" refers to a virus having a single-stranded negative strand (i.e., a minus strand) RNA as its genome.

The minus-strand RNA virus described above includes viruses belonging to Paramyxoviridae (including *Paramyxovirus, Morbillivirus, Rubulavirus,* and *Pneumovirus*), Rhabdoviridae (including *Vesiculovirus, Lyssavirus,* and *Ephemerovirus*), Filoviridae including Ebola virus, Orthomyxoviridae (including Influenza viruses A, B, and C, and Thogoto-like viruses), Bunyaviridae (including *Bunyavirus, Hantavirus, Nairovirus,* and *Phlebovirus*), and Arenaviridae.

Specific examples of the minus-strand RNA viruses used in the present invention include Sendai virus, Newcastle disease virus, mumps virus, measles virus, respiratory syncytial virus (RS virus), rinderpest virus, distemper virus, simian parainfluenza virus (SV5), and human parainfluenza viruses 1, 2, and 3, which belong to Paramyxoviridae; influenza virus, which belongs to Orthomyxoviridae; vesicular stomatitis virus and rabies virus, which belong to Rhabdoviridae; and Ebola virus, which belongs to Filoviridae. Incomplete viruses such as DI particles (J. Virol. 68, 8413-8417 (1994)), synthetic oligonucleotides, and the like may also be used.

In the present invention, the minus-strand RNA virus is preferably a Paramyxoviridae virus, more preferably a Paramyxovirinae virus, and most preferably a virus of the genus *Respirovirus* (also referred to as *Paramyxovirus*).

Furthermore, in the present invention, the preferred substitution of the amino acid at a position corresponding to position 1214 in the amino acid sequence of SEQ ID NO: 1 is substitution of tyrosine with phenylalanine (point mutation). Minus-strand RNA viruses in which the amino acid at a position corresponding to position 1214 in the amino acid sequence of SEQ ID NO: 1 is tyrosine include, for example, human parainfluenza virus (HPIV) and bovine parainfluenza virus (BPIV) belonging to *Respirovirus*; mink enteritis virus (MeV) and canine distemper virus (CDV) belonging to *Morbillivirus*; and mumps virus (MuV), Newcastle disease virus (NDV), and simian parainfluenza virus (SV5) belonging to *Rubulavirus.*

In the present invention, as long as tyrosine is substituted with phenylalanine, the number and position of substitutions in the nucleotides encoding the amino acid are not particular limited. Preferably, one or more transversion mutations (alterations that substitute a pyrimidine nucleotide with a purine nucleotide or *vice versa*) are involved. Substitution involving two or more nucleotide mutations is also preferred. In this case, it is preferred that at least one of them is a transversion mutation. In an embodiment, the substitution includes, for example, substitution of the nucleotides of tyrosine (TaT) with phenylalanine (TtT). Furthermore, the gene encoding a mutant L protein in which the wild type amino acid has been substituted include those having amino acid mutations at other positions. For example, the gene may have a mutation at a position corresponding to position 1602 in addition to a position corresponding to position 1214. Furthermore, a minus-strand RNA virus having the gene encoding a mutant L protein may contain mutations and/or deletions in other genes, or may have additional genes.

Mutations can be introduced by known methods of site-directed mutagenesis, for example, PCR methods and cassette mutagenesis methods (Deng, W. P. & Nickoloff, J. A., Anal. Biochem. 200:81, 1992; Haught, C., et al., BioTechniques 16(1):47-48, 1994; Zhu, L. and Holtz, A., Methods Mol. Biol. 57:119-137, 1996; Zhu, L., Methods Mol. Biol. 57:13-29, 1995; GeneEditor™ System, Altered Sites(R) II System, Promega Co. WI, USA; KOD - Plus-Mutagenesis Kit, Toyobo Co., Ltd. Osaka, Japan; Transformer Site-Directed Mutagenesis Kit, Takara Bio Inc., Otsu, Japan). For example, genomic cDNA of minus-strand RNA virus is prepared and the L gene is subcloned. A mutation is introduced into the resulting L gene in the codon encoding an amino acid at a position corresponding to position 1214, and thereby a nucleic acid encoding the mutant L protein in which the wild type amino acid at a position corresponding to position 1214 in the amino acid sequence of SEQ ID NO: 1 is substituted with another, and vectors carrying the nucleic acid, can be obtained. By expressing this nucleic acid, the mutant L protein in which the wild type amino acid at a position corresponding to position 1214 in the amino acid sequence of SEQ ID NO: 1 is substituted can be produced. A viral genome cDNA that has a mutation in the gene encoding the L protein can be obtained by replacing the L gene portion in the genomic cDNA of the minus-strand RNA virus with the obtained nucleic acid encoding the mutant L protein. This cDNA can be used to prepare attenuated minus-strand RNA viruses of the present invention.

The attenuated minus-strand RNA viruses of the present invention include minus-strand RNA viruses with the above-described mutations introduced into only the L gene of a natural virus. Pathogenic viruses can be attenuated by introducing mutations into the coding region for the amino acid at position 1214 in their L genes. It is also preferred that at least one or more of the genes encoding viral proteins constituting the envelope (envelope-constituting proteins) are deleted or inactivated in the attenuated minus-strand RNA viruses of the present invention. When vectors to be used in gene therapy or such are prepared, vectors that lack the ability to replicate infectious particles can be obtained by deleting genes encoding envelope-constituting proteins.

In the present invention, the "envelope-constituting proteins" include spike and envelope-lining proteins constituting the viral envelope. Specifically, such proteins include fusion (F), hemagglutinin (H), hemagglutinin (HA), hemagglutinin-neuraminidase (HN), glycoprotein (G), matrix (M), and matrix 1 (M1), which vary depending on the type of virus.

The envelope-constituting proteins in the present invention include not only the F, H, HN, G, M, and M1 proteins listed above but also proteins corresponding to the above envelope-constituting proteins in other minus-strand RNA viruses, even if their names are different from those listed above.

For example, in paramyxoviruses, M (matrix), F (fusion), and HN (hemagglutinin-neuraminidase) (or H (hemagglutinin)) genes are known as envelope-constituting genes. In Paramyxovirinae viruses, the respective genes are commonly listed as follows.

*Respirovirus* M F HN
*Rubulavirus* M F HN
*Morbillivirus* M F H

For example, the database accession numbers for the nucleotide sequences of each gene of Sendai virus, which is classified into the genus *Respirovirus* (also referred to as *Paramyxovirus*) of Paramyxoviridae, are D11446, K02742, M30202, M30203, M30204, M69046, U31956, X00584, and X53056 for M gene; D00152, D11446, D17334, D17335, M30202, M30203, M30204, M69046, X00152, and X02131 for F gene; and D26475, M12397, M30202, M30203, M30204, M69046, X00586, X02808, and X56131 for HN gene.

Examples of viral genes encoded by other viruses are as follows: CDV, M12669; DMV, Z30087; HPIV-1, S38067; HPIV-2, M62734; HPIV-3, D00130; HPIV-4a, D10241; HPIV-4b, D10242; Mumps, D86171; MV, AB012948; NDV, AF089819; PDPR, Z47977; PDV, X75717; RPV, M34018; SeV, U31956; and SV5, M32248 for M gene; CDV, M21849; DMV, AJ224704; HPN-1, M22347; HPIV-2, M60182; HPIV-3, X05303, HPIV-4a, D49821; HPIV-4b, D49822; Mumps, D86169; MV, AB003178; NDV, AF048763; PDPR, Z37017; PDV, AJ224706; RPV, M21514; SeV, D17334; and SV5, AB021962 for F gene; CDV, AF112189; DMV, AJ224705; HPIV-1, U709498; HPIV-2. D000865; HPIV-3, AB012132; HPIV-4A, M34033; HPIV-4B, AB006954; Mumps, X99040; MV, K01711; NDV, AF204872; PDPR, X74443; PDV, Z36979; RPV, AF132934; SeV, U06433; and SV-5, S76876 for NH (H or G) gene; and CDV, AF014953; DMV, AJ608288; HPIV-1, AF117818; SPIV-2, X57559; HPIV-3, AB012132; Mumps, AB040874; MV, K01711; NDV, AY049766; PDPR, AJ849636; PDV, Y09630; RPV,Z30698; and SV-5, D13868 for L gene.

However, since multiple strains for each virus are known, there are also genes composed of sequences other than those cited above as a result of strain variation.

In the attenuated minus-strand RNA viruses of the present invention, it is preferred that any one of or two or more of the genes encoding F, HN, and M proteins described above are deleted or inactivated.

For example, an attenuated minus-strand RNA virus in which the gene encoding M protein is deleted or inactivated has not only the characteristics of being attenuated according to the present invention but also the characteristics of not causing reinfection with daughter viruses, since no viral particles are released due to the loss of production of the M protein, which is essential for the particle formation after viral infection.

An attenuated minus-strand RNA virus in which the gene encoding F protein is deleted or inactivated not only has the characteristics of being attenuated according to the present invention but also becomes nontransmissible and thus secures safety.

An attenuated minus-strand RNA virus in which the gene encoding HN protein is deleted or inactivated has not only the characteristics of being attenuated according to the present invention but also the characteristics of preventing release of newly formed viral particles from the infected cells, since the activity of cleaving sugar chains on the cell surface at the sialic acid portion is inhibited.

In the present invention, there is no limitation on combinations of the genes encoding F, HN, and M proteins.

Furthermore, the present invention provides vectors comprising an attenuated minus-strand RNA virus of the present invention (hereinafter may be referred to as "vectors of the present invention).

The vectors of the present invention are viral particles that comprise a viral genome derived from an attenuated minus-strand RNA virus, lack the self-replication ability, and have the ability to introduce nucleic acid molecules into hosts. Specifically, they refer to vehicles based on an attenuated minus-strand RNA virus, which are carriers used to introduce nucleic acids into cells or hosts, and mean that the vectors have the backbone of an attenuated minus-strand RNA virus. The phrase "having the backbone of an attenuated minus-strand RNA virus" means that the nucleic acid molecule in the viral particle constituting the vector is based on the genome of the attenuated minus-strand RNA virus. For example, vectors in which the nucleic acid molecule contained in the viral particles has a packaging signal sequence derived from an attenuated minus-strand RNA virus genome are included in the vectors of the present invention. The vectors of the present invention also include viral vectors constructed using genetic recombination techniques. Viral vectors constructed using DNA encoding the viral genome and packaging cells are recombinant viral vectors included in the vectors of the present invention.

The vectors of the present invention include, for example, vectors in which the minus-strand RNA virus is Sendai virus, namely, Sendai virus vectors. Sendai virus vectors are characterized by the ability of high-level gene expression. Thus, while Y1214F reduces the activity of RdRp, Sendai virus vectors allow foreign genes (herein, also referred to as "carried genes") to be expressed at a level equivalent to or greater than that with adenoviruses. Y1214F also reduces the levels of Sendai virus antigens and viral RNA by reducing the activity of L protein. The present invention demonstrated that the introduction of the Y1214F mutation into the L gene, in combination with deletion of structural protein genes (for example, F gene deletion, M/F gene deletion, and M/F/HN gene deletion) from Sendai virus vectors, enables to produce effective viral vectors not only for gene therapy and vaccine development but also in the development of expression vectors used in basic research.

Thus, the deletion can be expected to improve Sendai virus to some extent. Furthermore, attenuation by the combination of gene deletion with regulation of the L activity is promising. It also attenuates the strong toxicity of Sendai virus *in vivo,* and is expected to be remarkably advantageous in expressing a foreign gene and avoiding immune responses.

Furthermore, the vectors of the present invention may carry a foreign gene in a manner allowing its expression. The foreign gene carried by the vectors of the present invention is not particularly limited, and any gene desired to be expressed in target cells can be used. For example, the foreign gene may be a nucleic acid encoding a protein, or alternatively, a nucleic acid that does not encode any protein, such as an antisense or ribozyme. The gene may be a naturally-derived sequence or an artificially-designed sequence. Artificial proteins may be, for example, fusion proteins with other proteins, dominant negative proteins (including soluble receptor molecules and membrane-bound dominant negative receptors), truncated forms of cell adhesion molecules, and soluble forms of cell surface molecules.

Furthermore, in the present invention, the foreign gene may be a marker gene to assess the gene transfer efficiency, expression stability, or the like. Such marker genes include, for example, the genes encoding green fluorescent protein (hereinafter also referred to as GFP), β-galactosidase, and luciferase.

Alternatively, when the purpose for the foreign gene of the present invention is gene therapy or such, a therapeutic gene for a target disease is inserted in the vectors of the present invention. When a foreign gene is inserted, for example, into a Sendai virus vector, it is necessary to insert the sequence between the transcription start sequence (S) and termination sequence (E) in such a way that the total number of genomic nucleotides becomes a multiple of six (Journal of Virology, Vol.67, No.8, 1993, p. 4822-4830). An E-I-S sequence (transcription termination sequence-intervening sequence-transcription initiation sequence) or portion thereof is inserted before or after the foreign gene if necessary so as not to interfere with expression of the genes before or after the foreign gene. The expression level of the inserted foreign gene can be regulated by the type of transcription initiation sequence added upstream of the foreign gene (WO 01/18223). It can also be regulated by the site of gene insertion and nucleotide sequences before and after the gene. For example, in Sendai virus, the closer the insertion site is to the 3'-end of negative-strand RNA (to the NP gene in the gene arrangement on the wild-type viral genome), the higher the expression level of the inserted gene is. To achieve a high expression level of a foreign gene, it is preferable to insert the foreign gene into an upstream region in the negative-strand genome (the 3'-side in the minus-strand). Conversely, the closer the insertion position is to the 5'-end of negative-strand RNA (to the L gene in the gene arrangement on the wild-type viral genome), the lower the expression level of the inserted gene is. To suppress the expression of a foreign gene to a low level, the foreign gene is inserted, for example, to the far most 5'-side of the negative-strand, that is, downstream of the L gene in the wild-type viral genome (the 5'- adjacent site of the L gene in the negative-strand) or upstream of the L gene (the 3'-adjacent site of the L gene in the negative-strand). To facilitate the insertion of a foreign gene, a cloning site may be designed at the position of insertion. The cloning site may be, for example, a recognition sequence for a restriction enzyme. Foreign gene fragments can be inserted into the restriction enzyme site in the vector DNA encoding the genome. The cloning site may be a so-called multi-cloning site having a plurality of restriction enzyme recognition sequences. The vectors of the present invention may thus carry additional foreign genes.

Recombinant minus-strand RNA viruses may be reconstituted using known methods. For example, such vectors can be produced by the steps of (a) transcribing DNA which encodes the genomic RNA of a minus-strand RNA virus or the complementary strand thereof (antigenomic RNA, plus-strand), in mammalian cells in the presence of viral proteins constituting RNP containing the genomic RNA of the minus-strand RNA virus, and (b) collecting the produced minus-strand RNA viruses or RNP containing the genomic RNA. The "viral proteins constituting RNP" mentioned above refers to proteins that form RNP together with the viral genomic RNA and constitute a nucleocapsid. These are a group of proteins necessary for genome replication and gene expression, and are typically N (nucleocapsid (also referred to as nucleoprotein (NP))-, P (phospho)-, and L (large)-proteins. Although these notations vary depending on viral species, corresponding proteins are known to those skilled in the art (Anjeanette Robert et al., Virology 247:1-6 (1998)). For example, "N" may be denoted as "NP".

When reconstituting viruses, a minus-strand RNA genome (i. e. antisense strand, which is the same as the viral genome) or the plus-strand RNA (antigenome, the complementary strand of the genomic RNA) may be generated as described above. However, in order to increase the efficiency of virus reconstitution, the plus-strand is preferably generated. Viral genomic RNA that encodes viral proteins required for RNP reconstitution can be replicated in infected cells even if it lacks genes encoding envelope-constituting proteins. Specifically, when the genomic RNA encodes the N, P, and L proteins, it is constructed so as not to encode viral proteins such as F, HN, and M. Such defective viruses can amplify the genomic RNA in cells, but do not release infectious virions, and thus are useful as highly safe gene transfer vectors (WO00/70055, WO00/70070, and WO03/025570; Li, H.-O. et al., J. Virol. 74(14) 6564-6569 (2000)). To produce a recombinant virus, the above envelope-constituting proteins are expressed separately in virus-producing cells to complement particle formation. In order to express viral proteins and RNA genome in cells, an expression vector linked with DNA that encodes the proteins or genome downstream of an appropriate promoter is introduced into host cells. The promoter used include, for example, CMV promoters and CAG promoters (Niwa, H. et al. (1991) Gene. 108: 193-199, and Japanese Patent Application Kokai Publication No. (JP-A) H3-168087 (unexamined, published Japanese patent application)).

The RNA terminals preferably reflect the terminals of the 3'-leader sequence and 5'-trailer sequence as accurately as possible, as in the natural viral genome. For example, a self-cleaving ribozyme is added at the 5'-end of the transcript to allow the ribozyme to accurately cleave off the end of the minus-strand RNA viral genome (Inoue, K. et al. J. Virol. Methods 107, 2003, 229-236). Alternatively, in order to accurately regulate the 5'-end of the transcript, the recognition sequence of bacteriophage RNA polymerase is used as a transcription initiation site, and the RNA polymerase is expressed within a cell to induce transcription. The bacteriophage RNA polymerase used include, for example, those of E. coli T3 phage and T7 phage, and Salmonella SP6 phage (Krieg, P.A. and Melton, D.A. 1987, Methods Enzymol. 155: 397-15; Milligan, J.F. et al., 1987, Nucleic Acids Res. 15: 8783-798; Pokrovskaya, I.D. and Gurevich, V.V, 1994, Anal. Biochem. 220: 420-23). Such bacteriophage RNA polymerases can be supplied using, for example, vaccinia viruses expressing the polymerases (Fuerst, T.R. et al., Proc. Natl. Acad. Sci. USA 83, 8122-8126 (1986), or supplied from expression vectors such as plasmids. To regulate the 3'-end of the transcript, for example, a method in which a self-cleaving ribozyme encoded at the 3'-end of the transcript is allowed to accurately cleave the 3'-end, is also known (Hasan, M. K. et al., J. Gen. Virol. 1997: 78: 2813-2820; Kato, A. et al., EMBO J. 1997, 16: 578-587; and Yu, D. et al., Genes Cells 1997, 2: 457-466). An auto-cleaving ribozyme derived from the antigenomic strand of delta hepatitis virus can be used.

In the reconstitution of viruses in which the envelope-constituting protein genes have been deleted, the infectivity of viruses may be complemented by the deleted envelope-constituting proteins. However, for example, the viruses may also be pseudotyped with envelope proteins other than the deleted proteins. Such an envelope protein used may be, for example, the G protein of vesicular stomatitis virus (VSV) (VSV-G) (J. Virology 39: 519-528 (1981)) (Hirata, T. et al., 2002, J. Virol. Methods, 104:125-133; Inoue, M. et al., 2003, J. Virol. 77:6419-6429; Inoue M. et al., J Gene Med. 2004; 6:1069-1081). Besides the viral envelope proteins, for example, polypeptides derived from adhesion factors, ligands, receptors, and the like, which may attach to particular cells, can be used for modification. Alternatively, chimeric proteins that have those polypeptides in the extracellular domain and have viral envelope-derived polypeptides in the intracellular domain can also be used. This makes it possible to produce vectors targeting particular tissues. Genes to be deleted from the genome include, for example, genes of spike proteins such as F, HN, H, and G, genes of envelope-lining proteins such as M, and any combinations thereof. Deletion of a spike protein gene is effective in rendering minus-strand RNA viruses nontransmissible, whereas deletion of the gene of an envelope-lining protein such as M protein is effective in disabling the particle formation from infected cells. For example, F gene-defective minus-strand RNA viruses (Li, H.-O. et al., J.Virol. 74, 6564-6569 (2000)), M gene-defective minus-strand RNA viruses (Inoue, M. et al., J.Virol. 77, 6419-6429 (2003)), and the like are preferably used. Moreover, greater safety would be assured with viruses defective in any combination of at least two of F, HN (or H) and M genes. For example, viruses lacking both M and F genes are non-transmissible and defective in particle formation while retaining high level infectivity and gene expression ability. These viruses are particularly useful since a high level of safety can be secured when they are used as viral vectors.

In an example of the production of F gene-defective recombinant viruses, for example, a plasmid expressing a minus-strand RNA viral genome defective in F gene or a complementary strand thereof is transfected into host cells along with an expression vector expressing F protein and expression vectors for N, P, and L proteins. Alternatively, viruses can be more efficiently produced by using host cells in which the F gene has been incorporated into their chromosomes (WO00/70070). In this case, a sequence-specific recombinase such as Cre/loxP and FLP/FRT and a target sequence thereof are preferably used so that the F gene can be inducibly expressed (see WO00/70055, WO00/70070; Hasan, M. K. et al., 1997, J. General Virology 78: 2813-2820). Specifically, for example, the envelope protein genes are integrated into a vector having a recombinase target sequence, such as the Cre/loxP inducible expression plasmid pCALNdlw (Arai, T. et al., J. Virology 72, 1998, p1115-1121). The expression is induced by, for example, infection with the adenovirus AxCANCre at an MOI of 3 to 5 (Saito et al., Nucl. Acids Res. 23: 3816-3821 (1995); and Arai, T. et al., J. Virol 72, 1115-1121 (1998)).

The minus-strand RNA viruses used in the present invention may be deficient in accessory genes. For example, by knocking out the V gene, one of the accessory genes of Sendai virus (SeV), the pathogenicity of SeV toward hosts such as mice is remarkably reduced without hindering gene expression and replication in cultured cells (Kato, A. et al., 1997, J. Virol. 71:7266-7272; Kato, A. et al., 1997, EMBO J. 16:578-587; Curran, J. et al.; WO01/04272; and EP1067179).

In addition to mutation sites in the L gene as described above, minus-strand RNA viruses used may further include mutations in the P gene or L gene so as to enhance the persistence of infection. Specific examples of such mutations include mutation of Glu at position 86 (E86) of the SeV P protein, substitution of Leu at position 511 (L511) of the SeV P protein with another amino acid, or substitution of homologous sites in the P protein of a different minus-strand RNA virus. Specific examples include substitution of the amino acid at position 86 with Lys, and substitution of the amino acid at position 511 with Phe. Regarding the L protein, examples include substitution ofAsn at position 1197 (N1197) and/or Lys at position 1795 (K1795) in the SeV L protein with other amino acids, or substitution of homologous sites in the L protein of another minus-strand RNA virus, and specific examples include substitution of the amino acid at position 1197 with Ser, and substitution of the amino acid at 1795 with Glu. Mutations of the P gene and L gene can be expected to significantly increase the effects of persistent infectivity, suppression of the release of secondary virions, and suppression of cytotoxicity.

Regarding more specific methods for the reconstitution of recombinant viruses, one can refer to, for example, the following references: WO97/16539; WO97/16538; WO00/70055; WO00/70070; WO01/18223; WO03/025570; WO2005/071092; Durbin, A. P. et al., 1997, Virology 235: 323-332; Whelan, S. P. et al., 1995, Proc. Natl. Acad. Sci. USA 92: 8388-8392; Schnell. M. J. et al., 1994, EMBO J. 13: 4195-4203; Radecke, F. et al., 1995, EMBO J. 14: 5773-5784; Lawson, N. D. et al., Proc. Natl. Acad. Sci. USA 92: 4477-4481; Garcin, D. et al., 1995, EMBO J. 14: 6087-6094; Kato, A. et al., 1996, Genes Cells 1: 569-579; Baron, M. D. and Barrett, T., 1997, J. Virol. 71: 1265-1271; Bridgen, A. and Elliott, R. M., 1996, Proc. Natl. Acad. Sci. USA 93: 15400-15404; Hasan, M. K. et al., J. Gen. Virol. 78: 2813-2820, 1997; Kato, A. et al., 1997, EMBO J. 16: 578-587; Yu, D. et al., 1997, Genes Cells 2: 457-466; Tokusumi, T. et al. Virus Res. 2002: 86; 33-38; Li, H.-O. et al., J. Virol. 2000: 74; 6564-6569. Following these methods, minus-strand RNA viruses including parainfluenza virus, vesicular stomatitis virus, rabies virus, measles virus, rinderpest virus, Sendai virus, and the like can be reconstituted from DNA.

In the present invention, the minus-strand RNA viruses include infectious viral particles, noninfectious viral particles (also referred to as virus-like particles (VLP)), and viral core (RNP complex containing the genome and genome-binding viral proteins). Specifically, the minus-strand RNA virus of the present invention refers to a complex containing a ribonucleoprotein (RNP) complex that contains genome RNA derived from a minus-strand RNA virus and viral proteins required for the replication of the RNA and the expression of a carried gene. The RNP is, for example, a complex containing genome RNA of the minus-strand RNA vitus or a complementary strand thereof (antigenomic RNA), and N, L, and P proteins. RNP (viral core) obtained by removing the envelope from a virion is, when introduced into cells, also capable of replicating the viral genomic RNA in the cells (WO 97/16538; WO 00/70055). RNP or VLP may be administered together with a transfection reagent or such to hosts (WO 00/70055; WO 00/70070).

Desired mammalian cells and the like can be used for virus production. Specific examples of such cells include cultured cells, such as LLC-MK2 cells (ATCC CCL-7) and CV-1 cells (for example, ATCC CCL-70) derived from monkey kidney, BHK cells (for example, ATCC CCL-10) derived from hamster kidney, and cells derived from humans. In addition, to obtain a large quantity of virus, a virus obtained from the above-described host can be used to infect embryonated hen eggs to amplify the virus. Methods for manufacturing viruses using hen eggs have already been developed (Nakanishi, et al., ed. (1993), "State-of-the-Art Technology Protocol in Neuroscience Research III, Molecular Neuron Physiology", Koseisha, Osaka, pp. 153-172). For example, a fertilized egg is placed in an incubator, and cultured for nine to twelve days at 37 to 38°C to grow an embryo. After the virus is inoculated into the allantoic cavity, the egg is then cultured for several days (for example, three days) to proliferate the virus. Conditions such as the period of culture may vary depending upon the recombinant Sendai virus being used. Then, allantoic fluids, including the virus, are recovered. Separation and purification of Sendai virus from allantoic fluids can be performed according to conventional methods (Tashiro, M., "Virus Experiment Protocol," Nagai, Ishihama, ed., Medical View Co., Ltd., pp. 68-73, (1995)).

The recovered viruses can be purified to be substantially pure. Purification can be achieved using known purification/separation methods, including filtration, centrifugation, adsorption, and column purification, or any combinations thereof. The phrase "substantially pure" means that the virus component constitutes a major proportion of a solution of the virus. For example, a viral composition can be deemed "substantially pure" based on the fact that the proportion of protein contained as the viral vector component as compared to the total protein (excluding proteins added as carriers and stabilizers) in the solution is 10% (w/w) or greater, preferably 20% or greater, more preferably 50% or greater, preferably 70% or greater, more preferably 80% or greater, and even more preferably 90% or greater. Specific purification methods for the paramyxovirus include, for example, methods using cellulose sulfate ester or cross-linked polysaccharide sulfate ester (Japanese Patent Application Kokoku Publication No. (JP-B) S62-30752 (examined, approved Japanese patent application published for opposition), JP-B S62-33879, and JP-B S62-30753) and methods including adsorption to fucose sulfate-containing polysaccharide and/or degradation products thereof (WO97/32010); however, the invention is not limited thereto.

The minus-strand RNA virus of the present invention can be prepared as a composition according to the purpose. When producing a composition containing the virus of the present invention, the virus may be combined with a desired pharmaceutically acceptable carrier or medium as necessary. The "pharmaceutically acceptable carrier or medium" includes, for example, desired solutions and the like that can be used in subjects to be administered with the virus or a processed product thereof. When the virus of the present invention is used as a vector, the carrier or medium is a material that can be administered together with the vector and do not significantly inhibit gene transfer mediated by the vector. For example, the virus of the present invention can be appropriately diluted with physiological saline, phosphate-buffered saline (PBS), or the like to prepare a composition. Furthermore, the composition containing the virus may also contain a carrier or medium such as deionized water or aqueous solution of 5% dextrose. The composition may further contain vegetable oils, suspending agents, surfactants, stabilizers, biocidal agents, and the like. Preservatives or other additional agents can also be added. Furthermore, an organic material such as a biopolymer, an inorganic material such as hydroxyapatite, specifically, collagen matrix, polylactic acid polymer or copolymer, polyethylene glycol polymer or copolymer, or a chemical derivative thereof, can be combined as a carrier. Compositions containing a minus-strand RNA virus of the present invention are useful as reagents and pharmaceuticals.

When a vector is prepared using a gene for treating diseases as a foreign gene, this vector can be administered to perform gene therapy. When applying the viral vector of the present invention to gene therapy, either direct administration or indirect (*ex vivo*) administration allows the expression of a foreign gene that is expected to produce a therapeutic effect, or endogenous gene that is insufficiently supplied in the patient's body, or the like. For example, when a gene encoding an antigen of bacteria or virus involved in an infection is used as a foreign gene, administration of this to an animal can induce immunity in the animal. In other words, the viral vector can be used as a vaccine. Thus, the vectors of the present invention may be clinically applicable to gene therapy or the like.

The amount and number of doses of the vector can be appropriately determined by those skilled in the art, although it varies depending on the disease, patient's weight, age, sex, symptom, purpose of administration, form of composition administered, administration method, gene to be introduced, and such. The route of administration can be appropriately selected, and includes, for example, percutaneous, intranasal, transbronchial, intramuscular, intraperitoneal, intravenous, intraarticular, intraspinal, and subcutaneous administrations, but is not limited thereto. The vector may be administered locally or systemically. Subjects administered with a composition comprising the vector of the present invention include all mammals such as humans, monkeys, mice, rats, rabbits, sheep, bovines, dogs, etc.

Furthermore, the present invention provides methods for attenuating minus-strand RNA viruses. Specifically, a minus-strand RNA virus can be attenuated by introducing a mutation that results in amino acid substitution at a position corresponding to position 1214 in the amino acid sequence of SEQ ID NO: 1 in the gene encoding L protein of the minus-strand RNA virus.

As in the attenuated minus-strand RNA viruses described above, the amino acid substitution in the above-described methods of the present invention for attenuating minus-strand RNA viruses is preferably substitution of tyrosine with phenylalanine (substitution by point mutation).

In the above methods of the present invention for attenuating minus-strand RNA viruses, it is preferred that at least one or more of the genes encoding the envelope-constituting proteins of the minus-strand RNA virus are deleted or inactivated; or the methods further comprise the step of deleting or inactivating at least one or more of the genes. In the present invention, the deleted or inactivated genes, or genes to be deleted or inactivated include any one of, or a combination of two or more of, the genes encoding F, HN, and M proteins.

By using the methods of the present invention for attenuating minus-strand RNA viruses, the present inventors discovered that the Sendai virus vector having Y1214F exhibited almost no cytotoxicity and the methods significantly reduced the cytotoxicity. The present inventors also revealed that the vector showed a foreign gene-expressing ability comparable to that of adenovirus and allowed the expression of a foreign gene to be maintained at a sufficient level, while the viral antigenicity was significantly reduced by Y1214F mutation. In addition, the present inventors found that the Y1214F mutation may reduce the activity of L protein and as a result reduce the amounts of SeV antigen and viral RNA, leading to the reduction of immune response. Thus, the methods of the present invention for attenuating minus-strand RNA viruses can be conducted to reduce the cytotoxicity to cells introduced with the viruses, to improve the persistency of foreign gene expression, or to reduce immune responses.

The minus-strand RNA viruses to be attenuated by the methods of the present invention for attenuating minus-strand RNA viruses preferably include Paramyxoviridae viruses, more preferably Sendai virus.

Furthermore, the present invention provides methods for producing an attenuated minus-strand RNA virus. Specifically, the methods for producing an attenuated minus-strand RNA virus comprise the step of introducing a mutation in the gene encoding L protein of a minus-strand RNA virus to substitute the amino acid at a position corresponding to position 1214 in the amino acid sequence of SEQ ID NO: 1.

As in the above-described attenuated minus-strand RNA viruses or in the above-described methods for attenuating minus-strand RNA viruses, the amino acid substitution in the above-described methods of the present invention for producing minus-strand RNA viruses include substitution of tyrosine with phenylalanine (substitution by point mutation).

In the above-described methods of the present invention for producing an attenuated minus-strand RNA virus, it is preferred that at least one or more of the genes encoding the envelope-constituting proteins of an minus-strand RNA virus are deleted or inactivated; or the methods further comprise the step of deleting or inactivating at least one or more of the genes. In the present invention, the "deleted or inactivated genes, or genes to be deleted or inactivated" is any one of, or a combination of two or more of, the genes encoding F, HN, and M proteins.

The minus-strand RNA viruses in the methods of the present invention for producing an attenuated minus-strand RNA virus preferably include Paramyxoviridae viruses, more preferably Sendai virus.
All the prior-art documents cited herein are incorporated as parts of this description.

Examples

Hereinbelow, the present invention will be specifically described using Examples; however, it is not to be construed as being limited thereto.

Materials and methods

[Example 1] Genes

1-1. Sendai virus

Sendai virus used was Z strain (15 kb). Some genes were deleted or amino acid mutations were inserted.

1-2. Reporter genes

EGFP: a green fluorescent protein with an altered nucleotide sequence derived from luminous *Aequorea victoria*; 720 b (Accession No. U57606) gene was inserted into the Sendai virus genome.
LacZ: β-galactosidase; 3.1 kb (Accession No. U13184) gene was inserted into the Sendai virus genome.

[Example 2] Cell culture

2-1. Cell lines

LLC-MK2: cell line derived from Rhesus monkey kidney
CV-1: cell line derived from African green monkey kidney
HEK 293: cell line derived from human fetal kidney
Mouse bone marrow mesenchymal cells: collected from the thigh of C57BL/6 mice

2-2. Cell culture

Cells of LLC-MK2 (ATCC CCL-7) and CV-1 (ATCC CCL-70) lines, which are monkey kidney-derived cell lines, were suspended in minimal essential medium (MEM) (Invitrogen-GIBCO, Cat. No. 11095-080) containing 10% fetal bovine serum (FBS; GIBCO-BRL, Cat. No. 10099-141), 100 μg/ml penicillin, and 100 units/ml streptomycin (Nacarai Tesque, Cat. No. 26253-84) and cultured under 5% carbon dioxide at 37°C. Cells of HEK 293 line (ATCC CRL-1573) were suspended in Dulbecco's Modified Eagle Medium (DMEM) (Invitrogen-GIBCO, Cat. No. 11995-065) containing 10% FBS, 100 μg/ml penicillin, and 100 units/ml streptomycin, and cultured under the same conditions as described above. Mouse bone marrow cells were collected from the thigh of C57BL/6 mice, and then suspended in RPMI1640 (Invitrogen-GIBCO, Cat. No. 11875-093) containing 10% FBS. The cells were cultured under the same conditions as described above.

[Example 3] Preparation of virus

3-1. Preparation of Sendai virus vector

F gene-deficient vector (SeV/ΔF) and M gene-deficient vector (SeV/ΔM) were harvested using the packaging cell lines: LLC-MK2/F7 (cells expressing F protein) (Li, H. O., Zhu, Y F., Asakawa, M., Kuma, H., Hirata, T., Ueda, Y., Lee, Y. S., Fukumura, M., Iida, A., Kato, A., et al. (2000), J Virol 74, p. 6564-6569) and LLC-MK2/F7/M62 (cells expressing M protein) (Inoue, M., Tokusumi, Y., Ban, H., Kanaya, T., Shirakura, M., Tokusumi, T., Hirata, T., Nagai, Y., Iida, A., and Hasegawa, M. (2003), J Virol 77, p. 6419-6429), respectively. The packaging cell lines stably supply the proteins whose encoding genes have been deleted from the vectors.

3-2. Preparation of adenovirus vector

The respective proteins whose encoding genes had been deleted were induced using adenovirus vector (Ax-

CANCre) (Nakano, 2003, P147) that expressed Cre recombinase. LacZ-expressing type 5 adenovirus vector (Adeno-CALacZ) was harvested using HEK 293 cells, and its titer was determined using Adeno-X™ Rapid Titer Kit (BD Bioscience, Cat. No. K1653-1).

[Example 4] Preparation of mutant SeV strains

4-1. Establishment of cells persistently infected with SeV/ΔM-GFP

Fully confluent LLC-MK2 cells (1 x $10^6$ cells/well) prepared in 6-well plates were infected at a MOI of 3 with M gene-deficient SeV vector (SeV/VM-GFP) carrying the GFP gene, and then cultured in serum-free MEM at 37°C. After two days, 2 x $10^5$ cells were passaged into freshly prepared serum-containing MEM. The passage was repeated five times. When the cells became confluent at the fifth passage, the infected cells were plated at 1 cell/well in 96-well plates by the limiting dilution method. Two weeks after plating, the surviving cells were grown to form colonies. Only GFP-positive cell clones were selected as cells persistently infected with SeV/ΔM-GFP.

4-2. Isolation of virus from persistently infected cells

Viruses were isolated from cells persistently infected with SeV/ΔM-GFP by the following procedure. Cells of clone persistently infected with SeV/ΔM-GFP were expanded in 12-well plates. When the cells became fully confluent, the entire cells and culture supernatants were transferred into Eppendorf tubes. Three cycles of freeze-thawing yielded cytoplasmic ribonucleoproteins (RNPs) and primary virus. The resulting primary virus was allowed to infect freshly prepared LLC-MK2/F7/M62 after M protein induction. The cells were cultured in MEM containing trypsin at 32°C for five days. Then, the daughter virus vector in the culture supernatant was harvested. The presence of daughter viral vector particles in the culture supernatant was confirmed by HA assay.

4-2-1. HA assay

The culture supernatant containing daughter virus was step-diluted with PBS in a round-bottomed 96-well plate (Iwaki, Cat. No. 3870-096), and the volumes were adjusted to 50 μl/well. Chicken erythrocytes were washed five times with PBS and adjusted to 8 x $10^7$ cells/ml. After step dilution, chicken erythrocytes (50 μl) were added to the plate, and allowed stand for one hour at 4°C. The resulting agglutination reaction was observed.

4-3. Selection of isolated viruses

Fully confluent LLC-MK2/F7/M62 after M protein induction prepared in 12-well plates was infected with the isolated daughter viral vectors at a constant amount. The cells were cultured in MEM containing trypsin at 32°C or 37°C for five days. Then, the culture supernatants for each temperature were collected and assessed by HA assay. When exhibiting the same tendency as parental SeV/ΔM-GFP, the cells were classified as mutant -; when being different from parental SeV/ΔM-GFP, the cells were classified as mutant +.

Fully confluent LLC-MK2 cells (5 x $10^6$ cells/well) prepared in 12-well plates were infected with the isolated daughter virus vectors at a constant amount. The cells were cultured in MEM containing trypsin at 32°C or 37°C for five days. The morphologies of the cells were observed. When exhibiting cytotoxicity and cell fusion such as observed with parental SeV/ΔM-GFP, the cells were classified as mutant -; when the degree of morphological change was little, the cells were classified as mutant ± or + depending on the degree; and when there was no morphological change, the cells were classified as mutant ++.

4-3-1. Flow cytometry

Isolated viruses assigned as "mutant ++" were allowed to infect LLC-MK2 at a MOI of 1. After infection, the expression of GFP was assayed with a FACS Calibur™ flow cytometer (Becton Dickinson). Non-infected cells were used as a negative control.

[Example 5] Identification of mutant strains

Viruses predicted to be mutant ++, +, or ± were assessed for the mutations by determining the nucleotide sequences of their viral genomes. RNAs were extracted from the harvested viral vectors using QIAamp viral RNA minikit (QIAGEN, Cat. No. 52906). Reverse transcription (RT)-PCR was carried out using Superscript™ RT-PCR system (Invitrogen, Cat.No. 10928-042) with random hexamer and SeV-specific primers. Then, the nucleotide sequences were determined with ABI PRISM™ 377 (Applied Biosystem Japan). All clones predicted to be mutant ++ were sequenced over the entire viral genome, while clones predicted to be mutant + or ± were sequenced for the F and HN genes involved in cell fusion.

[Example 6] Construction of F gene-deficient SeV plasmids that comprise mutation and carry EGFP

6-1. Construction of F gene-deficient SeV plasmid that comprises two types of mutations and carries EGFP (pSeV/ΔF-GFP-1214-1602)

F gene-deficient SeV plasmid that comprises two types of mutations and carries EGFP (pSeV/ΔF-GFP-1214-1602) was constructed by the following procedure from SeV/ΔM-GFP clone#37 having the two types of mutations (Y1214F and M1602L) identified in the L gene. The SeV RNA genome was extracted from SeV/ΔM-GFP clone#37. After RNA purification, cDNA was synthesized using random hexamer and Superscript™ II (Invitrogen, Cat.No.18064-041). A PCR fragment having the two types of mutations was amplified with SeV-specific primers using the synthesized cDNA as the template.
5'-ATCACTGCTAGATCTGTGCTGC-3' (sense) (SEQ ID NO: 3) 5'-GGACTCCTATACCTCTTGTCCT-3' (antisense) (SEQ ID NO: 4)
The amplified 2-kb fragment, which was an inner fragment of the L gene, had *Nhe*I and *Xho*I, both of which are SeV unique sites. Thus, the PCR fragment was digested with restriction enzymes *Nhe*I and *Xho*I, and inserted between the same sites within the L gene of pSeV/ΔF-GFP. The constructed pSeV/ΔF-GFP-1214-1602 was sequenced to confirm the nucleotide sequence of the site introduced with the mutation.

6-2. Construction of F gene-deficient SeV plasmids that comprise a single mutation and carry EGFP (pSeV/ΔF-GFP-1214 and pSeV/ΔF-GFP-1602)

F gene-deficient SeV plasmids that carry EGFP and comprise either of the two types of mutations (Y1214F and M1602L) identified in the L gene were constructed (pSeV/ΔF-GFP-1214 and pSeV/ΔF-GFP-1602, respectively) by the following procedure. First, a portion of the wild type L gene was amplified using the same primers as used in 6-1 and the wild type SeV-L gene as the template. Then, the amplified fragment was inserted into pGEM-T vector (Promega, Cat. No. A1360) to construct pGEM-L (2 kb) having the portion (2 kb) of SeV-L. pGEM-L-1214 and pGEM-L-1602 were constructed by PCR using pGEM-L as the template and the primers listed below.
Y1214F
5'-gctataagcctcccctTttttggatcagccactgatg-3' (sense) (SEQ ID NO: 5)
5'-catcagtggctgatccaaaaAaggggattcttatagc-3' (antisense) (SEQ ID NO: 6)
M1602L
5'-cagatgtggccgacTtgaggaggtcctctttcttg- 3' (sense) (SEQ ID NO: 7)
5'-caagaaagaggacctcctcaAgtcggccacatctg- 3' (antisense) (SEQ ID NO: 8)
The constructed pGEM-L-1214 and pGEM-L-1602 were sequenced to confirm their nucleotide sequences.
Next, pGEM-L-1214 and pGEM-L-1602 were digested with restriction enzymes *Nhe*I and *Xho*I, and each of the resulting 1.5-kb fragments was inserted between the same sites in pSeV/ΔF-GFP. The constructed pSeV/ΔF-GFP-1214 and pSeV/ΔF-GFP-1602 were sequenced to confirm the nucleotide sequence of the site introduced with the mutation.

6-3. Insertion of LacZ gene

To determine the expression level of the foreign gene carried by the SeV genome, the LacZ gene was inserted upstream of the coding region of NP gene by the following procedure. pSeV$^{18+}$LacZ/ΔMΔF (Inoue, M., Tokusumi, Y., Ban, H., Shirakura, M., Kanaya, T., Yoshizaki, M., Hironaka, T., Nagai, Y., Iida, A., and Hasegawa, M. (2004), J Gene Med 6, p. 1069-1081) was digested with restriction enzyme *Not*I to excise a *Not*I fragment of the LacZ gene containing the transcription end-intervening-transcription start (EIS) element. The fragment was inserted into the *Not*I site between the NP gene and start signal to construct pSeV$^{18+}$LacZ/ΔF and pSeV$^{18+}$LacZ/ΔF-1214.

[Example 7] Construction of F gene-deficient SeV vector comprising mutation

LLC-MK2 cells plated at about 1 x $10^7$ cells/10-cm dish were co-transfected with pCAGGS plasmid (WO 2005/71085) carrying the NP, P, F, HN, and L gene of SeV, each of F gene-deficient SeV plasmids that comprise mutation and carry EGFP (pSeV/ΔF-GFP-1214, pSeV/ΔF-GFP-1602, and pSeV/ΔF-GFP-1214-1602), and F gene-deficient SeV plasmid that comprise mutation and carry the LacZ gene (pSeV$^{18+}$LacZ/ΔF-1214 and pSeV$^{18+}$LacZ/ΔF-1214-1602).

The LLC-MK2 cells were cultured in MEM containing 7.5 μg/ml trypsin for 24 hours. Then, LLC-MK2/F7 which had been pre-infected with AxCANCre at a MOI of 5 to induce F protein was overlaid onto the LLC-MK2 cells. The cells were incubated. 48 hours after overlay, the whole co-cultured cells were harvested and treated with three cycles of freeze-thawing in Opti-MEM to give cytoplasmic RNPs (ribonucleoprotein) and primary virus. The resulting primary virus was allowed to infect freshly prepared LLC-MK2/F7 after F protein induction. The cells were cultured in MEM containing trypsin at 32°C for five to ten days. The F gene-deficient SeV vectors carried the GFP gene. Therefore, if a transmission of GFP expression to the adjacent cells was observed, it suggests that the viral vectors have been released to the culture supernatants. The culture supernatants containing the viral vectors were collected, and then freshly prepared packaging cells were infected with these culture supernatants. This step was repeated to amplify the vectors. The viral vectors prepared by two rounds of amplification were combined with a final concentration of 1% of BSA, and stored at -80°C. These viral vectors were used in the experiments described herein.

The titers of harvested viral vectors were determined by calculating the proportions of GFP expressing cells and cells positive for LacZ staining per 1 ml.

[Example 8] Determination of cytotoxicity

About 4 x $10^4$ CV-1 cells in each well (fully confluent in 96-well plates) were infected with SeV/ΔF-GFP, SeV/ΔF-GFP-1214, SeV/ΔF-GFP-1602, or SeV/ΔF-GFP-1214-1602 at a MOI of 0.1, 0.3, 1, 3, or 10 at 32°C or 37°C for six hours. The cells were cultured in serum-free MEM. The culture supernatants were collected three days after infection. The supernatants were assayed for lactase dehydrogenase (LDH) using Cytotoxicity Detection Kit (Roche Cat. No. 1664793) (Decker, T., and Lohmann-Matthes, M. L. (1988), J Immunol Methods 115, p. 61-69).

[Example 9] Assessment for the persistent infectivity

LLC-MK2 (5 x $10^4$ cells/well) and CV-1 cells (1 x $10^5$ cells/well) prepared in 6-well plates were infected with SeV/ΔF-GFP or SeV/ΔF-GFP-1214 at a MOI of 100, and then cultured in MEM containing 10% FBS at 37°C. The infected cells were sampled every day to count the GFP-positive viable cells.

On day five after infection, the cells were photographed under a fluorescence microscope. 2 x $10^5$ LLC-MK2 cells infected with each vector were passaged for five times. After five passages, the cells were photographed under a fluorescence microscope.

[Example 10] Ability to express genes in primary culture cells

Bone marrow mesenchymal cells were collected from the thigh of C57BL/6 mice, and plated at 1 x $10^5$ cells/well in poly-L-lysine-coated 6-well plates (SUMILON, Cat. No. MS-0006L). After one week of culture, the cells were infected with SeV/ΔF-GFP or SeV/ΔF-GFP-1214 at a MOI of 100 for 24 hours, and then cultured in RPMI1640 containing 50 μM 2-mercaptoethanol, 100 μM MEM NEAA (Non-Essential Amino Acid solution; GIBCO, Cat. No. 11140-050), 1 mM Sodium pyruvate (SIGMA, Cat. No. S8636), 2 mM L-Glutamine solution (GIBCO, Cat. No. 25030-081), and 10% FBS. The cells were observed for GFP expression under a fluorescence microscope on day 7 and 14 after infection.

[Example 11] Quantitation of cytoplasmic SeV RNA by real-time PCR

11-1. Preparation of infected cells

Fully confluent LLC-MK2 cells prepared in 12-well plates (5 x $10^6$ cells/well) were infected with SeV/ΔF-GFP, SeV/ΔF-GFP-1214, SeV/ΔF-GFP-1602, or SeV/ΔF-GFP-1214-1602 at a MOI of 3, and cultured in serum-free MEM at 32°C or 37°C. The cells were harvested during the period of 2 to 32 hours after infection. At the time of harvest, the number of viable cells was counted to determine the cell count per well. The same procedure was used for F gene-deficient Se V that comprises mutation and carries the LacZ gene.

11-2. Preparation of cytoplasmic RNA

Cytoplasmic RNA was prepared from the infected cells harvested by the method of Gough (Gough, N. M. (1988), Anal Biochem 173, p. 93-95). The pellets of harvested cells were suspended in 100 μl of lysis buffer (10 mM Tris-HCl (pH 7.5), 150 mM NaCl, 1.5 mM $MgCl_2$, 0.65%(w/v) NP-40). The cell nuclei were removed by centrifugation. After an equal volume of extraction buffer (10 mM Tris-HCl (pH 7.5), 350 mM NaCl, 10 mM EDTA, 7 M Urea, 1%(w/v) SDS) was added to the obtained cytoplasmic liquid, phenol/chloroform purification was carried out to remove cell proteins. The aqueous layer containing cytoplasmic RNA was isolated and the RNA was precipitated using three parts ethanol The resulting pellet was air dried, and then dissolved with sterile water so that the concentration of cytoplasmic RNA was 1 μg/μl. The following formula was used:

[Number of cells derived from 1 μg of purified cytoplasmic RNA] = [Number of cells used to prepare cytoplasmic RNA (cells)]/[total amount of cytoplasmic RNA (μg)]

11-3. Synthesis of SeV cDNA from purified cytoplasmic RNA

SeV cDNA was synthesized by reverse transcription (RT) using 1 μg of purified cytoplasmic RNA as a template, Superscript™ II (Invitrogen, Cat. No. 18064-041), and oligo dT primer (12-18) (primer for SeV mRNA), random hexamer (primer for total SeV RNA), or the primers listed below. The volume of synthesized SeV cDNA was adjusted to 20 μl.
5'-caagagaaaaacatgtatgg-3' (primer for SeV genome) (SEQ ID NO: 9)
5'-agagtttaagagatatgtagcc-3' (primer for SeV antigenome) (SEQ ID NO: 10)
The following formula was used:

[Number of cells used to prepare 20 μl of synthesized SeV cDNA] = [Number of cells corresponding to 1 μg of purified cytoplasmic RNA].

11-4. RNA quantitation using synthesized SeV cDNA

Real-time PCR was carried out using as a template a constant amount of each of synthesized SeV cDNAs, QuantiTect™ SYBR Green Kit (QIAGEN Cat.No.204143), ABI PRISM™ 7700 (Applied Biosystem Japan), and the primers listed below. The reaction volume was 50 μl.
Inside SeV-L gene
5'-cctccactaatctatctcatagg-3' (sense) (SEQ ID NO: 11)
5'-ataacaatacttggctgaacgtg-3' (anti sense) (SEQ ID NO: 12)
Inside LacZ gene
5'-cggattggcctgaactgc-3' (sense) (SEQ ID NO: 13)
5'-aacaggcggcagtaaggc- 3' (anti sense) (SEQ ID NO: 14)

[Number of cells corresponding to a single tube of real-time PCR] = [Number of cells corresponding to 1 μg of purified cytoplasmic RNA] x ([SeV cDNA (μl) used in real-time PCR]/[20 μl of total SeV cDNA])

[SeV RNA copy number per cell] = [Value determined by real-time PCR detection (mean)]/[Number of cells corresponding to a single tube of real-time PCR]
pSeV$^{18+}$LacZ/ΔF plasmid was used as a standard to prepare a calibration curve.

[Example 12] Detection and quantitation of LacZ protein expression

12-1. LacZ staining

Fully confluent LLC-MK2 cells prepared in 6-well plates (1 x $10^6$ cells/well) were infected with SeV$^{18+}$LacZ/ΔF or SeV$^{8+}$LacZ/ΔF-1214 at a MOI of 3, and then cultured in serum-free MEM at 32°C or 37°C. After 22 hours, the infected cells were LacZ-stained by the following procedure. The infected cells were washed with PBS, and fixed with a fixative (0.05% glutaraldehyde, 2% formamide) at 4°C for ten minutes. Then, the cells were incubated in an X-gal (5-bromo-4-chloro-3-indolyl-β-D-galactosidase) staining solution (1 mg/ml X-gal, 5 mM $K_3Fe(CN)_6$, 5 mM $K_4Fe(CN)_6$, 2 mM $MgCl_2$) at 37°C for 12 hours.

12-2. LacZ activity assay

The infected cells 22 hours after infection were prepared like as in LacZ staining. After harvesting, the number of cells was counted to determine the cell count per well. The cells were suspended in 250 mM Tris-HCl buffer (pH 8.0) and sonicated to crush them. The amount of cellular protein was determined based on OD at 595 nm using Bradford reagent (BIOLAD, Cat. No. 500-0006). Solution A (1 mM $MgCl_2$, 45 mM 2-mercaptoethanol) and 88 μg of o-nitrophenyl-β-D-galactosidase were added to a predetermined volume of the cellar protein fraction, and the resulting mixture was incubated at 37°C for 30 minutes. The reaction was terminated by adding an equal volume of a stop solution (100 mM sodium phosphate (pH 7.5), 1 M $Na_2CO_3$) to the reaction mixture. The mixture was assayed based on OD at 420 nm. A calibration curve was prepared by step-diluting LacZ whose concentration was known (Sigma, Cat. No. G4155).

Results

[Example 13] Establishment of mutant SeV strain (SeV/ΔM-GFP clone#37)

A possible means to obtain a mutant SeV strain with reduced cytotoxicity is to establish persistently infected cells. Thus, persistently infected cells were selected by a method based on infection of LLC-MK2 cells with SeV/ΔM-GFP vector. In general, persistently infected cells are obtained by using wild type viruses. In this case, after infection, daughter virus particles and the third-generation viral particles with the complete envelope are released into culture supernatant, and thus the cells are infected repeatedly. In the case of gene transfer vector, however, the purpose is to persistently express a gene of interest in the cells initially introduced with the vector, and thus the system where the persistent infection is established by the particles released after infection does not meet this original purpose. On the other hand, the M gene-deficient vector (SeV/ΔM) lacks the M gene in its genome, and therefore M protein essential for the particle formation is not produced after infection. Thus, no viral particles are released into culture supernatant. Specifically, there is no re-infection with the daughter virus. The genome of initially infecting SeV/ΔM-GFP vector is accumulated in the cytoplasm as a result of repeated transcription and replication. The emergence of cells that survive in spite of such accumulation indicates that the cells have acquired a mechanism of SeV tolerance or SeV has been introduced with some mutations that are beneficial for the continuous transcription/replication. The present inventors considered that persistent SeV infection could be established through either of the two phenomena. In this context, SeV/ΔM vector was used in this Example.

Because of the cytotoxicity, 90% or more of the infected cells were killed six days after SeV/ΔM-GFP infection. The remaining cells were cultured, and then cloned at the fifth passage. Since the vector used for infection carried GFP, GFP fluorescence was employed as a cloning marker. When cells were positive for GFP fluorescence, they were judged to be positive for SeV infection. Thus, 74 clones were obtained from the infected cells. Viruses could be isolated from cells of 60 clones among the 74 clones. Packaging cells expressing M protein (M-expressing LLC-MK2/M62) were infected with the isolated viruses, and cultured at 32°C or 37°C. Then, the daughter virus vector was compared to the parental strain SeV/ΔM-GFP with regard to the amount of released vector. The result showed that 59 of the 60 clones had the same tendency as that of the parental strain SeV/ΔM-GFP, but a single clone (clone#37) exhibited very different characteristics. It was revealed that at 37°C the number of particles was below the detection limit of HA assay while at 32°C the number of particles was comparable to that of the parental strain SeV/ΔM-GFP.

LLC-MK2 cells and the packaging cells were simultaneously infected with viruses of the isolated 60 clones, and they were visually observed for the cell fusion ability. However, there was no clear difference among most of the clones, and thus such differences could not be used as an indicator in the assessment. There was also no difference in the intensity of vector-derived GFP fluorescence between the parental strain SeV/ΔM-GFP and the clones at the two temperatures. However, likewise, only one clone (clone#37) exhibited very different characteristics. The clone gave only GFP fluorescence of a very low intensity at 37°C while at 32°C the fluorescence intensity was comparable to that of the parental strain SeV/ΔM-GFP (Figs. 1 and 3). Furthermore, the clone showed the same tendency in the infection of CV-1 cells that are sensitive to SeV infection. The clone exhibited markedly reduced cytotoxicity associated with SeV infection (Fig. 2).

The findings described above lead to the following prediction:

(1) since the GFP expression pattern varied depending on temperature, clone #37 has mutations involved in the temperature sensitivity; and

(2) since clone#37 showed no cytotoxicity even it infected at the same dose as the parental strain, clone#37 has mutations involved in the attenuation.

Of the isolated viruses, 59 clones predicted to be mutation + showed the same GFP fluorescence intensity as that of the parental strain. Nonetheless, the 59 clones could be passaged up to five generations. This led to the assumption that mutation has occurred in F or HN protein, both of which are displayed on the surface of infected cells. Thus, the 59 clones were sequenced to study the nucleotide sequences of their F and HN genes. Meanwhile, a single clone (clone #37) exhibited very different characteristics, and this led to the prediction that mutation has occurred in NP, P, or L protein, all of which are essential for the vector function. Thus, clone #37 was sequenced to confirm its entire SeV nucleotide sequence. The results showed that of the 59 clones, 15 clones had a total of 17 mutations within the F and HN genes. Of the 17 mutations, 12 mutations resulted in amino acid mutation. Clone #37 had no mutation within the F and HN genes; however, the clone had two mutations within L gene, which resulted in amino acid mutation (Fig. 4).

Since the indentified amino acid mutation at position 126 in the HN gene was found in three clones, the present inventors predicted that the mutation resulted in some phenotypic alterations. In spite of further analysis of the three clones, there was no detectable difference in the viral productivity, sialic acid-binding activity, and cytotoxicity when compared to the parental strain SeV/ΔM-GFP.

[Example 14] Identification of mutations

14-1. Harvest of F gene-deficient vector comprising mutations

F gene-deficient SeV vector (SeV/ΔF) was inserted with each of the two mutations Y1214F and M1602L in the L gene of SeV/ΔM-GFP clone#37 identified as described herein, in order to investigate which mutation is involved in the phenotypic alteration. SeV/ΔF is a vector lacking the F gene in its genome so that it is non-transmissible and consequently secured safe. Furthermore, the cytotoxicity has also been reduced as compared to the SeV vector called the first generation (without gene deletion). For these reasons, there have been various studies using SeV/ΔF as a backbone, and they have demonstrated its usefulness. The present invention aims at identifying mutations to further reduce the cytotoxicity and to achieve prolonged expression of carried genes after SeV vector-mediated gene transfer. Thus, the identified two types of mutations (Y1214F and M1602L) were introduced into SeV/ΔF, which is currently best studied among envelope gene-deficient SeV vectors, to assess (1) the effect of further reducing the cytotoxicity; and (2) sustained expression of carried genes. SeV/ΔF-GFP-1214 and SeV/ΔF-GFP-1602 introduced with a single mutation, and SeV/ΔF-GFP-1214-1602 having both mutations were prepared by the method of plasmid-based reverse genetics using F-expressing cells (LLC-MK2/F7). Every vector could be harvested in a yield of 1 x $10^8$ CIU/ml or more.

14-1. Cytotoxicity determination

CV-1 cells were infected with each of SeV/ΔF-GFP comprising mutation (SeV/ΔF-GFP-1214 and SeV/ΔF-GFP-1602 introduced with a single mutation, and SeV/ΔF-GFP-1214-1602 having both mutations) at a MOI of 0.1 to 10. The cytotoxicity at 32°C and 37°C was compared to that of SeV/ΔF-GFP (Fig. 5). The result showed that the cytotoxicity at 32°C was comparable between SeV/ΔF-GFP and SeV/ΔF-GFP-1602. Even at 37°C the reduction in the cytotoxicity was only 20%. The cytotoxicities of SeV/ΔF-GFP-1214 and SeV/ΔF-GFP-1214-1602 were reduced by 80% as compared to SeV/ΔF-GFP at each of the two temperatures.

When infected with SeV/ΔF-GFP or SeV/ΔF-GFP-1602 at a MOI of 30, most of the cells were observed to be detached. In the case of SeV/ΔF-GFP-1214 or SeV/ΔF-GFP-1214-1602, there were no detached cells, and thus GFP could be observed even three days after infection (Fig. 6). Moreover, GFP could be observed even five days after infection.

This result suggests that Y1214F mutation is involved in the reduction of cytotoxicity.

14-2. Productivity for F gene-deficient vector comprising mutation

Packaging cells LLC-MK2/F7 were infected with SeV/ΔF-GFP comprising each mutation at a MOI of 3, and the vector productivity at 32°C and 37°C was compared to that of SeV/ΔF-GFP (Fig. 7). The result showed that the productivity was comparable between SeV/ΔF-GFP and SeV/ΔF-GFP-1602 at each of the two temperatures. At 32°C, both SeV/ΔF-GFP-1214 and SeV/ΔF-GFP-1214-1602 were found to be produced a little more slowly; however, the rate became comparable to that of SeV/ΔF-GFP on day three. On the other hand, at 37°C, the productivity was 1/10000 as compared to SeV/ΔF-GFP up to three days. This result suggests that Y1214F mutation is involved in the temperature sensitivity.

[Example 15] Gene transfer to mouse bone marrow mesenchymal cells

The SeV/ΔF-GFP-1214-meidated expression of GFP was detectable in LLC-MK2 and CV-1 cells. Then, primary cultured bone marrow mesenchymal cells from C57BL/6 mice were infected to assess whether the cells also exhibit the same phenotype as that of the cell lines. The result showed that SeV/ΔF-GFP-i2i4 having Y1214F mutation in its L gene exhibited temperature sensitivity, like the cell lines tested as described above, and at 32°C the expression level was comparable to or greater than that of SeV/ΔF-GFP without the mutation. Furthermore, like the results described above, GFP fluorescence was detectable up to day 14 after infection in the cells infected with SeV/ΔF-GFP-1214 because of its cytotoxicity reducing effect (Figs. 8 and 9).

[Example 16] Detection of cytoplasmic SeV RNA by real-time PCR

Based on the results described above, Y1214F alone can be judged to be responsible for the temperature sensitivity and the reduction of cytotoxicity. Then, SeV/ΔF-GFP-1214 having mutation Y1214F involved in the phenotypic alteration and SeV/ΔF-GFP-1602 having M1602L, which resulted in no phenotypic alteration but was also found in clone#37 identified as a mutant strain, were compared to SeV/ΔF-GFP without mutations in regard to the copy number for the transcription/replication in the cytoplasm (Fig. 10). LLC-MK2 cells were infected with each of the SeV vectors at a MOI of 3, and the level of cytoplasmic SeV RNA was determined 20 hours after infection. SeV has a minus strand RNA as the genome. Thus, the minus strand RNA (SeV genome), plus strand RNA (SeV antigenome), and mRNA could be distinguished by using properly selected primers in the synthesis of cDNA from the RNA.

The result showed that the mRNA levels of SeV/ΔF-GFP-1214 and SeV/ΔF-GFP-1602 were both reduced as compared to SeV/ΔF-GFP. The mRNA level of SeV/ΔF-GFP-1602 was reduced by 30% regardless of the temperature difference. The mRNA level of SeV/ΔF-GFP-1214 was reduced by 86% and 98% at 32°C and 37°C, respectively, as compared to SeV/ΔF-GFP.

Furthermore, the level of SeV antigenome (plus strand RNA) showed the same tendency. In particular, the genome and antigenome copy numbers of SeV/ΔF-GFP-1214 at 37°C were low, namely seven copies per cell. When infected at a MOI of 3, in theory each cell contains three copies of the genome in the cytoplasm at the time of infection. According to the calculation based on the theoretical value, SeV vector having Y1214F replicates only once or twice in 20 hours after infection.

In the case of infection with SeV/ΔF-GFP-1602, there was no detectable quantitative alteration depending on temperature in the copy numbers of cytoplasmic mRNA and antigenome; however, the genome copy number tended to be reduced at 37°C. Based on this result, M1602L was assumed to be a mutation that is responsible for temperature-dependent replication inhibition in the stage of replication from the antigenome to genome. However, the inhibitory effect is assumed to be too weak to alter the phenotype.

These results demonstrated that the reason why Y1214F gives the phenotypic alteration, such as reduction of cytotoxicity and ability responsible for the persistent infection, is that Y1214F reduces the level of synthesized mRNA after infection, resulting in reduction of the genome replication level.

[Example 17] Stability with regard to the expression ability and ability responsible for persistent infection

LLC-MK2 and CV-1 cells were infected with SeV/ΔF-GFP-1214 containing Y1214F mutation that exhibited the effect of reducing the cytotoxicity or SeV/ΔF-GFP without the mutation at MOI of 100, in order to assess the influence of SeV infection on the growth of host cells (Fig. 11).

CV-1 cells, which are sensitive to SeV infection, were severely damaged by SeV/ΔF-GFP. Without proliferating, the cells were detached and killed. By contrast, in SeV/ΔF-GFP-1214 infection, the cell growth was decelerated temporarily on the third and fourth day after infection, but on day five the cell count was recovered to a level comparable to that of the non-infected cells.

When LLC-MK2 cells were infected with SeV/ΔF-GFP, the growth was found to be retarded from the second day after infection. The cells were not grown any more after the third day while the cell count was kept constant. By contrast, when infected with SeV/ΔF-GFP-1214, LLC-MK2 cells were grown at the same rate as the non-infected cells and the expression level of GFP was maintained constant (Fig. 12). Then, LLC-MK2 cells infected with SeV/ΔF-GFP-1214 were passaged up to five generations and their GFP expression level was observed. The result showed that the GFP expression level five days after infection was comparable to that five days after five passages (Fig. 13). These findings demonstrate that Y1214F alone is responsible for the ability of persistent infection.

[Example 18] Expression level of carried gene

The LacZ gene was inserted into vectors in order to construct SeV$^{18+}$LacZ/ΔF-1214 with Y1214F mutation responsible for the temperature sensitivity, reduction of cytotoxicity, and the ability of persistent infection; SeV$^{18+}$LacZ/ΔF without the mutation; and adenovirus vector carrying LacZ gene (AdenoCALacZ). The kinetics for the expression level of the carried gene was studied using the constructed vectors, and the expression levels were compared between the vectors (Fig. 14). The result showed that at 32°C the level of LacZ gene expression by SeV$^{18+}$LacZ/ΔF-1214 was increased 24 hours after infection and the level 32 hours after infection was 70% of the level of LacZ gene expression by SeV$^{18+}$LacZ/ΔF. The level was ten times greater when compared to the adenovirus vector, while at 37°C the expression level was comparable to that of the adenovirus vector.

[Example 19] Quantitation of mRNA of carried gene and genome

Real-time PCR was carried out to kinetically determine the expression level of LacZ gene, as well as the copy number of LacZ gene mRNA and copy number of SeV genome in the cytoplasm of cells infected with the vectors.

At 37°C the accumulation of LacZ mRNA in cells infected with $SeV^{18+}$LacZ/ΔF-1214 was significantly retarded as compared to $SeV^{18+}$LacZ/ΔF. The accumulation of LacZ mRNA in cells infected with $SeV^{18+}$LacZ/ΔF was increased proportionally up to 10 hours after infection, and then increased exponentially up to 22 hours after infection. The latter increase is assumed to be ascribed to mRNA synthesis using as the template the genome newly replicated ten hours after infection. Indeed, the SeV genome having the wild type L gene replicated ten hours after infection. In the case of $SeV^{18+}$LacZ/ΔF-1214, LacZ mRNA accumulation was started as late as ten hours after infection, and its amount was very small. In the case of $SeV^{18+}$LacZ/ΔF, the accumulation of LacZ mRNA reached a peak 22 hours after infection. On the other hand, in $SeV^{18+}$LacZ/ΔF-1214, LacZ mRNA was continuously accumulated in a proportional manner even 22 hours after infection. The $SeV^{18+}$LacZ/ΔF-derived LacZ mRNA, once accumulated, was decreased after the period of 22 hours; however, $SeV^{18+}$LacZ/ΔF still retained the ability to express LacZ (Fig. 15).

The accumulation of LacZ mRNA in cells infected with $SeV^{18+}$LacZ/ΔF-1214 was also significantly retarded at 32°C as compared to $SeV^{18+}$LacZ/ΔF. However, the accumulation of LacZ mRNA and SeV genome was detected 10 hours after infection, and did not reach a peak even 32 hours after infection (Fig. 16).

Industrial Applicability

The present inventors revealed that, for example when cells were infected with SeV/ΔF vector having Y1214F, both of the following occurred:

(1) The transcription was suppressed in the early infection period, and after that the reduction in the replication rate (reduced to 1/10) was more significant than the transcription suppression. The infected cells exhibited no cytotoxicity because of the impairment of transcriptional activity of L and ability of autonomous replication. Although the ability to express carried genes is inferior as compared to SeV/ΔF vector having wild type L, it is comparable or greater than the gene expression ability of the adenovirus vector. In addition, the expression was sustained for a long period. Furthermore, it was demonstrated that the expression of carried genes was maintained even after several passages of infected cells, and that phenylalanine at position 1214, which was the mutation site in the SeV genome, did not revert to wild type tyrosine. Specifically, the mutation in L enables to reduce the viral components in a well-balanced fashion while retaining the ability to express carried genes at a practically satisfactory level.

(2) Like SeV/ΔF having L without the mutation, SeV/ΔF vector having the mutant L could replicate at 32°C, which is equivalent to the temperature in the nasal cavity, a vaccine administration site. At 37°C, SeV/ΔF vector having the mutant L exhibited temperature sensitivity and thus almost no viral particles were produced. When mouse bone marrow mesenchymal cells were infected and cultured at 32°C, the gene expression was more stable and sustained for a longer period as compared to SeV/ΔF vector having wild type L.

These findings suggest the possibility of developing a gene expression system that hardly exhibits cytotoxicity and that allows stable, long-term expression, which has the advantage characteristic of Sendai virus that it can exist independently of the chromosome, by introducing Y1214F mutation of L into SeV/ΔF vectors that can be used as gene transfer vectors.

Furthermore, it was demonstrated that the amino acid sequence of RdRp is highly homologous among related viruses and tyrosine at position 1214 in L of SeV is evolutionarily well conserved among the RNA polymerases of the respective viruses. This finding supports that generality of the phenotype resulting from Y1214F mutation, which impairs the RNA polymerase activity. Specifically, there is an expectation that other viruses or viral vectors can be attenuated by introducing them with the same mutation as Y1214F mutation in L of Sendai virus. The mutation has the generality and stability so that it can also be used to develop, for example, vaccines such as against parainfluenza virus.

In addition, L protein is extremely well conserved among paramyxoviruses, and thus there is an expectation that Y1214F is used to attenuate other viruses such as human respiratory viruses against which vaccines are under development.

## Claims

1. An attenuated minus-strand RNA virus, comprising a gene encoding a mutant L protein in which a wild-type amino acid at a position corresponding to position 1214 in the amino acid sequence of SEQ ID NO: 1 has been substituted with another.

2. The minus-strand RNA virus of claim 1, wherein the attenuation is reduction in genome replication activity and/or transcription activity.

3. The minus-strand RNA virus of claim 1 or 2, wherein the substitution is of tyrosine with phenylalanine.

4. The minus-strand RNA virus of any one of claims 1 to 3, in which at least one or more of the genes encoding envelope-constituting proteins is deleted or inactivated.

5. The minus-strand RNA virus of claim 4, wherein the deleted or inactivated gene is any one of, or a combination of two or more of, the genes encoding F, HN, and M proteins.

6. The minus-strand RNA virus of any one of claims 1 to 5, which is a Paramyxoviridae virus.

7. The minus-strand RNA virus of claim 6, wherein the Paramyxoviridae virus is Sendai virus.

8. A viral vector comprising the minus-strand RNA virus of any one of claims 1 to 7.

9. The viral vector of claim 8, comprising a foreign gene in an expressible manner.

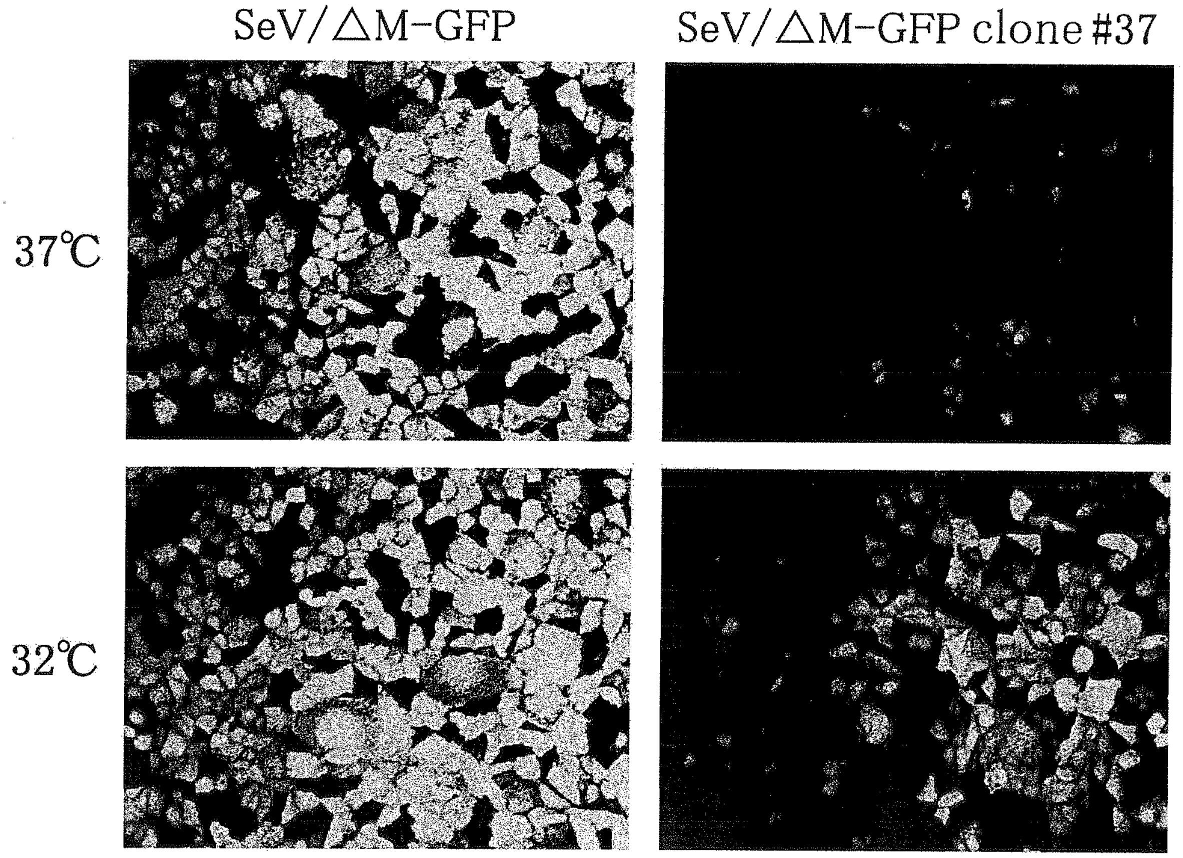


FIG. 1

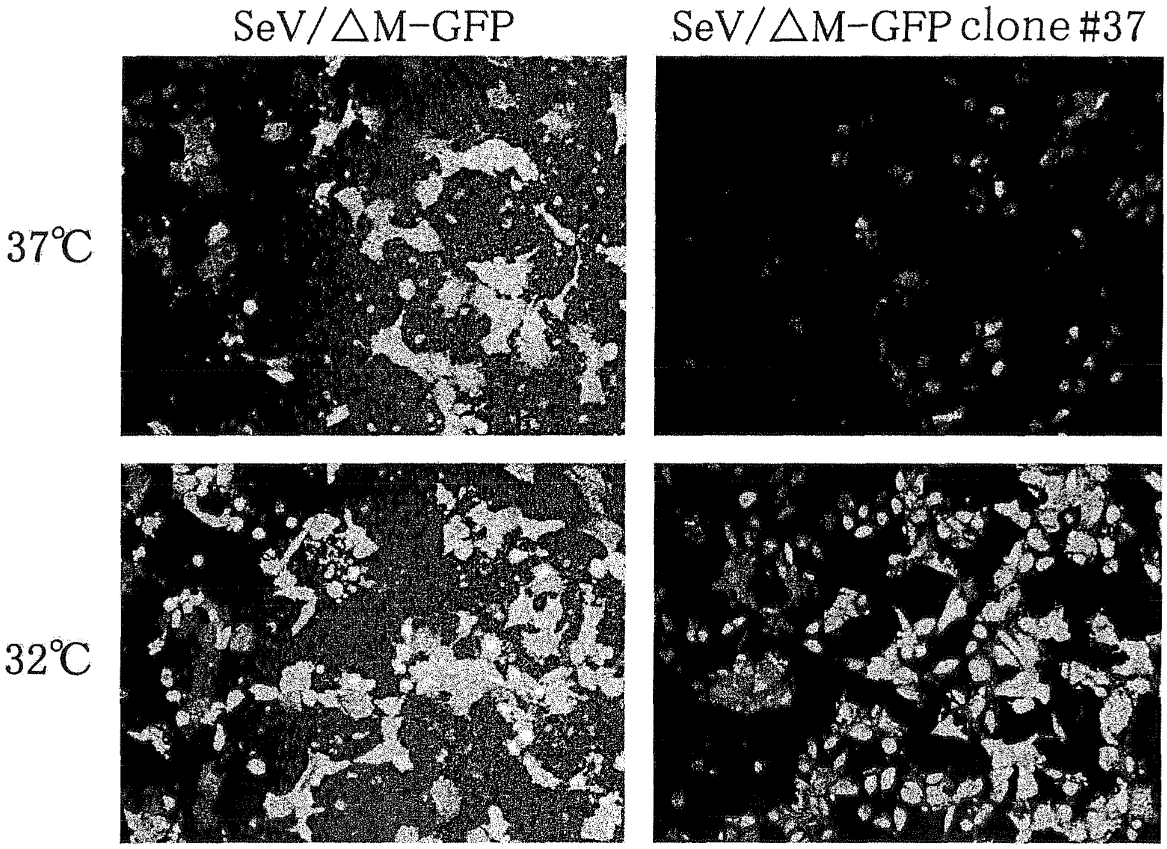


FIG. 2

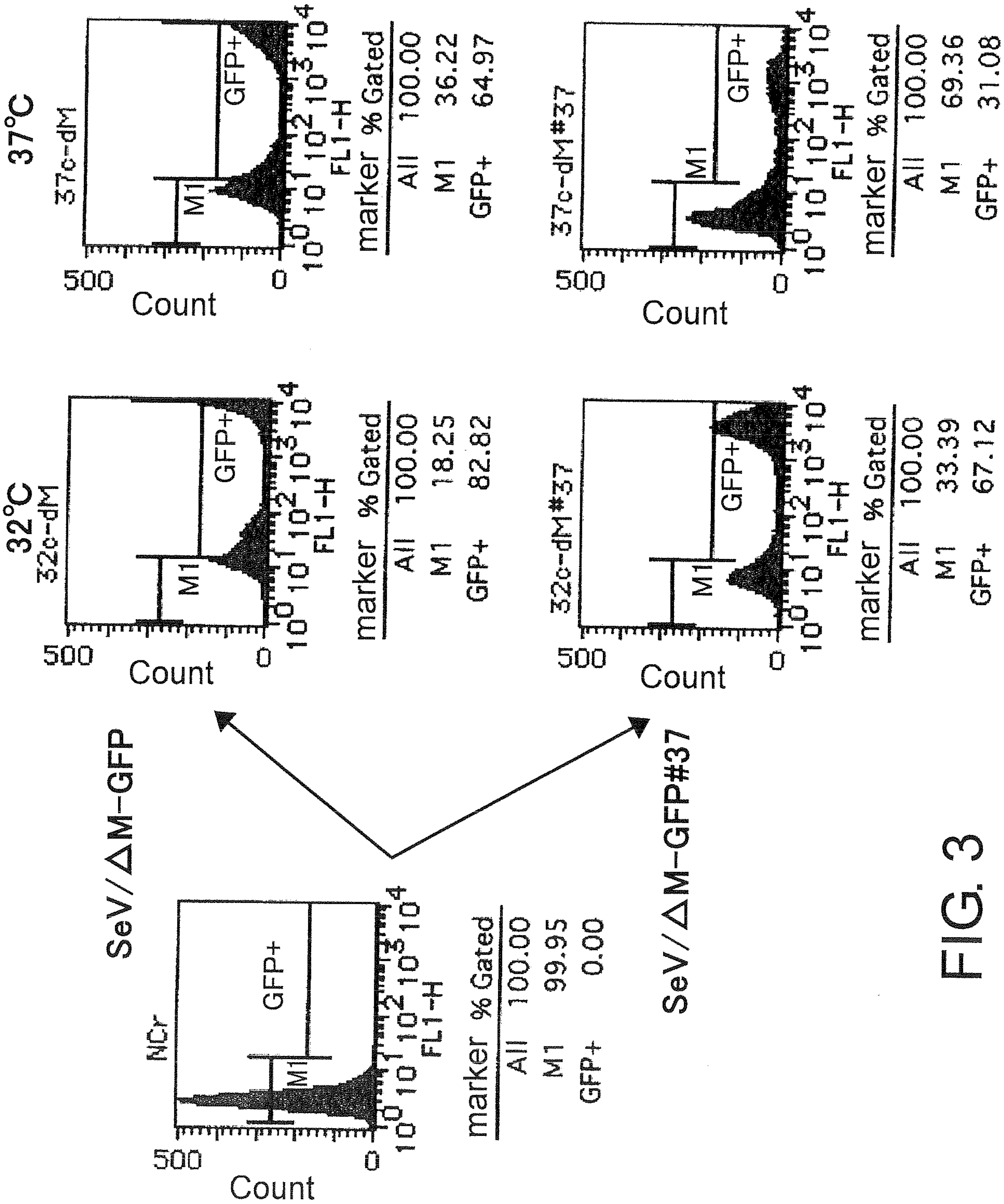

SeV/ΔM-GFP
SeV/ΔM-GFP#37
32°C
37°C
NCr
32c-dM
37c-dM
32c-dM#37
37c-dM#37
Count
500
0
FL1-H
M1
GFP+
marker % Gated
All 100.00
M1 99.95
GFP+ 0.00
All 100.00
M1 18.25
GFP+ 82.82
All 100.00
M1 36.22
GFP+ 64.97
All 100.00
M1 33.39
GFP+ 67.12
All 100.00
M1 69.36
GFP+ 31.08


FIG. 3

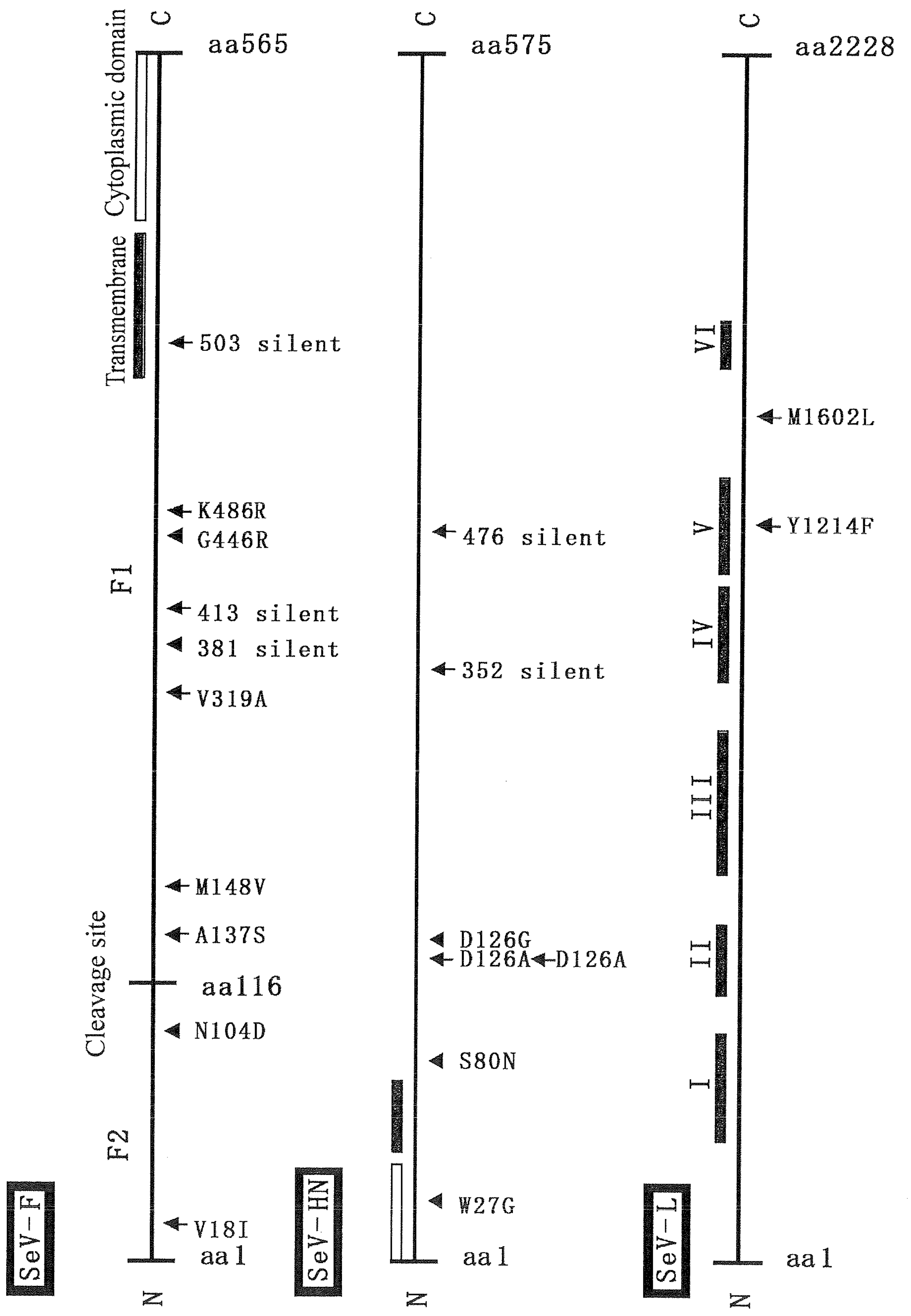
SeV-F
N
C
aa565
Cytoplasmic domain
Transmembrane
503 silent
K486R
G446R
F1
413 silent
381 silent
V319A
M148V
A137S
Cleavage site
aa116
N104D
F2
V18I
aa1
SeV-HN
N
C
aa575
476 silent
352 silent
D126G
D126A
D126A
S80N
W27G
aa1
SeV-L
N
C
aa2228
VI
M1602L
V
Y1214F
IV
III
II
I
aa1

FIG. 4

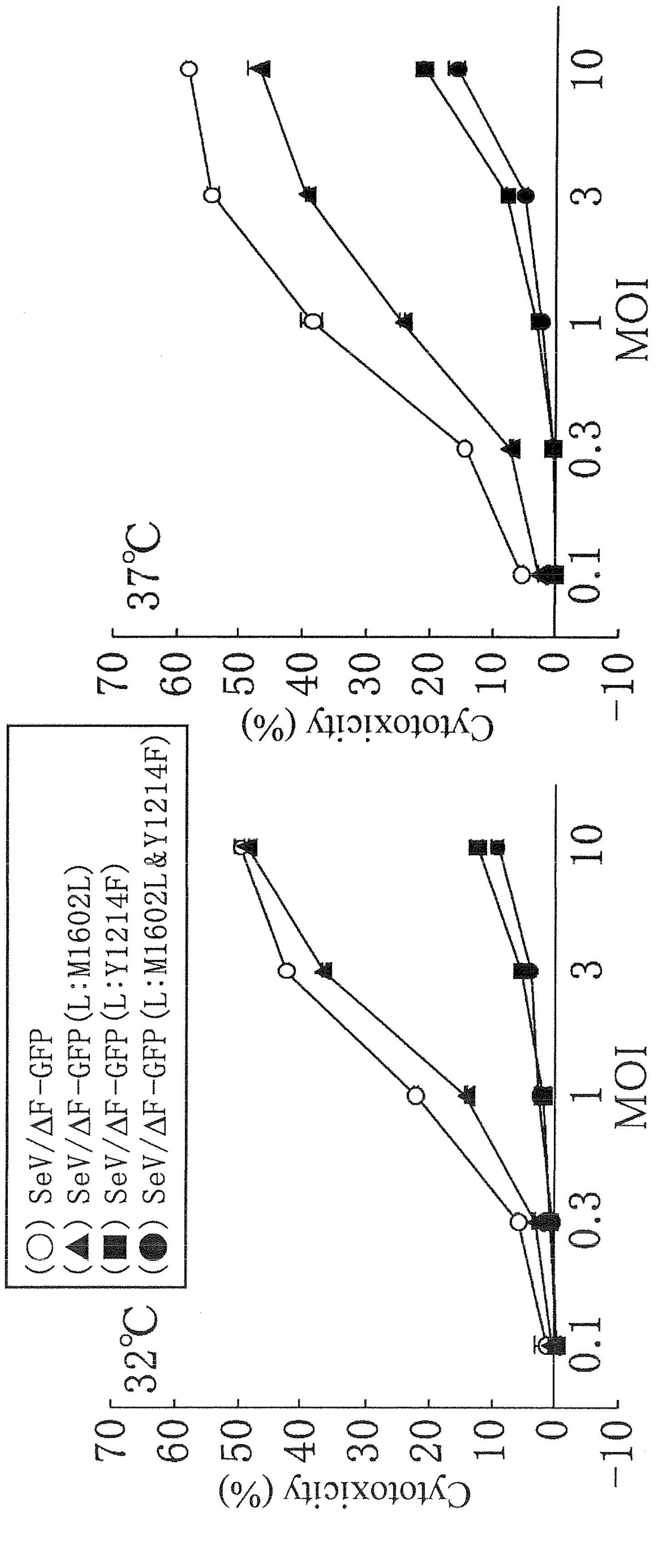
(○) SeV/ΔF-GFP
(▲) SeV/ΔF-GFP (L:M1602L)
(■) SeV/ΔF-GFP (L:Y1214F)
(●) SeV/ΔF-GFP (L:M1602L&Y1214F)
32°C
37°C
Cytotoxicity (%)
70
60
50
40
30
20
10
0
−10
MOI
0.1
0.3
1
3
10

FIG. 5

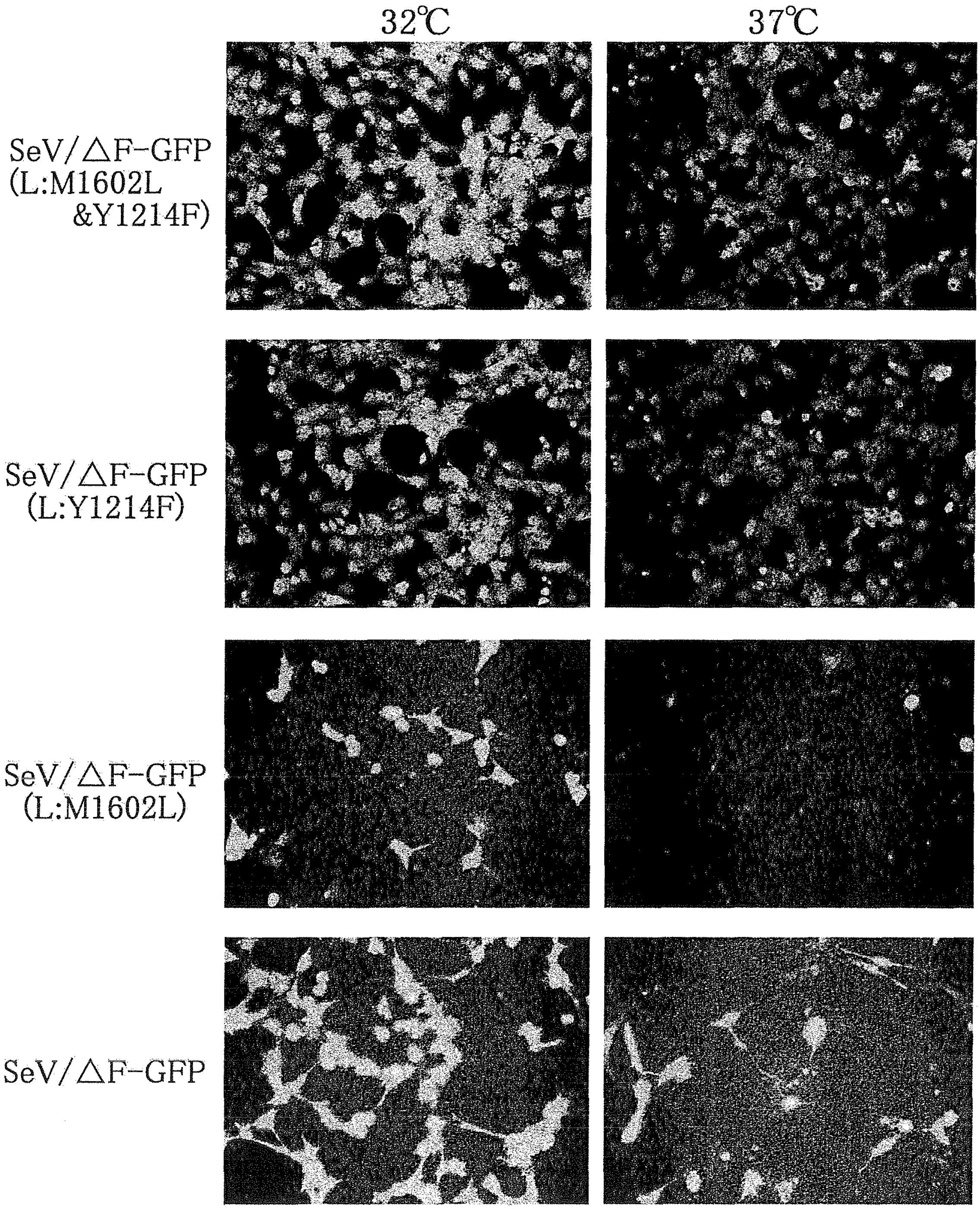
32℃
37℃
SeV/△F-GFP (L:M1602L &Y1214F)
SeV/△F-GFP (L:Y1214F)
SeV/△F-GFP (L:M1602L)
SeV/△F-GFP

FIG. 6

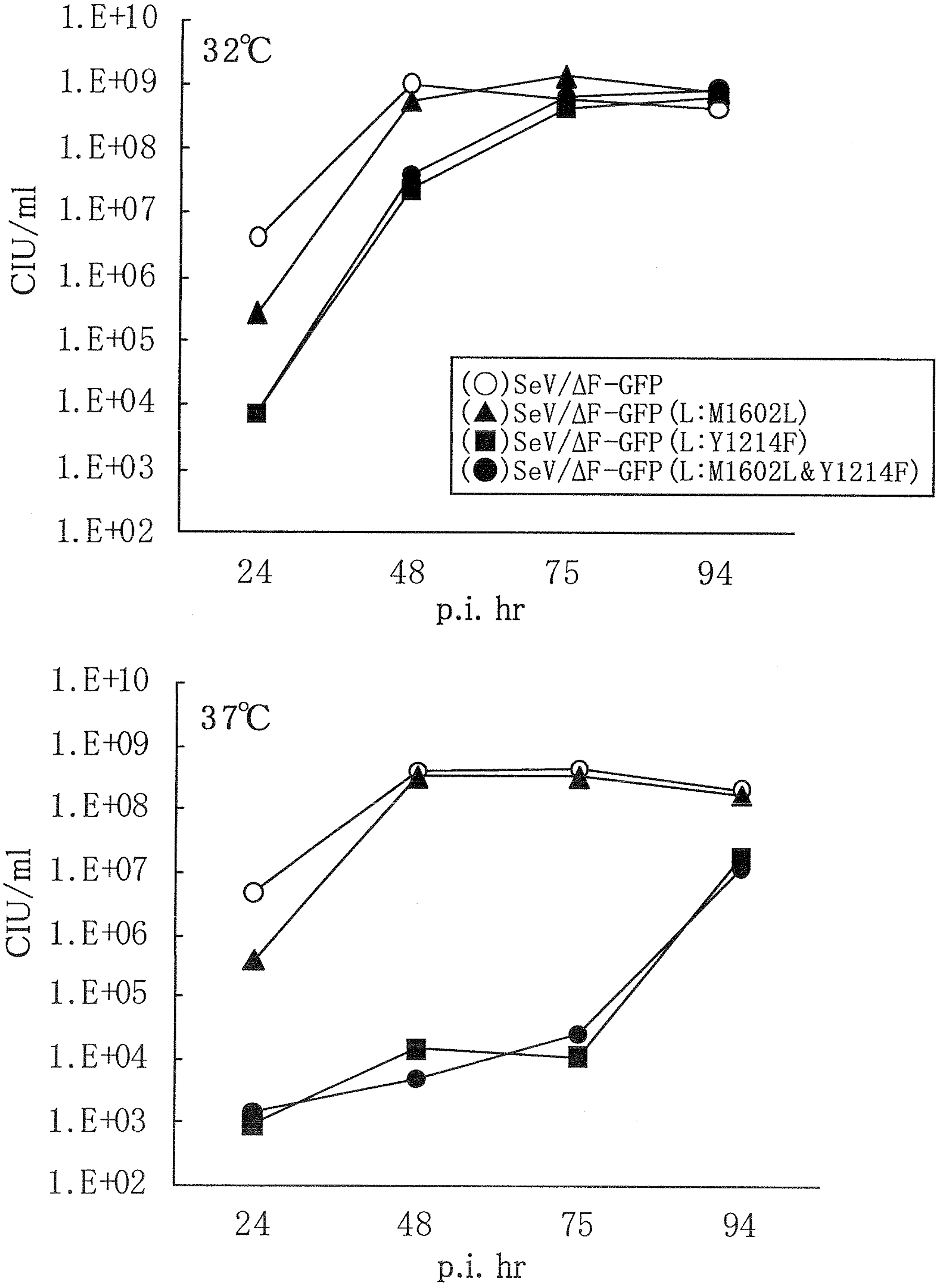
1.E+10
1.E+09
1.E+08
1.E+07
1.E+06
1.E+05
1.E+04
1.E+03
1.E+02
32℃
CIU/ml
(○)SeV/ΔF-GFP
(▲)SeV/ΔF-GFP(L:M1602L)
(■)SeV/ΔF-GFP(L:Y1214F)
(●)SeV/ΔF-GFP(L:M1602L&Y1214F)
24
48
75
94
p.i. hr
1.E+10
1.E+09
1.E+08
1.E+07
1.E+06
1.E+05
1.E+04
1.E+03
1.E+02
37℃
CIU/ml
24
48
75
94
p.i. hr

FIG. 7

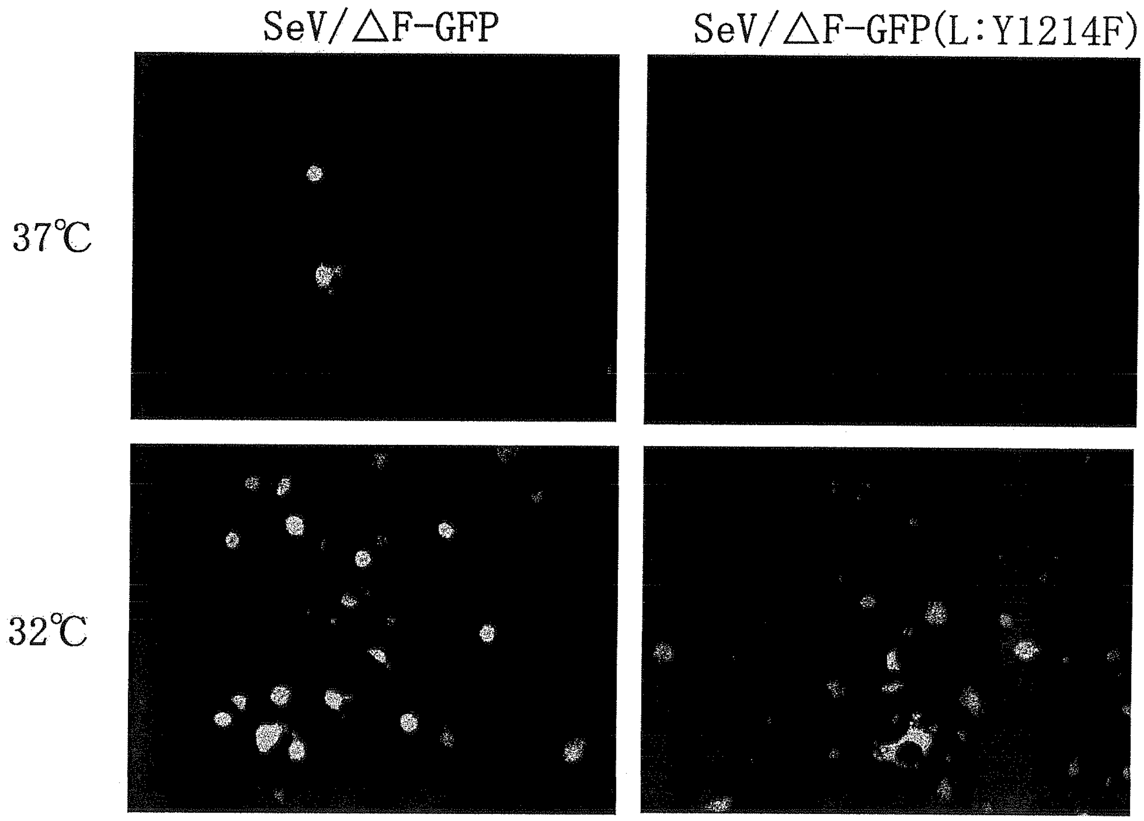


FIG. 8

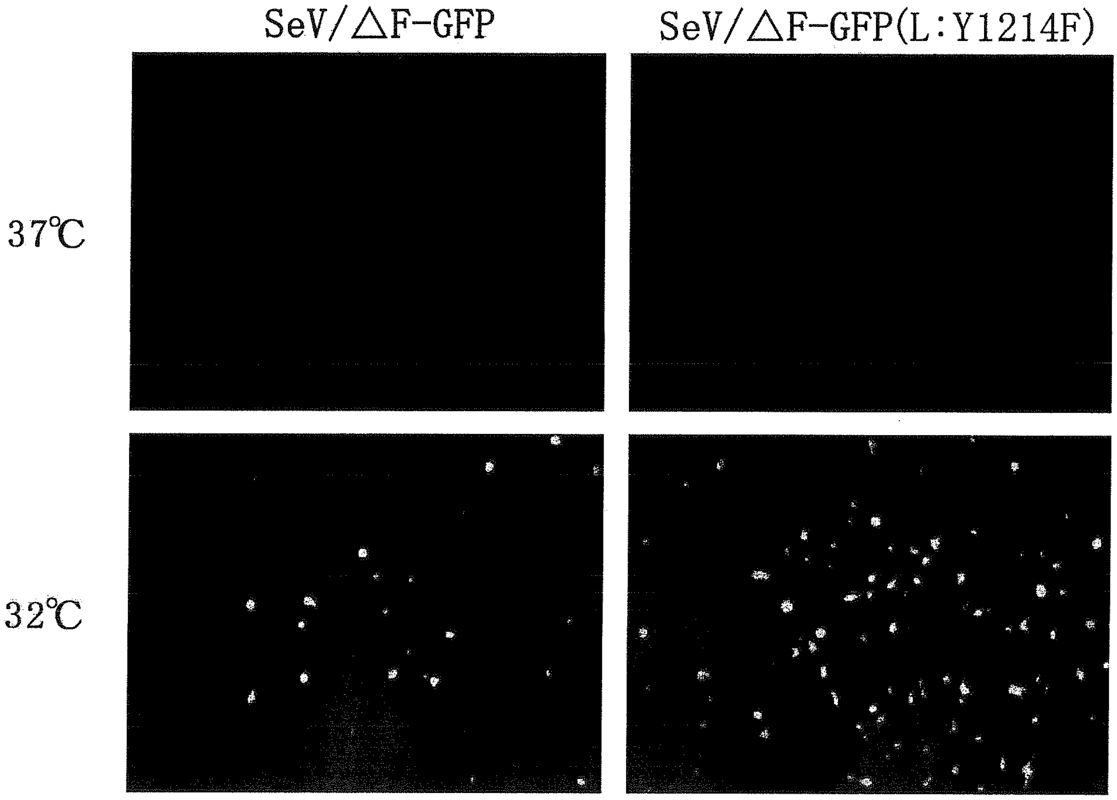


FIG. 9

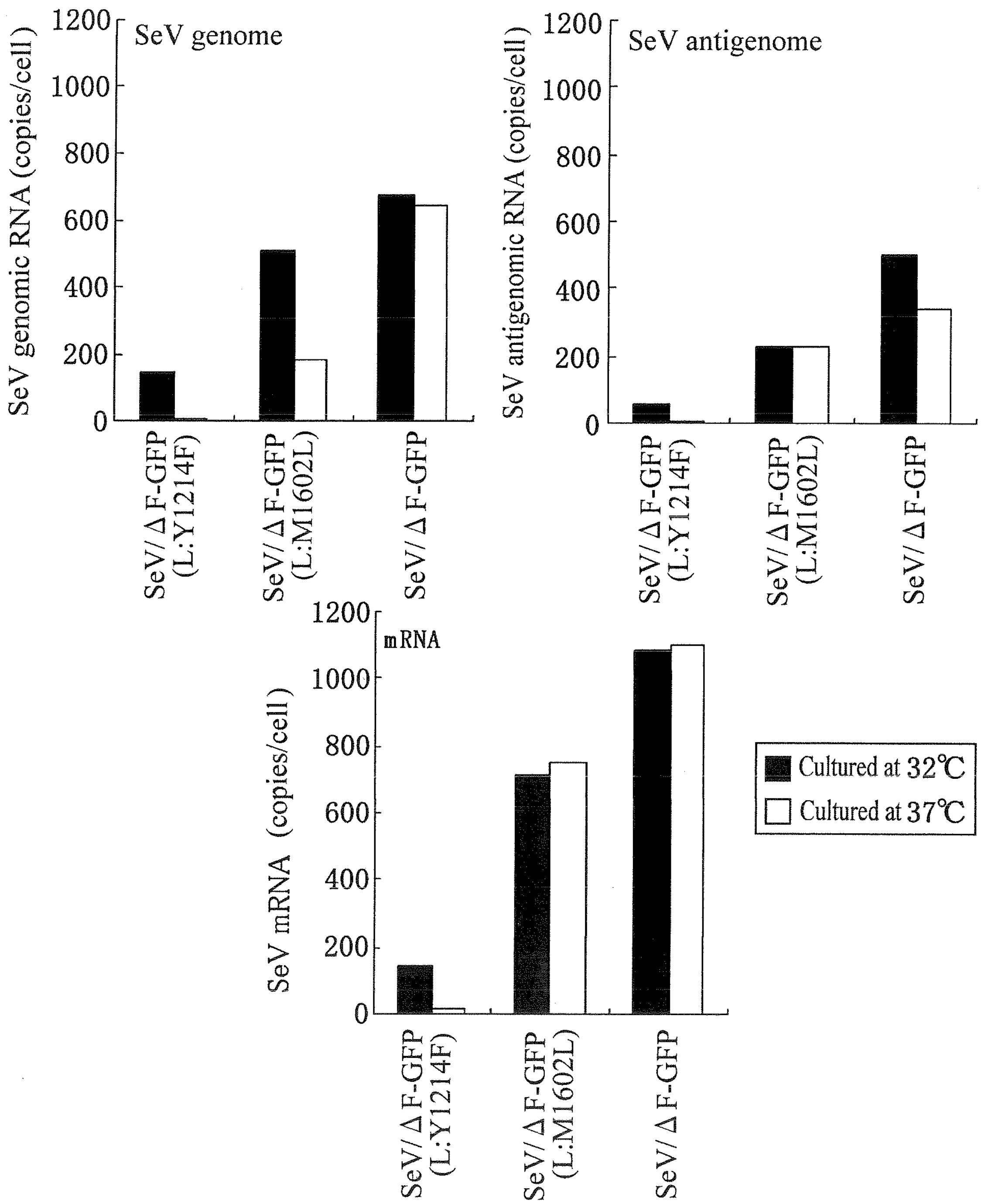
SeV genome
SeV genomic RNA (copies/cell)
1200
1000
800
600
400
200
0
SeV/Δ F-GFP (L:Y1214F)
SeV/Δ F-GFP (L:M1602L)
SeV/Δ F-GFP
SeV antigenome
SeV antigenomic RNA (copies/cell)
mRNA
SeV mRNA (copies/cell)
Cultured at 32℃
Cultured at 37℃

FIG. 10

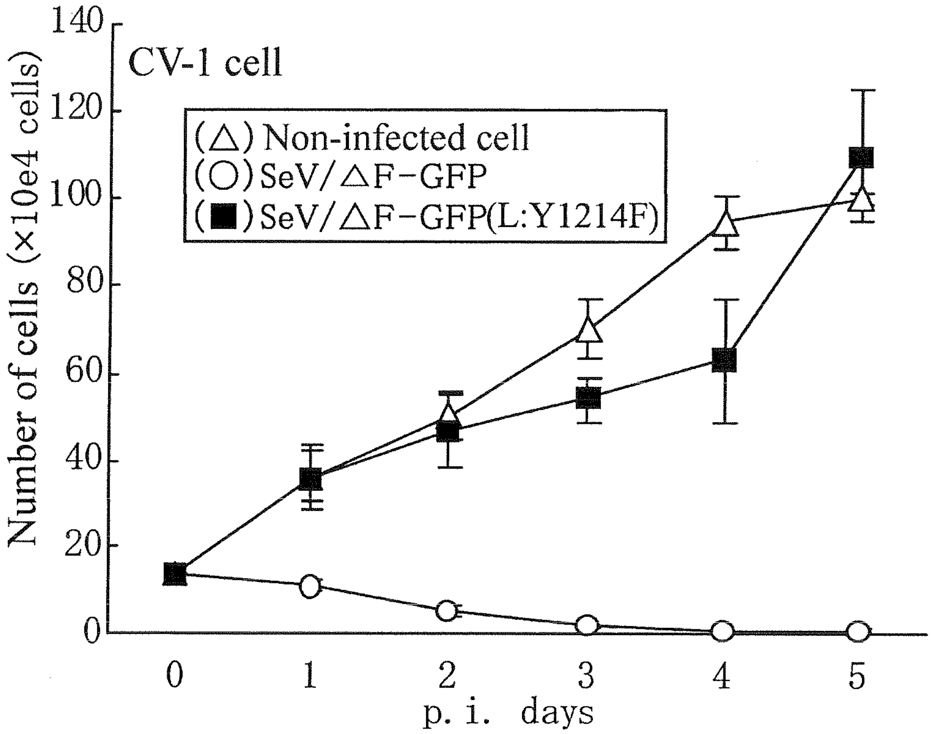
CV-1 cell
(△) Non-infected cell
(○) SeV/△F-GFP
(■) SeV/△F-GFP(L:Y1214F)
Number of cells (×10e4 cells)
140
120
100
80
60
40
20
0
0
1
2
3
4
5
p. i. days

FIG. 11

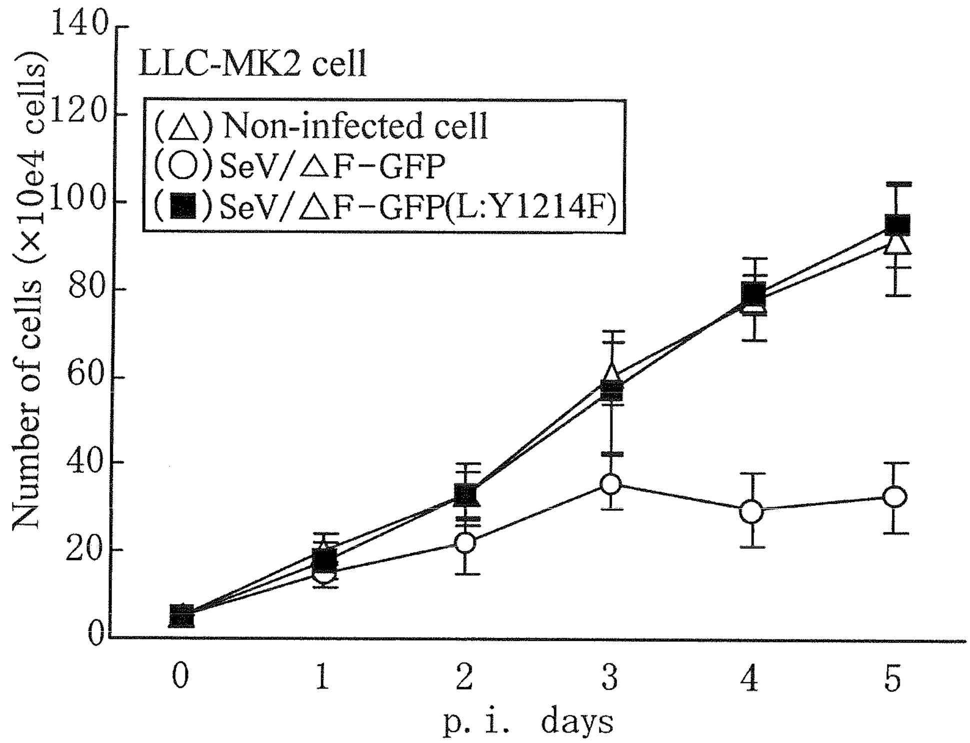
LLC-MK2 cell
(△) Non-infected cell
(○) SeV/△F-GFP
(■) SeV/△F-GFP(L:Y1214F)
Number of cells (×10e4 cells)
140
120
100
80
60
40
20
0
0
1
2
3
4
5
p. i. days

FIG. 12

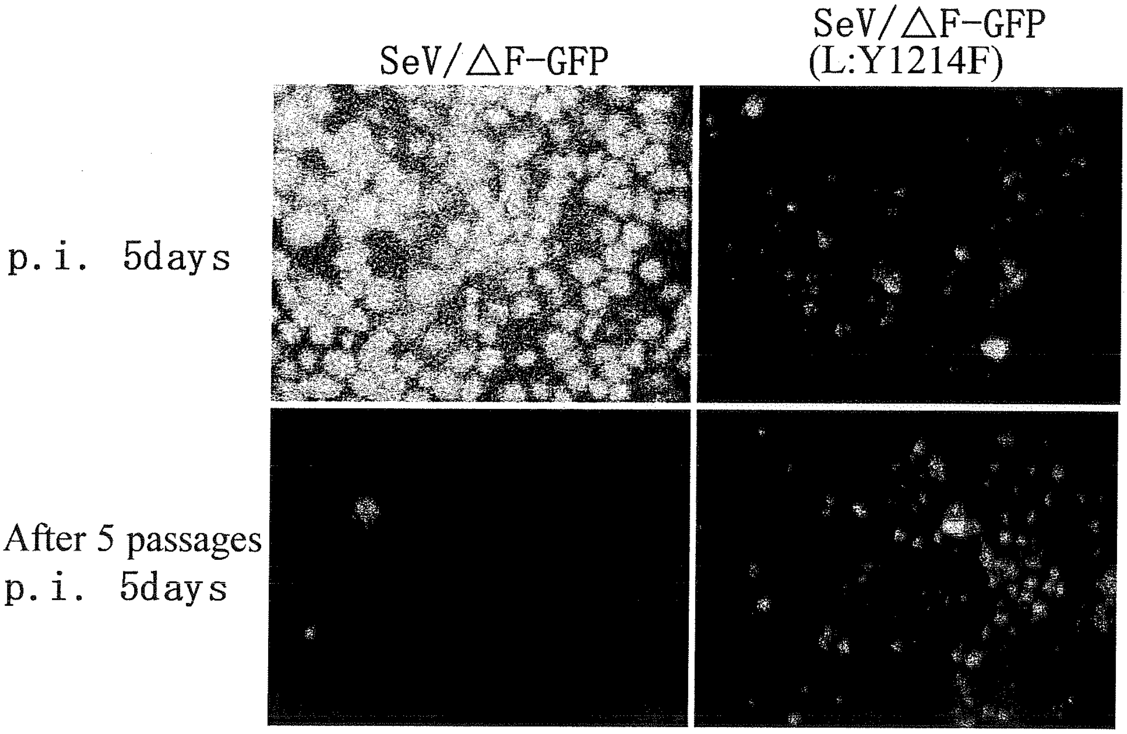


FIG. 13

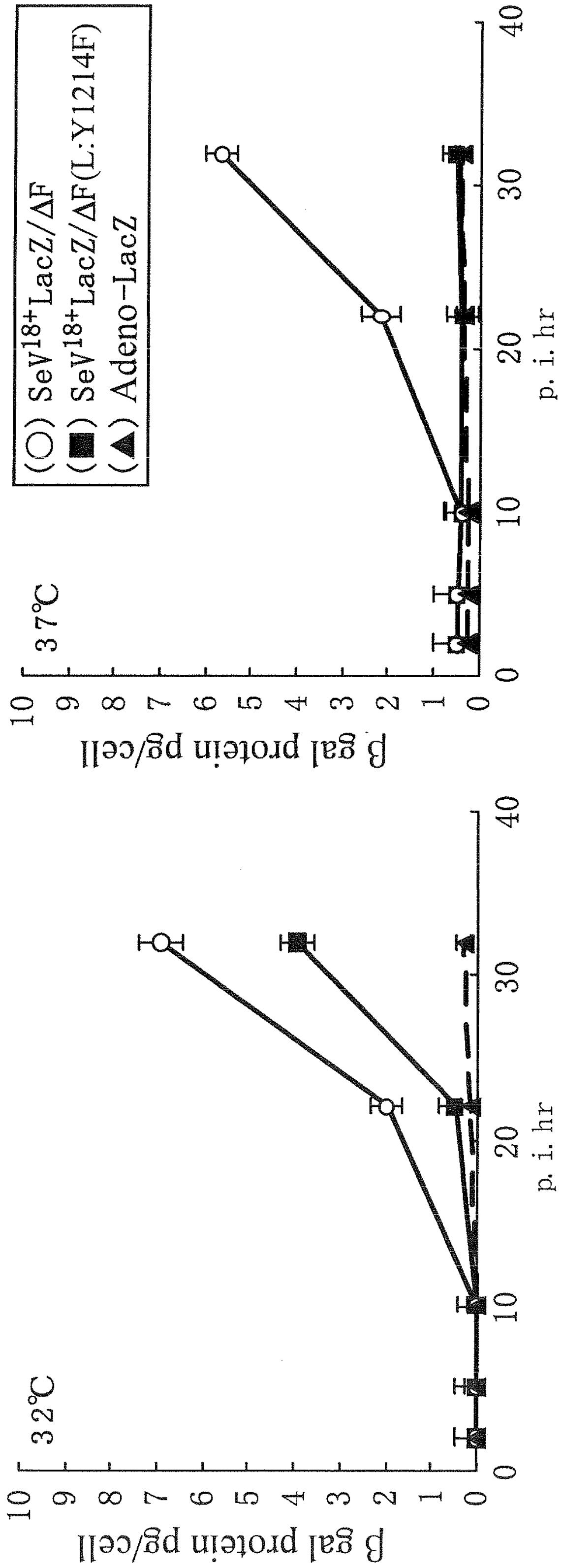
32°C
37°C
β gal protein pg/cell
p. i. hr
(○) SeV18+LacZ/ΔF
(■) SeV18+LacZ/ΔF(L:Y1214F)
(▲) Adeno-LacZ
0
1
2
3
4
5
6
7
8
9
10
0
10
20
30
40

FIG. 14

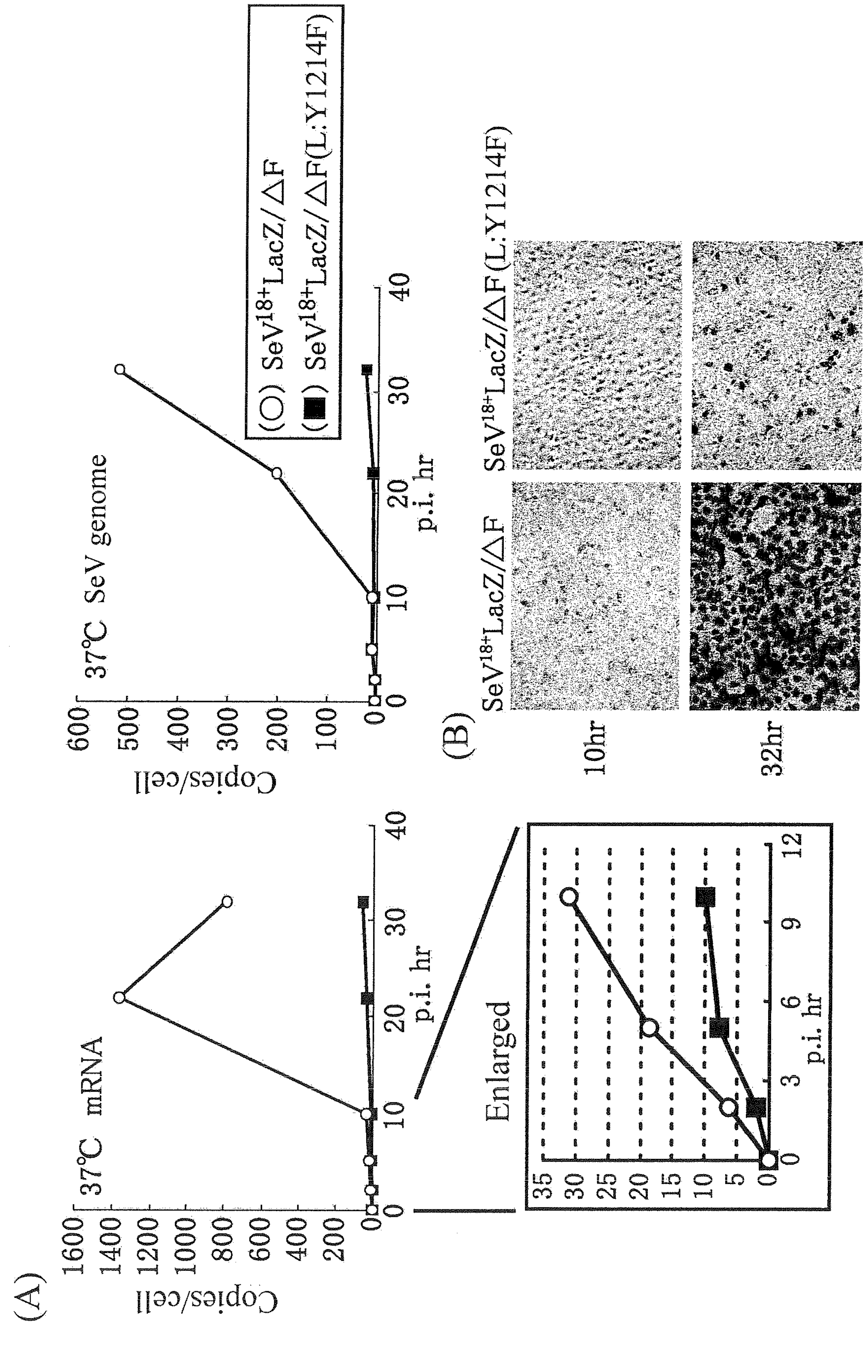
(A)
37℃ mRNA
Copies/cell
1600
1400
1200
1000
800
600
400
200
0
0
10
20
30
40
p.i. hr
Enlarged
35
30
25
20
15
10
5
0
0
3
6
9
12
p.i. hr
37℃ SeV genome
Copies/cell
600
500
400
300
200
100
0
0
10
20
30
40
p.i. hr
(○) SeV18+LacZ/ΔF
(■) SeV18+LacZ/ΔF(L:Y1214F)
(B)
SeV18+LacZ/ΔF
SeV18+LacZ/ΔF(L:Y1214F)
10hr
32hr

FIG. 15

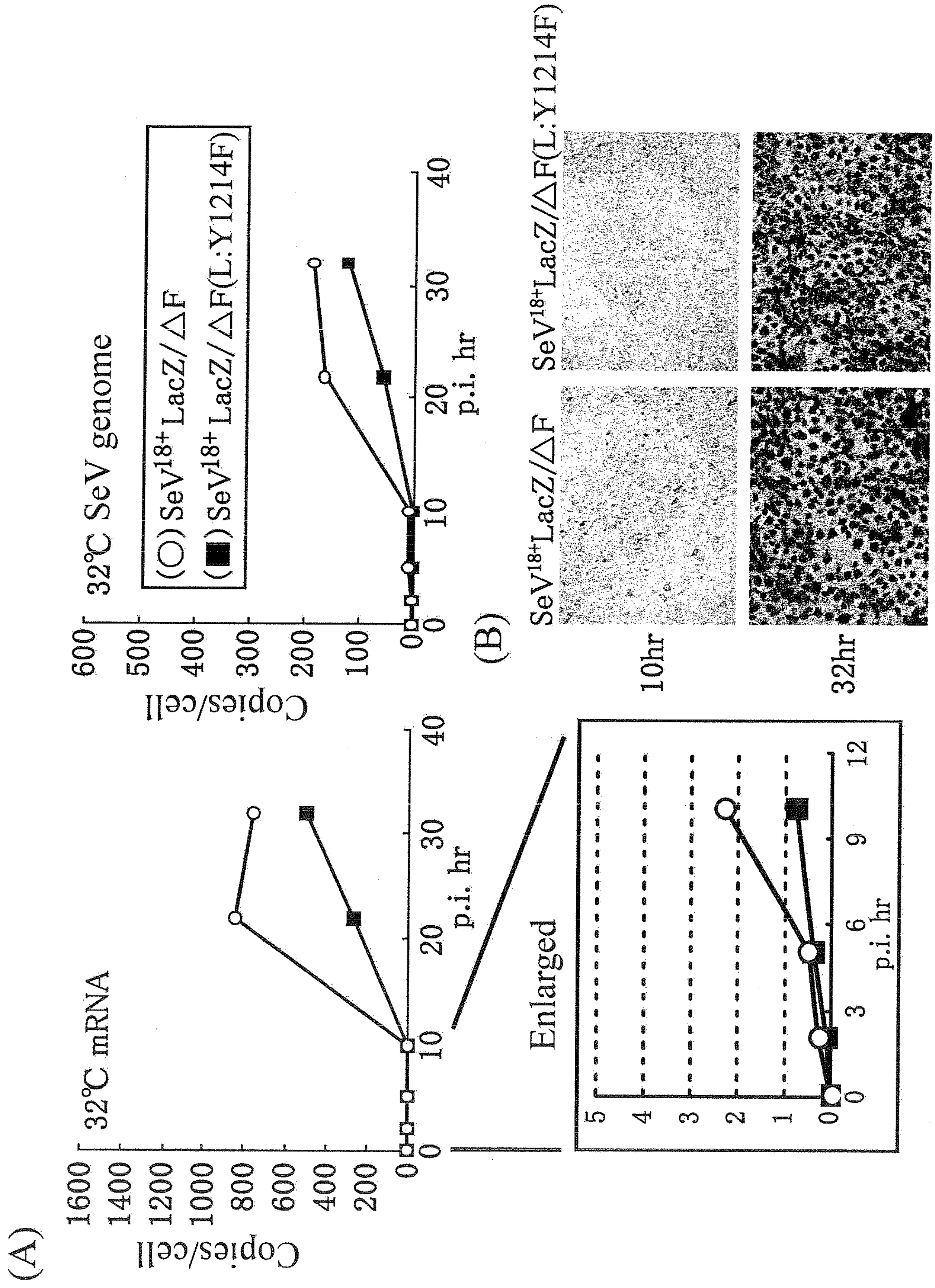
(A)
32°C mRNA
Copies/cell
1600
1400
1200
1000
800
600
400
200
0
0
10
20
30
40
p.i. hr
Enlarged
5
4
3
2
1
0
0
3
6
9
12
p.i. hr
32°C SeV genome
(○) SeV18+LacZ/ΔF
(■) SeV18+LacZ/ΔF(L:Y1214F)
Copies/cell
600
500
400
300
200
100
0
0
10
20
30
40
p.i. hr
(B)
SeV18+LacZ/ΔF
SeV18+LacZ/ΔF(L:Y1214F)
10hr
32hr

FIG. 16

**INTERNATIONAL SEARCH REPORT**

International application No.
PCT/JP2008/052016

A. CLASSIFICATION OF SUBJECT MATTER
*C12N15/09*(2006.01)i, *C12N7/00*(2006.01)i, *C07K14/08*(2006.01)n, *C07K14/115* (2006.01)n

According to International Patent Classification (IPC) or to both national classification and IPC

B. FIELDS SEARCHED

Minimum documentation searched (classification system followed by classification symbols)
C12N15/09, C12N7/00, C07K14/08, C07K14/115

Documentation searched other than minimum documentation to the extent that such documents are included in the fields searched

Electronic data base consulted during the international search (name of data base and, where practicable, search terms used)
BIOSIS/MEDLINE/WPIDS(STN), CA(STN), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X<br>Y | Fujii Y et al., Identification of mutations associated with attenuation of virulence of a field Sendai virus isolate by egg passage, Virus Genes, 2002, Vol.25, No.2, pp.189-193 | 1-9<br>4-9 |
| X<br>Y | Cortese CK et al., Mutations in domain V of the Sendai virus L polymerase protein uncouple transcription and replication and differentially affect replication in vitro and in vivo, Virology, 2000, Vol.277, No.2, pp.387-396 | 1-9<br>4-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 February, 2008 (28.02.08) | 11 March, 2008 (11.03.08) |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No. PCT/JP2008/052016

C (Continuation). DOCUMENTS CONSIDERED TO BE RELEVANT

| Category* | Citation of document, with indication, where appropriate, of the relevant passages | Relevant to claim No. |
|---|---|---|
| Y | Makoto INOUE et al., "M,F,HN Envelope Kanren 3 Idenshi Subete o Kesshitsu shita Sendai Virus Vector Sakushutsu no Seiko to Sono Seino Hyoka", Dai 52 Kai The Japanese Society of Virology Gakujutsu Shukai Program Shorokushu, 2004, pages 149 | 4-9 |
| A | Horikami SM et al., Alternative amino acids at a single site in the Sendai virus L protein produce multiple defects in RNA synthesis in vitro, Virology, 1995, Vol.211, No.2, pp.577-82 | 1-9 |
| A | Feller JA et al., Comparison of identical temperature-sensitive mutations in the L polymerase proteins of sendai and parainfluenza3 viruses, Virology, 2000, Vol.276, No.1, pp.190-201 | 1-9 |
| A | Itoh M et al., Isolation of an avirulent mutant of Sendai virus with two amino acid mutations from a highly virulent field strain through adaptation to LLC-MK2 cells, J. Gen. Virol., 1997, Vol.78, Pt.12, pp.3207-3215 | 1-9 |
| A | Newman JT et al., Generation of recombinant human parainfluenza virus type 1 vaccine candidates by importation of temperature-sensitive and attenuating mutations from heterologous paramyxoviruses, J. Virol., 2004, Vol.78, No.4, pp.2017-2028 | 1-9 |
| A | Bartlett EJ et al., Human parainfluenza virus type I (HPIV1) vaccine candidates designed by reverse genetics are attenuated and efficacious in African green monkeys, Vaccine, 2005, Vol.23, No.38, pp.4631-4646 | 1-9 |
| A | Smallwood S et al., Different substitutions at conserved amino acids in domains II and III in the Sendai L RNA polymerase protein inactivate viral RNA synthesis, Virology, 2002, Vol.304, No.1, pp.135-145 | 1-9 |
| A | Ito M et al., Early stage of establishment of persistent Sendai virus infection: unstable dynamic phase and then selection of viruses which are tightly cell associated, temperature sensitive, and capable of establishing persistent infection, J. Virol., 2004, Vol.78, No.21, pp.11939-11951 | 1-9 |

Form PCT/ISA/210 (continuation of second sheet) (April 2007)

**INTERNATIONAL SEARCH REPORT**

International application No.
PCT/JP2008/052016

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-325531 A (National Institute of Advanced Industrial Science and Technology), 07 December, 2006 (07.12.06), (Family: none) | 1-9 |
| A | WO 2000/70070 A1 (Dnavec Corp.), 23 November, 2000 (23.11.00), & US 2002/169306 A1 & EP 1186667 A1 | 1-9 |
| A | WO 2000/61737 A2 (THE GOVERNMENT OF THE UNITED STATES OF AMERICA ), 19 October, 2000 (19.10.00), & US 2005/287540 A1 & EP 1171623 A1 | 1-9 |
| P,A | WO 2007/044483 A2 (PRESIDENT AND FELLOWS OF HARVARD COLLEGE), 19 April, 2007 (19.04.07), (Family: none) | 1-9 |
| P,A | WO 2008/007581 A1 (Dnavec Corp.), 17 January, 2008 (17.01.08), (Family: none) | 1-9 |

Form PCT/ISA/210 (continuation of second sheet) (April 2007)

**INTERNATIONAL SEARCH REPORT**

International application No.
PCT/JP2008/052016

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: See extra sheet <A>
because they relate to parts of the international application that do not comply with the prescribed requirements to such an extent that no meaningful international search can be carried out, specifically:
See extra sheet <B>.

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

INTERNATIONAL SEARCH REPORT

International application No.
PCT/JP2008/052016

Continuation of Box No.II-2 of continuation of first sheet(2)

<A>
Parts of claims 1-6, 8 and 9 which relate to a virus other than Sendai virus.

<B>
The inventions of claims 1-5, 8 and 9 relate to "a minus-stranded RNA virus which has a gene encoding a mutant L-protein having the substitution of an amino acid residue located at Position-1214 in the amino acid sequence for a wild-type one depicted in SEQ ID NO:1 and which is attenuated". However, one which is disclosed in the meaning within PCT Article 5 is limited to "a Sendai virus which has a gene encoding a mutant L-protein having the substitution of an amino acid residue located at Position-1214 in the amino acid sequence for a wild-type one depicted in SEQ ID NO:1 and which is attenuated" as disclosed in the description. Therefore, these claims are not supported in the meaning within PCT Article 6.

The invention of claim 6 relates to "a virus belonging to the family *Paramyxoviridae* which has a gene encoding a mutant L-protein having the substitution of an amino acid residue located at Position-1214 in the amino acid sequence for a wild-type one depicted in SEQ ID NO:1 and which is attenuated". However, one which is disclosed in the meaning within PCT Article 5 is limited to "a Sendai virus which has a gene encoding a mutant L-protein having the substitution of an amino acid residue located at Position-1214 in the amino acid sequence for a wild-type one depicted in SEQ ID NO:1 and which is attenuated" as disclosed in the description. Therefore, this claim is not supported in the meaning within PCT Article 6.

Such being the case, the search was made on a scope which is supported by and disclosed in the description, namely, "a Sendai virus which has a gene encoding a mutant L-protein having the substitution of an amino acid residue located at Position-1214 in the amino acid sequence for a wild-type one depicted in SEQ ID NO:1 and which is attenuated" which is disclosed in the description specifically.

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0118223 A
- WO 0070055 A
- WO 0070070 A
- WO 03025570 A
- JP H3168087 A
- WO 0104272 A, Curran, J.
- EP 1067179 A
- WO 9716539 A
- WO 9716538 A
- WO 2005071092 A
- JP S6230752 B
- JP S6233879 B
- JP S6230753 B
- WO 9732010 A
- WO 200571085 A


### Non-patent literature cited in the description


- **Bukreyev, A. ; Skiadopoulos, M. H. ; Murphy, B. R. ; Collins, P. L.** Nonsegmented negative-strand viruses as vaccine vectors. *J Virol,* 2006, vol. 80, 10293-10306
- **Bastien, N. ; Ward, D. ; Van Caeseele, P. ; Brandt, K. ; Lee, S. H. ; McNabb, G. ; Klisko, B. ; Chan, E. ; Li, Y.** Human metapneumovirus infection in the Canadian population. *J Clin Microbiol,* 2003, vol. 41, 4642-4646
- **Boivin, G. ; Abed, Y ; Pelletier, G. ; Ruel, L. ; Moisan, D. ; Cote, S. ; Peret, T. C. ; Erdman, D. D. ; Anderson, L. J.** Virological features and clinical manifestations associated with human metapneumovirus: a new paramyxovirus responsible for acute respiratory-tract infections in all age groups. *J Infect Dis,* 2002, vol. 186, 1330-1334
- **Biacchesi, S. ; Skiadopoulos, M. H. ; Yang, L. ; Lamirande, E. W. ; Tran, K. C. ; Murphy, B. R. ; Collins, P. L. ; Buchholz, U. J.** Recombinant human Metapneumovirus lacking the small hydrophobic SH and/or attachment G glycoprotein: deletion of G yields a promising vaccine candidate. *J Virol,* 2004, vol. 78, 12877-12887
- **Whitehead, S. S. ; Bukreyev, A. ; Teng, M. N. ; Firestone, C. Y ; St Claire, M. ; Elkins, W. R. ; Collins, P. L. ; Murphy, B. R.** Recombinant respiratory syncytial virus bearing a deletion of either the NS2 or SH gene is attenuated in chimpanzees. *J Virol,* 1999, vol. 73, 3438-3442
- **Li, H. O. ; Zhu, Y F. ; Asakawa, M. ; Kuma, H. ; Hirata, T. ; Ueda, Y ; Lee, Y S. ; Fukumura, M. ; Iida, A. ; Kato, A. et al.** A cytoplasmic RNA vector derived from nontransmissible Sendai virus with efficient gene transfer and expression. *J Virol,* 2000, vol. 74, 6564-6569
- **Inoue, M. ; Tokusumi, Y ; Ban, H. ; Shirakura, M. ; Kanaya, T. ; Yoshizaki, M. ; Hironaka, T. ; Nagai, Y ; Iida, A. ; Hasegawa, M.** Recombinant Sendai virus vectors deleted in both the matrix and the fusion genes: efficient gene transfer with preferable properties. *J Gene Med,* 2004, vol. 6, 1069-1081
- **Yoshizaki, M. ; Hironaka, T. ; Iwasaki, H. ; Ban, H. ; Tokusumi, Y ; Iida, A. ; Nagai, Y ; Hasegawa, M. ; Inoue, M.** Naked Sendai virus vector lacking all of the envelope-related genes: reduced cytopathogenicity and immunogenicity. *J Gene Med,* 2006, vol. 8, 1151-1159
- **Skiadopoulos, M. H. ; Surman, S. ; Tatem, J. M. ; Paschalis, M. ; Wu, S. L. ; Udem, S. A. ; Durbin, A. P. ; Collins, P. L. ; Murphy, B. R.** Identification of mutations contributing to the temperature-sensitive, cold-adapted, and attenuation phenotypes of the live-attenuated cold-passage 45 (cp45) human parainfluenza virus 3 candidate vaccine. *J Virol,* 1999, vol. 73, 1374-1381
- **Haller, A. A. ; MacPhail, M. ; Mitiku, M. ; Tang, R. S.** A single amino acid substitution in the viral polymerase creates a temperature-sensitive and attenuated recombinant bovine parainfluenza virus type 3. *Virology,* 2001, vol. 288, 342-350
- **McAuliffe, J. M. ; Surman, S. R. ; Newman, J. T. ; Riggs, J. M. ; Collins, P. L. ; Murphy, B. R. ; Skiadopoulos, M. H.** Codon substitution mutations at two positions in the L polymerase protein of human parainfluenza virus type 1 yield viruses with a spectrum of attenuation in vivo and increased phenotypic stability in vitro. *J Virol,* 2004, vol. 78, 2029-2036

- **Bartlett, E. J. ; Amaro-Carambot, E. ; Surman, S. R. ; Newman, J. T. ; Collins, P. L. ; Murphy, B. R. ; Skiadopoulos, M. H.** Human parainfluenza virus type I (HPIV1) vaccine candidates designed by reverse genetics are attenuated and efficacious in African green monkeys. *Vaccine,* 2005, vol. 23, 4631-4646
- **Newman, J. T. ; Riggs, J. M. ; Surman, S. R. ; McAuliffe, J. M. ; Mulaikal, T. A. ; Collins, P. L. ; Murphy, B. R. ; Skiadopoulos, M. H.** Generation of recombinant human parainfluenza virus type 1 vaccine candidates by importation of temperature-sensitive and attenuating mutations from heterologous paramyxoviruses. *J Virol,* 2004, vol. 78, 2017-2028
- **Fujita, K. ; Silver, J. ; Peden, K.** Changes in both gp120 and gp41 can account for increased growth potential and expanded host range of human immunodeficiency virus type 1. *J Virol,* 1992, vol. 66, 4445-4451
- **Sawyer, L. S. ; Wrin, M. T. ; Crawford-Miksza, L. ; Potts, B. ; Wu, Y ; Weber, P. A. ; Alfonso, R. D. ; Hanson, C. V.** Neutralization sensitivity of human immunodeficiency virus type 1 is determined in part by the cell in which the virus is propagated. *J Virol,* 1994, vol. 68, 1342-1349
- **Graff, J. ; Kasang, C. ; Normann, A. ; Pfister-Hunt, M. ; Feinstone, S. M. ; Flehmig, B.** Mutational events in consecutive passages of hepatitis A virus strain GBM during cell culture adaptation. *Virology,* 1994, vol. 204, 60-68
- **Cao, J. X. ; Ni, H. ; Wills, M. R. ; Campbell, G. A. ; Sil, B. K. ; Ryman, K. D. ; Kitchen, I. ; Barrett, A. D.** Passage of Japanese encephalitis virus in HeLa cells results in attenuation of virulence in mice. *J Gen Virol,* 1995, vol. 76 (11), 2757-2764
- **Nagata, I. ; Kimura, Y. ; Ito, Y. ; Tanaka, T.** Temperature-sensitive phenomenon of viral maturation observed in BHK cells persistently infected with HVJ. *Virology,* 1972, vol. 49, 453-461
- **Yoshida, T. ; Nagai, Y. ; Maeno, K. ; Iinuma, M. ; Hamaguchi, M. ; Matsumoto, T. ; Nagayoshi, S. ; Hoshino, M.** Studies on the role of M protein in virus assembly using a ts mutant of HVJ (Sendai virus). *Virology,* 1979, vol. 92, 139-154
- **Itoh, M. ; Isegawa, Y ; Hotta, H. ; Homma, M.** Isolation of an avirulent mutant of Sendai virus with two amino acid mutations from a highly virulent field strain through adaptation to LLC-MK2 cells. *J Gen Virol,* 1997, vol. 78 (12), 3207-3215
- **Adachi, A. ; Kanda, T. ; Shibuta, H.** Isolation and characterization of temperature-sensitive mutants of Sendai virus. *Microbiol Immunol,* 1980, vol. 24, 1053-1068
- **Kanda, T. ; Shibuta, H.** Restricted viral RNA synthesis in establishment of persistent infection in Vero cells with a Sendai virus mutant. *Microbiol Immunol,* 1982, vol. 26, 1045-1055
- **Nishio, M. ; Tsurudome, M. ; Ito, M. ; Kawano, M. ; Komada, H. ; Ito, Y.** Characterization of Sendai virus persistently infected L929 cells and Sendai virus pi strain: recombinant Sendai viruses having Mpi protein shows lower cytotoxicity and are incapable of establishing persistent infection. *Virology,* 2003, vol. 314, 110-124
- **Nishio, M. ; Nagata, A. ; Tsurudome, M. ; Ito, M. ; Kawano, M. ; Komada, H. ; Ito, Y.** Recombinant Sendai viruses with L1618V mutation in their L polymerase protein establish persistent infection, but not temperature sensitivity. *Virology,* 2004, vol. 329, 289-301
- **Chandrika, R. et al.** *Virology,* 1995, vol. 213, 352-363
- **Cortese, C. K. et al.** *Virology,* 2000, vol. 277, 387-396
- **Feller, J. A. et al.** *Virology,* 2000, vol. 269, 426-439
- **Horikami, S. M. ; Moyer, S. A.** *Virology,* 1995, vol. 211, 577-582
- **Smallwood, S. et al.** *Virology,* 1999, vol. 262, 375-383
- **Smallwood, S. et al.** *Virology,* 2002, vol. 304, 135-145
- **Smallwood, S. et al.** *Virology,* 2002, vol. 304, 235-245
- **Yoshitake, J. et al.** *J Virol,* 2004, vol. 78, 8709-8719
- **Nishio, M. et al.** *Virology,* 2004, vol. 329, 289-301
- **Yurchak, L. K. et al.** *J Biol Chem,* 1996, vol. 271, 12549-12554
- **McAuliffe, J. M. et al.** *J Virol,* 2004, vol. 78, 2029-2036
- **Murphy, B. R. ; Collins, P. L.** *J Clin Invest,* 2002, vol. 110, 21-27
- **Malur, A. G. et al.** *Gene Expr,* 2002, vol. 10, 93-100
- **Schnell, M. J. ; Conzelmann, K. K.** *Virology,* 1995, vol. 214, 522-530
- **Ferron, F. et al.** *Trends Biochem Sci,* 2002, vol. 27, 222-224
- **Skiadopoulos, M. H. et al.** *J Virol,* 1999, vol. 73, 1374-1381
- **Haller, A. A. et al.** *Virology,* 2001, vol. 288, 342-350
- **Gubbay, O. et al.** *J Gen Virol,* 2001, vol. 82, 2895-2903
- **Yoshizaki, M. ; Hironaka, T. ; Iwasaki, H. ; Ban, H. ; Tokusumi, Y. ; Iida, A. ; Nagai, Y ; Hasegawa, M. ; Inoue, M.** Naked Sendai virus vector lacking all of the envelope-related genes: reduced cytopathogenicity and immunogenicity. *J Gene Med,* 2006, vol. 8, 1151-1159
- **Tokusumi, T. et al.** *Virus Res,* 2002, vol. 86, 33-38

- **Karlin S ; Altschul SF.** *Proc. Natl. Acad. Sci. USA,* 1990, vol. 87, 2264-2268
- **Karlin S ; Altschul SF.** *Proc. Natl. Acad Sci. USA,* vol. 90, 5873-587799993
- **Altschul SF et al.** *J. Mol. Biol.,* 1990, vol. 215, 403
- **Thompson JD et al.** *Nucleic Acids Res,* 1994, vol. 22, 4673-4680
- *J. Virol.,* 1994, vol. 68, 8413-8417
- **Deng, W. P. ; Nickoloff, J. A.** *Anal. Biochem.,* 1992, vol. 200, 81
- **Haught, C. et al.** *BioTechniques,* 1994, vol. 16 (1), 47-48
- **Zhu, L. ; Holtz, A.** *Methods Mol. Biol.,* 1996, vol. 57, 119-137
- **Zhu, L.** *Methods Mol. Biol.,* 1995, vol. 57, 13-29
- *Journal of Virology,* 1993, vol. 67 (8), 4822-4830
- **Anjeanette Robert et al.** *Virology,* 1998, vol. 247, 1-6
- **Li, H.-O. et al.** *J. Virol.,* 2000, vol. 74 (14), 6564-6569
- **Niwa, H. et al.** *Gene,* 1991, vol. 108, 193-199
- **Inoue, K. et al.** *J. Virol. Methods,* 2003, vol. 107, 229-236
- **Krieg, P.A. ; Melton, D.A.** *Methods Enzymol.,* 1987, vol. 155, 397-15
- **Milligan, J.F. et al.** *Nucleic Acids Res.,* vol. 15, 8783-798
- **Pokrovskaya, I.D. ; Gurevich, V.V.** *Anal. Biochem.,* 1994, vol. 220, 420-23
- **Fuerst, T.R. et al.** *Proc. Natl. Acad. Sci. USA,* 1986, vol. 83, 8122-8126
- **Hasan, M. K. et al.** *J. Gen. Virol.,* 1997, vol. 78, 2813-2820
- **Kato, A. et al.** *EMBO J.,* 1997, vol. 16, 578-587
- **Yu, D. et al.** *Genes Cells,* 1997, vol. 2, 457-466
- *J. Virology,* 1981, vol. 39, 519-528
- **Hirata, T. et al.** *J. Virol. Methods,* 2002, vol. 104, 125-133
- **Inoue, M. et al.** *J. Virol.,* 2003, vol. 77, 6419-6429
- **Inoue M. et al.** *J Gene Med.,* 2004, vol. 6, 1069-1081
- **Li, H.-O. et al.** *J.Virol.,* 2000, vol. 74, 6564-6569
- **Inoue, M. et al.** *J.Virol.,* 2003, vol. 77, 6419-6429
- **Hasan, M. K. et al.** *J. General Virology,* 1997, vol. 78, 2813-2820
- **Arai, T. et al.** *J. Virology,* 1998, vol. 72, 1115-1121
- **Saito et al.** *Nucl. Acids Res.,* 1995, vol. 23, 3816-3821
- **Arai, T. et al.** *J. Virol,* 1998, vol. 72, 1115-1121
- **Kato, A. et al.** *J. Virol.,* 1997, vol. 71, 7266-7272
- **Durbin, A. P. et al.** *Virology,* 1997, vol. 235, 323-332
- **Whelan, S. P. et al.** *Proc. Natl. Acad. Sci. USA,* 1995, vol. 92, 8388-8392
- **Schnell. M. J. et al.** *EMBO J.,* 1994, vol. 13, 4195-4203
- **Radecke, F. et al.** *EMBO J.,* 1995, vol. 14, 5773-5784
- **Lawson, N. D. et al.** *Proc. Natl. Acad. Sci. USA,* vol. 92, 4477-4481
- **Garcin, D. et al.** *EMBO J.,* 1995, vol. 14, 6087-6094
- **Kato, A. et al.** *Genes Cells,* 1996, vol. 1, 569-579
- **Baron, M. D. ; Barrett, T.** *J. Virol.,* 1997, vol. 71, 1265-1271
- **Bridgen, A. ; Elliott, R. M.** *Proc. Natl. Acad. Sci. USA,* vol. 93, 15400-15404
- **Tokusumi, T. et al.** *Virus Res.,* 2002, vol. 86, 33-38
- **Li, H.-O. et al.** *J. Virol.,* 2000, vol. 74, 6564-6569
- State-of-the-Art Technology Protocol in Neuroscience Research III, Molecular Neuron Physiology. Koseisha, 1993, 153-172
- **Tashiro, M.** Virus Experiment Protocol. Medical View Co., Ltd, 1995, 68-73
- **Li, H. O. ; Zhu, Y F. ; Asakawa, M. ; Kuma, H. ; Hirata, T. ; Ueda, Y. ; Lee, Y. S. ; Fukumura, M. ; Iida, A. ; Kato, A. et al.** *J Virol,* 2000, vol. 74, 6564-6569
- **Inoue, M. ; Tokusumi, Y. ; Ban, H. ; Kanaya, T. ; Shirakura, M. ; Tokusumi, T. ; Hirata, T. ; Nagai, Y. ; Iida, A. ; Hasegawa, M.** *J Virol,* 2003, vol. 77, 6419-6429
- **Inoue, M. ; Tokusumi, Y. ; Ban, H. ; Shirakura, M. ; Kanaya, T. ; Yoshizaki, M. ; Hironaka, T. ; Nagai, Y. ; Iida, A. ; Hasegawa, M.** *J Gene Med,* 2004, vol. 6, 1069-1081
- **Decker, T. ; Lohmann-Matthes, M. L.** *J Immunol Methods,* 1988, vol. 115, 61-69
- **Gough, N. M.** *Anal Biochem,* 1988, vol. 173, 93-95

SEQUENCE LISTING

<110> DNAVEC CORPORATION

<120> ATTENUATED MINUS-STRANDED RNA VIRUS

<130> R2403 EP S3

<140> EP 08 71 0906.2
<141> 2008-02-07

<150> JP 2007-027520
<151> 2007-02-07

<160> 14

<170> PatentIn version 3.3

<210> 1
<211> 2228
<212> PRT
<213> Sendai virus

<400> 1

```
Met Asp Gly Gln Glu Ser Ser Gln Asn Pro Ser Asp Ile Leu Tyr Pro
1               5                   10                  15

Glu Cys His Leu Asn Ser Pro Ile Val Arg Gly Lys Ile Ala Gln Leu
            20                  25                  30

His Val Leu Leu Asp Val Asn Gln Pro Tyr Arg Leu Lys Asp Asp Ser
        35                  40                  45

Ile Ile Asn Ile Thr Lys His Lys Ile Arg Asn Gly Gly Leu Ser Pro
    50                  55                  60

Arg Gln Ile Lys Ile Arg Ser Leu Gly Lys Ala Leu Gln Arg Thr Ile
65                  70                  75                  80

Lys Asp Leu Asp Arg Tyr Thr Phe Glu Pro Tyr Pro Thr Tyr Ser Gln
                85                  90                  95

Glu Leu Leu Arg Leu Asp Ile Pro Glu Ile Cys Asp Lys Ile Arg Ser
            100                 105                 110

Val Phe Ala Val Ser Asp Arg Leu Thr Arg Glu Leu Ser Ser Gly Phe
        115                 120                 125

Gln Asp Leu Trp Leu Asn Ile Phe Lys Gln Leu Gly Asn Ile Glu Gly
    130                 135                 140
```

```
Arg Glu Gly Tyr Asp Pro Leu Gln Asp Ile Gly Thr Ile Pro Glu Ile
145                 150                 155                 160

Thr Asp Lys Tyr Ser Arg Asn Arg Trp Tyr Arg Pro Phe Leu Thr Trp
                165                 170                 175

Phe Ser Ile Lys Tyr Asp Met Arg Trp Met Gln Lys Thr Arg Pro Gly
            180                 185                 190

Gly Pro Leu Asp Thr Ser Asn Ser His Asn Leu Leu Glu Cys Lys Ser
        195                 200                 205

Tyr Thr Leu Val Thr Tyr Gly Asp Leu Val Met Ile Leu Asn Lys Leu
    210                 215                 220

Thr Leu Thr Gly Tyr Ile Leu Thr Pro Glu Leu Val Leu Met Tyr Cys
225                 230                 235                 240

Asp Val Val Glu Gly Arg Trp Asn Met Ser Ala Ala Gly His Leu Asp
                245                 250                 255

Lys Lys Ser Ile Gly Ile Thr Ser Lys Gly Glu Glu Leu Trp Glu Leu
            260                 265                 270

Val Asp Ser Leu Phe Ser Ser Leu Gly Glu Glu Ile Tyr Asn Val Ile
        275                 280                 285

Ala Leu Leu Glu Pro Leu Ser Leu Ala Leu Ile Gln Leu Asn Asp Pro
    290                 295                 300

Val Ile Pro Leu Arg Gly Ala Phe Met Arg His Val Leu Thr Glu Leu
305                 310                 315                 320

Gln Thr Val Leu Thr Ser Arg Asp Val Tyr Thr Asp Ala Glu Ala Asp
                325                 330                 335

Thr Ile Val Glu Ser Leu Leu Ala Ile Phe His Gly Thr Ser Ile Asp
            340                 345                 350

Glu Lys Ala Glu Ile Phe Ser Phe Phe Arg Thr Phe Gly His Pro Ser
        355                 360                 365

Leu Glu Ala Val Thr Ala Ala Asp Lys Val Arg Ala His Met Tyr Ala
    370                 375                 380
```

```
Gln Lys Ala Ile Lys Leu Lys Thr Leu Tyr Glu Cys His Ala Val Phe
385                 390                 395                 400

Cys Thr Ile Ile Ile Asn Gly Tyr Arg Glu Arg His Gly Gly Gln Trp
                405                 410                 415

Pro Pro Cys Asp Phe Pro Asp His Val Cys Leu Glu Leu Arg Asn Ala
            420                 425                 430

Gln Gly Ser Asn Thr Ala Ile Ser Tyr Glu Cys Ala Val Asp Asn Tyr
        435                 440                 445

Thr Ser Phe Ile Gly Phe Lys Phe Arg Lys Phe Ile Glu Pro Gln Leu
    450                 455                 460

Asp Glu Asp Leu Thr Ile Tyr Met Lys Asp Lys Ala Leu Ser Pro Arg
465                 470                 475                 480

Lys Glu Ala Trp Asp Ser Val Tyr Pro Asp Ser Asn Leu Tyr Tyr Lys
                485                 490                 495

Ala Pro Glu Ser Glu Glu Thr Arg Arg Leu Ile Glu Val Phe Ile Asn
            500                 505                 510

Asp Glu Asn Phe Asn Pro Glu Glu Ile Ile Asn Tyr Val Glu Ser Gly
        515                 520                 525

Asp Trp Leu Lys Asp Glu Glu Phe Asn Ile Ser Tyr Ser Leu Lys Glu
    530                 535                 540

Lys Glu Ile Lys Gln Glu Gly Arg Leu Phe Ala Lys Met Thr Tyr Lys
545                 550                 555                 560

Met Arg Ala Val Gln Val Leu Ala Glu Thr Leu Leu Ala Lys Gly Ile
                565                 570                 575

Gly Glu Leu Phe Ser Glu Asn Gly Met Val Lys Gly Glu Ile Asp Leu
            580                 585                 590

Leu Lys Arg Leu Thr Thr Leu Ser Val Ser Gly Val Pro Arg Thr Asp
        595                 600                 605

Ser Val Tyr Asn Asn Ser Lys Ser Ser Glu Lys Arg Asn Glu Gly Met
    610                 615                 620
```

```
Glu Asn Lys Asn Ser Gly Gly Tyr Trp Asp Glu Lys Lys Arg Ser Arg
625                 630                 635                 640

His Glu Phe Lys Ala Thr Asp Ser Ser Thr Asp Gly Tyr Glu Thr Leu
                645                 650                 655

Ser Cys Phe Leu Thr Thr Asp Leu Lys Lys Tyr Cys Leu Asn Trp Arg
            660                 665                 670

Phe Glu Ser Thr Ala Leu Phe Gly Gln Arg Cys Asn Glu Ile Phe Gly
        675                 680                 685

Phe Lys Thr Phe Phe Asn Trp Met His Pro Val Leu Glu Arg Cys Thr
    690                 695                 700

Ile Tyr Val Gly Asp Pro Tyr Cys Pro Val Ala Asp Arg Met His Arg
705                 710                 715                 720

Gln Leu Gln Asp His Ala Asp Ser Gly Ile Phe Ile His Asn Pro Arg
                725                 730                 735

Gly Gly Ile Glu Gly Tyr Cys Gln Lys Leu Trp Thr Leu Ile Ser Ile
            740                 745                 750

Ser Ala Ile His Leu Ala Ala Val Arg Val Gly Val Arg Val Ser Ala
        755                 760                 765

Met Val Gln Gly Asp Asn Gln Ala Ile Ala Val Thr Ser Arg Val Pro
    770                 775                 780

Val Ala Gln Thr Tyr Lys Gln Lys Lys Asn His Val Tyr Glu Glu Ile
785                 790                 795                 800

Thr Lys Tyr Phe Gly Ala Leu Arg His Val Met Phe Asp Val Gly His
                805                 810                 815

Glu Leu Lys Leu Asn Glu Thr Ile Ile Ser Ser Lys Met Phe Val Tyr
            820                 825                 830

Ser Lys Arg Ile Tyr Tyr Asp Gly Lys Ile Leu Pro Gln Cys Leu Lys
        835                 840                 845

Ala Leu Thr Lys Cys Val Phe Trp Ser Glu Thr Leu Val Asp Glu Asn
    850                 855                 860
```

Arg Ser Ala Cys Ser Asn Ile Ser Thr Ser Ile Ala Lys Ala Ile Glu
865 870 875 880

Asn Gly Tyr Ser Pro Ile Leu Gly Tyr Cys Ile Ala Leu Tyr Lys Thr
885 890 895

Cys Gln Gln Val Cys Ile Ser Leu Gly Met Thr Ile Asn Pro Thr Ile
900 905 910

Ser Pro Thr Val Arg Asp Gln Tyr Phe Lys Gly Lys Asn Trp Leu Arg
915 920 925

Cys Ala Val Leu Ile Pro Ala Asn Val Gly Gly Phe Asn Tyr Met Ser
930 935 940

Thr Ser Arg Cys Phe Val Arg Asn Ile Gly Asp Pro Ala Val Ala Ala
945 950 955 960

Leu Ala Asp Leu Lys Arg Phe Ile Arg Ala Asp Leu Leu Asp Lys Gln
965 970 975

Val Leu Tyr Arg Val Met Asn Gln Glu Pro Gly Asp Ser Ser Phe Leu
980 985 990

Asp Trp Ala Ser Asp Pro Tyr Ser Cys Asn Leu Pro His Ser Gln Ser
995 1000 1005

Ile Thr Thr Ile Ile Lys Asn Ile Thr Ala Arg Ser Val Leu Gln
1010 1015 1020

Glu Ser Pro Asn Pro Leu Leu Ser Gly Leu Phe Thr Glu Thr Ser
1025 1030 1035

Gly Glu Glu Asp Leu Asn Leu Ala Ser Phe Leu Met Asp Arg Lys
1040 1045 1050

Val Ile Leu Pro Arg Val Ala His Glu Ile Leu Gly Asn Ser Leu
1055 1060 1065

Thr Gly Val Arg Glu Ala Ile Ala Gly Met Leu Asp Thr Thr Lys
1070 1075 1080

Ser Leu Val Arg Ala Ser Val Arg Lys Gly Gly Leu Ser Tyr Gly
1085 1090 1095

```
Ile Leu  Arg Arg Leu Val Asn  Tyr Asp Leu Leu Gln  Tyr Glu Thr
    1100                 1105                 1110

Leu Thr  Arg Thr Leu Arg Lys  Pro Val Lys Asp Asn  Ile Glu Tyr
    1115                 1120                 1125

Glu Tyr  Met Cys Ser Val Glu  Leu Ala Val Gly Leu  Arg Gln Lys
    1130                 1135                 1140

Met Trp  Ile His Leu Thr Tyr  Gly Arg Pro Ile His  Gly Leu Glu
    1145                 1150                 1155

Thr Pro  Asp Pro Leu Glu Leu  Leu Arg Gly Ile Phe  Ile Glu Gly
    1160                 1165                 1170

Ser Glu  Val Cys Lys Leu Cys  Arg Ser Glu Gly Ala  Asp Pro Ile
    1175                 1180                 1185

Tyr Thr  Trp Phe Tyr Leu Pro  Asp Asn Ile Asp Leu  Asp Thr Leu
    1190                 1195                 1200

Thr Asn  Gly Cys Pro Ala Ile  Arg Ile Pro Tyr Phe  Gly Ser Ala
    1205                 1210                 1215

Thr Asp  Glu Arg Ser Glu Ala  Gln Leu Gly Tyr Val  Arg Asn Leu
    1220                 1225                 1230

Ser Lys  Pro Ala Lys Ala Ala  Ile Arg Ile Ala Met  Val Tyr Thr
    1235                 1240                 1245

Trp Ala  Tyr Gly Thr Asp Glu  Ile Ser Trp Met Glu  Ala Ala Leu
    1250                 1255                 1260

Ile Ala  Gln Thr Arg Ala Asn  Leu Ser Leu Glu Asn  Leu Lys Leu
    1265                 1270                 1275

Leu Thr  Pro Val Ser Thr Ser  Thr Asn Leu Ser His  Arg Leu Lys
    1280                 1285                 1290

Asp Thr  Ala Thr Gln Met Lys  Phe Ser Ser Ala Thr  Leu Val Arg
    1295                 1300                 1305

Ala Ser  Arg Phe Ile Thr Ile  Ser Asn Asp Asn Met  Ala Leu Lys
    1310                 1315                 1320
```

```
Glu Ala  Gly Glu Ser Lys Asp  Thr Asn Leu Val Tyr  Gln Gln Ile
    1325                 1330                 1335

Met Leu  Thr Gly Leu Ser Leu  Phe Glu Phe Asn Met  Arg Tyr Lys
    1340                 1345                 1350

Lys Gly  Ser Leu Gly Lys Pro  Leu Ile Leu His Leu  His Leu Asn
    1355                 1360                 1365

Asn Gly  Cys Cys Ile Met Glu  Ser Pro Gln Glu Ala  Asn Ile Pro
    1370                 1375                 1380

Pro Arg  Ser Thr Leu Asp Leu  Glu Ile Thr Gln Glu  Asn Asn Lys
    1385                 1390                 1395

Leu Ile  Tyr Asp Pro Asp Pro  Leu Lys Asp Val Asp  Leu Glu Leu
    1400                 1405                 1410

Phe Ser  Lys Val Arg Asp Val  Val His Thr Val Asp  Met Thr Tyr
    1415                 1420                 1425

Trp Ser  Asp Asp Glu Val Ile  Arg Ala Thr Ser Ile  Cys Thr Ala
    1430                 1435                 1440

Met Thr  Ile Ala Asp Thr Met  Ser Gln Leu Asp Arg  Asp Asn Leu
    1445                 1450                 1455

Lys Glu  Met Ile Ala Leu Val  Asn Asp Asp Asp Val  Asn Ser Leu
    1460                 1465                 1470

Ile Thr  Glu Phe Met Val Ile  Asp Val Pro Leu Phe  Cys Ser Thr
    1475                 1480                 1485

Phe Gly  Gly Ile Leu Val Asn  Gln Phe Ala Tyr Ser  Leu Tyr Gly
    1490                 1495                 1500

Leu Asn  Ile Arg Gly Arg Glu  Glu Ile Trp Gly His  Val Val Arg
    1505                 1510                 1515

Ile Leu  Lys Asp Thr Ser His  Ala Val Leu Lys Val  Leu Ser Asn
    1520                 1525                 1530

Ala Leu  Ser His Pro Lys Ile  Phe Lys Arg Phe Trp  Asn Ala Gly
    1535                 1540                 1545
```

Val Val Glu Pro Val Tyr Gly Pro Asn Leu Ser Asn Gln Asp Lys
1550 1555 1560

Ile Leu Leu Ala Leu Ser Val Cys Glu Tyr Ser Val Asp Leu Phe
1565 1570 1575

Met His Asp Trp Gln Gly Gly Val Pro Leu Glu Ile Phe Ile Cys
1580 1585 1590

Asp Asn Asp Pro Asp Val Ala Asp Met Arg Arg Ser Ser Phe Leu
1595 1600 1605

Ala Arg His Leu Ala Tyr Leu Cys Ser Leu Ala Glu Ile Ser Arg
1610 1615 1620

Asp Gly Pro Arg Leu Glu Ser Met Asn Ser Leu Glu Arg Leu Glu
1625 1630 1635

Ser Leu Lys Ser Tyr Leu Glu Leu Thr Phe Leu Asp Asp Pro Val
1640 1645 1650

Leu Arg Tyr Ser Gln Leu Thr Gly Leu Val Ile Lys Val Phe Pro
1655 1660 1665

Ser Thr Leu Thr Tyr Ile Arg Lys Ser Ser Ile Lys Val Leu Arg
1670 1675 1680

Thr Arg Gly Ile Gly Val Pro Glu Val Leu Glu Asp Trp Asp Pro
1685 1690 1695

Glu Ala Asp Asn Ala Leu Leu Asp Gly Ile Ala Ala Glu Ile Gln
1700 1705 1710

Gln Asn Ile Pro Leu Gly His Gln Thr Arg Ala Pro Phe Trp Gly
1715 1720 1725

Leu Arg Val Ser Lys Ser Gln Val Leu Arg Leu Arg Gly Tyr Lys
1730 1735 1740

Glu Ile Thr Arg Gly Glu Ile Gly Arg Ser Gly Val Gly Leu Thr
1745 1750 1755

Leu Pro Phe Asp Gly Arg Tyr Leu Ser His Gln Leu Arg Leu Phe
1760 1765 1770

Gly Ile Asn Ser Thr Ser Cys Leu Lys Ala Leu Glu Leu Thr Tyr
1775 1780 1785

Leu Leu Ser Pro Leu Val Asp Lys Asp Lys Asp Arg Leu Tyr Leu
1790 1795 1800

Gly Glu Gly Ala Gly Ala Met Leu Ser Cys Tyr Asp Ala Thr Leu
1805 1810 1815

Gly Pro Cys Ile Asn Tyr Tyr Asn Ser Gly Val Tyr Ser Cys Asp
1820 1825 1830

Val Asn Gly Gln Arg Glu Leu Asn Ile Tyr Pro Ala Glu Val Ala
1835 1840 1845

Leu Val Gly Lys Lys Leu Asn Asn Val Thr Ser Leu Gly Gln Arg
1850 1855 1860

Val Lys Val Leu Phe Asn Gly Asn Pro Gly Ser Thr Trp Ile Gly
1865 1870 1875

Asn Asp Glu Cys Glu Ala Leu Ile Trp Asn Glu Leu Gln Asn Ser
1880 1885 1890

Ser Ile Gly Leu Val His Cys Asp Met Glu Gly Gly Asp His Lys
1895 1900 1905

Asp Asp Gln Val Val Leu His Glu His Tyr Ser Val Ile Arg Ile
1910 1915 1920

Ala Tyr Leu Val Gly Asp Arg Asp Val Val Leu Ile Ser Lys Ile
1925 1930 1935

Ala Pro Arg Leu Gly Thr Asp Trp Thr Arg Gln Leu Ser Leu Tyr
1940 1945 1950

Leu Arg Tyr Trp Asp Glu Val Asn Leu Ile Val Leu Lys Thr Ser
1955 1960 1965

Asn Pro Ala Ser Thr Glu Met Tyr Leu Leu Ser Arg His Pro Lys
1970 1975 1980

Ser Asp Ile Ile Glu Asp Ser Lys Thr Val Leu Ala Ser Leu Leu
1985 1990 1995

```
Pro Leu  Ser Lys Glu Asp Ser  Ile Lys Ile Glu Lys  Trp Ile Leu
    2000                 2005                 2010

Ile Glu  Lys Ala Lys Ala His  Glu Trp Val Thr Arg  Glu Leu Arg
    2015                 2020                 2025

Glu Gly  Ser Ser Ser Ser Gly  Met Leu Arg Pro Tyr  His Gln Ala
    2030                 2035                 2040

Leu Gln  Thr Phe Gly Phe Glu  Pro Asn Leu Tyr Lys  Leu Ser Arg
    2045                 2050                 2055

Asp Phe  Leu Ser Thr Met Asn  Ile Ala Asp Thr His  Asn Cys Met
    2060                 2065                 2070

Ile Ala  Phe Asn Arg Val Leu  Lys Asp Thr Ile Phe  Glu Trp Ala
    2075                 2080                 2085

Arg Ile  Thr Glu Ser Asp Lys  Arg Leu Lys Leu Thr  Gly Lys Tyr
    2090                 2095                 2100

Asp Leu  Tyr Pro Val Arg Asp  Ser Gly Lys Leu Lys  Thr Ile Ser
    2105                 2110                 2115

Arg Arg  Leu Val Leu Ser Trp  Ile Ser Leu Ser Met  Ser Thr Arg
    2120                 2125                 2130

Leu Val  Thr Gly Ser Phe Pro  Asp Gln Lys Phe Glu  Ala Arg Leu
    2135                 2140                 2145

Gln Leu  Gly Ile Val Ser Leu  Ser Ser Arg Glu Ile  Arg Asn Leu
    2150                 2155                 2160

Arg Val  Ile Thr Lys Thr Leu  Leu Asp Arg Phe Glu  Asp Ile Ile
    2165                 2170                 2175

His Ser  Ile Thr Tyr Arg Phe  Leu Thr Lys Glu Ile  Lys Ile Leu
    2180                 2185                 2190

Met Lys  Ile Leu Gly Ala Val  Lys Met Phe Gly Ala  Arg Gln Asn
    2195                 2200                 2205

Glu Tyr  Thr Thr Val Ile Asp  Asp Gly Ser Leu Gly  Asp Ile Glu
    2210                 2215                 2220
```

```
Pro Tyr  Asp Ser Ser
    2225
```

<210>  2
<211>  6687
<212>  DNA
<213>  Sendai virus

<400>  2

```
atggatgggc aggagtcctc ccaaaaccct tctgacatac tctatccaga atgccacctg     60
aactctccca tagtcagggg gaagatagca cagttgcacg tcttgttaga tgtgaaccag    120
ccctacagac tgaaggacga cagcataata aatattacaa agcacaaaat taggaacgga    180
ggattgtccc cccgtcaaat taagatcagg tctctgggta aggctcttca acgcacaata    240
aaggatttag accgatacac gtttgaaccg tacccaacct actctcagga attacttagg    300
cttgatatac cagagatatg tgacaaaatc cgatccgtct tcgcggtctc ggatcggctg    360
accagggagt tatctagtgg gttccaggat ctttggttga atatcttcaa gcaactaggc    420
aatatagaag gaagagaggg gtacgatccg ttgcaggata tcggcaccat cccggagata    480
actgataagt acagcaggaa tagatggtat aggccattcc taacttggtt cagcatcaaa    540
tatgacatgc ggtggatgca gaagaccaga ccggggggac ccctcgatac ctctaattca    600
cataacctcc tagaatgcaa atcatacact ctagtaacat acggagatct tgtcatgata    660
ctgaacaagt tgacattgac agggtatatc ctaacccctg agctggtctt gatgtattgt    720
gatgttgtag aaggaaggtg gaatatgtct gctgcagggc atctagataa gaagtccatt    780
gggataacaa gcaaaggtga ggaattatgg gaactagtgg attccctctt ctcaagtctt    840
ggagaggaaa tatacaatgt catcgcacta ttggagcccc tatcacttgc tctcatacaa    900
ctaaatgatc ctgttatacc tctacgtggg gcatttatga ggcatgtgtt gacagagcta    960
cagactgttt taacaagtag agacgtgtac acagatgctg aagcagacac tattgtggag   1020
tcgttactcg ccattttcca tggaacctct attgatgaga aagcagagat cttttccttc   1080
tttaggacat ttggccaccc cagcttagag gctgtcactg ccgccgacaa ggtaagggcc   1140
catatgtatg cacaaaaggc aataaagctt aagaccctat acgagtgtca tgcagttttt   1200
tgcactatca tcataaatgg gtatagagag aggcatggcg gacagtggcc cccctgtgac   1260
ttccctgatc acgtgtgtct agaactaagg aacgctcaag ggtccaatac ggcaatctct   1320
tatgaatgtg ctgtagacaa ctatacaagt ttcataggct tcaagtttcg gaagtttata   1380
gaaccacaac tagatgaaga tctcacaata tatatgaaag acaaagcact atcccccagg   1440
aaggaggcat gggactctgt atacccggat agtaatctgt actataaagc cccagagtct   1500
```

```
gaagagaccc ggcggcttat tgaagtgttc ataaatgatg agaatttcaa cccagaagaa   1560
attatcaatt atgtggagtc aggagattgg ttgaaagacg aggagttcaa catctcgtac   1620
agtctcaaag agaaagagat caagcaagag ggtcgtctat tcgcaaaaat gacttataag   1680
atgcgagccg tacaggtgct ggcagagaca ctactggcta aggaatagg agagctattc   1740
agcgaaaatg ggatggttaa aggagagata gacctactta aaagattgac tactctttct   1800
gtctcaggcg tccccaggac tgattcagtg tacaataact ctaaatcatc agagaagaga   1860
aacgaaggca tggaaaataa gaactctggg gggtactggg acgaaaagaa gaggtccaga   1920
catgaattca aggcaacaga ttcatcaaca gacggctatg aaacgttaag ttgcttcctc   1980
acaacagacc tcaagaaata ctgcttaaac tggagatttg agagtactgc attgtttggt   2040
cagagatgca acgagatatt tggcttcaag accttcttta actggatgca tccagtcctt   2100
gaaaggtgta caatatatgt tggagatcct tactgtccag tcgccgaccg gatgcatcga   2160
caactccagg atcatgcaga ctctggcatt ttcatacata atcctagggg gggcatagaa   2220
ggttactgcc agaagctgtg gaccttaatc tcaatcagtg caatccacct agcagctgtg   2280
agagtgggtg tcagggtctc tgcaatggtt cagggtgaca atcaagctat agccgtgaca   2340
tcaagagtac ctgtagctca gacttacaag cagaagaaaa atcatgtcta tgaggagatc   2400
accaaatatt tcggtgctct aagacacgtc atgtttgatg tagggcacga gctaaaattg   2460
aacgagacca tcattagtag caagatgttt gtctatagta aaaggatata ctatgatggg   2520
aagattttac cacagtgcct gaaagccttg accaagtgtg tattctggtc cgagacactg   2580
gtagatgaaa acagatctgc ttgttcgaac atctcaacat ccatagcaaa agctatcgaa   2640
aatgggtatt ctcctatact aggctactgc attgcgttgt ataagacctg tcagcaggtg   2700
tgcatatcac tagggatgac tataaatcca actatcagcc cgaccgtaag agatcaatac   2760
tttaagggta agaattggct gagatgtgca gtgttgattc cagcaaatgt tggaggattc   2820
aactacatgt ctacatctag atgctttgtt agaaatattg gagaccccgc agtagcagcc   2880
ctagctgatc tcaaaagatt catcagagcg gatctgttag acaagcaggt attatacagg   2940
gtcatgaatc aagaacccgg tgactctagt tttctagatt gggcttcaga cccttattcg   3000
tgtaacctcc cgcattctca gagtataact acgattataa agaatatcac tgctagatct   3060
gtgctgcagg aatccccgaa tcctctactg tctggtctct tcaccgagac tagtggagaa   3120
gaggatctca acctggcctc gttccttatg gaccggaaag tcatcctgcc gagagtggct   3180
catgagatcc tgggtaattc cttaactgga gttagggagg cgattgcagg gatgcttgat   3240
acgaccaagt ctctagtgag agccagcgtt aggaaaggag gattatcata tgggatattg   3300
```

aggaggcttg tcaattatga tctattgcag tacgagacac tgactagaac tctcaggaaa 3360
ccggtgaaag acaacatcga atatgagtat atgtgttcag ttgagctagc tgtcggtcta 3420
aggcagaaaa tgtggatcca cctgacttac gggagaccca tacatgggct agaaacacca 3480
gaccctttag agctcttgag gggaatattt atcgaaggtt cagaggtgtg caagctttgc 3540
aggtctgaag gagcagaccc catctataca tggttctatc ttcctgacaa tatagacctg 3600
gacacgctta caaacggatg tccggctata agaatcccct attttggatc agccactgat 3660
gaaaggtcgg aagcccaact cgggtatgta agaaatctaa gcaaacccgc aaaggcggcc 3720
atccggatag ctatggtgta tacgtgggcc tacgggactg atgagatatc gtggatggaa 3780
gccgctctta tagcccaaac aagagctaat ctgagcttag agaatctaaa gctgctgact 3840
cctgtttcaa cctccactaa tctatctcat aggttgaaag atacggcaac ccagatgaag 3900
ttctctagtg caacactagt ccgtgcaagt cggttcataa caatatcaaa tgataacatg 3960
gcactcaaag aagcagggga gtcgaaggat actaatctcg tgtatcagca gattatgcta 4020
actgggctaa gcttgttcga gttcaatatg agatataaga aaggttcctt agggaagcca 4080
ctgatattgc acttacatct taataacggg tgctgtataa tggagtcccc acaggaggcg 4140
aatatccccc caaggtccac attagattta gagattacac aagagaacaa taaattgatc 4200
tatgatcctg atccactcaa ggatgtggac cttgagctat ttagcaaggt cagagatgtt 4260
gtacacacag ttgacatgac ttattggtca gatgatgaag ttatcagagc aaccagtatc 4320
tgtactgcaa tgacgatagc tgatacaatg tctcaattag atagagacaa cttaaaagag 4380
atgatcgcac tagtaaatga cgatgatgtc aacagcttga ttactgagtt tatggtgatt 4440
gatgttcctt tattttgctc aacgttcggg ggtattctag tcaatcagtt tgcatactca 4500
ctctacggct taaacatcag aggaagggaa gaaatatggg gacatgtagt ccggattctt 4560
aaagatacct cccacgcagt tttaaaagtc ttatctaatg ctctatctca tcccaaaatc 4620
ttcaaacgat tctggaatgc aggtgtcgtg gaacctgtgt atgggcctaa cctctcaaat 4680
caggataaga tactcttggc cctctctgtc tgtgaatatt ctgtggatct attcatgcac 4740
gattggcaag gggtgtaccc gcttgagatc tttatctgtg acaatgaccc agatgtggcc 4800
gacatgagga ggtcctcttt cttggcaaga catcttgcat acctatgcag cttggcagag 4860
atatctaggg atgggccaag attagaatca atgaactctc tagagaggct cgagtcacta 4920
aagagttacc tggaactcac atttcttgat gacccggtac tgaggtacag tcagttgact 4980
ggcctagtca tcaaagtatt cccatctact ttgacctata tccggaagtc atctataaaa 5040
gtgttaagga caagaggtat aggagtccct gaagtcttag aagattggga tcccgaggca 5100

gataatgcac tgttagatgg tatcgcggca gaaatacaac agaatattcc tttgggacat 5160

cagactagag cccctttttg ggggttgaga gtatccaagt cacaggtact gcgtctccgg 5220

gggtacaagg agatcacaag aggtgagata ggcagatcag gtgttggtct gacgttacca 5280

ttcgatggaa gatatctatc tcaccagctg aggctctttg gcatcaacag tactagctgc 5340

ttgaaagcac ttgaacttac ctacctattg agccccttag ttgacaagga taaagatagg 5400

ctatatttag ggaaggagc tggggccatg ctttcctgtt atgacgctac tcttggccca 5460

tgcatcaact attataactc aggggtatac tcttgtgatg tcaatgggca gagagagtta 5520

aatatatatc ctgctgaggt ggcactagtg ggaaagaaat taaacaatgt tactagtctg 5580

ggtcaaagag ttaaagtgtt attcaacggg aatcctggct cgacatggat tgggaatgat 5640

gagtgtgagg ctttgatttg gaatgaatta cagaatagct cgataggcct agtccactgt 5700

gacatggagg gaggagatca taaggatgat caagttgtac tgcatgagca ttacagtgta 5760

atccggatcg cgtatctggt gggggatcga gacgttgtgc ttataagcaa gattgctccc 5820

aggctgggca cggattggac caggcagctc agcctatatc tgagatactg ggacgaggtt 5880

aacctaatag tgcttaaaac atctaaccct gcttccacag agatgtatct cctatcgagg 5940

caccccaaat ctgacattat agaggacagc aagacagtgt tagctagtct cctccctttg 6000

tcaaaagaag atagcatcaa gatagaaaag tggatcttaa tagagaaggc aaaggctcac 6060

gaatgggtta ctcgggaatt gagagaagga agctcttcat cagggatgct tagaccttac 6120

catcaagcac tgcagacgtt tggctttgaa ccaaacttgt ataaattgag cagagatttc 6180

ttgtccacca tgaacatagc tgatacacac aactgcatga tagctttcaa cagggttttg 6240

aaggatacaa tcttcgaatg ggctagaata actgagtcag ataaaaggct taaactaact 6300

ggtaagtatg acctgtatcc tgtgagagat tcaggcaagt tgaagacaat ttctagaaga 6360

cttgtgctat cttggatatc tttatctatg tccacaagat tggtaactgg gtcattccct 6420

gaccagaagt ttgaagcaag acttcaattg ggaatagttt cattatcatc ccgtgaaatc 6480

aggaacctga gggttatcac aaaaacttta ttagacaggt ttgaggatat tatacatagt 6540

ataacgtata gattcctcac caaagaaata aagattttga tgaagatttt aggggcagtc 6600

aagatgttcg gggccaggca aaatgaatac acgaccgtga ttgatgatgg atcactaggt 6660

gatatcgagc catatgacag ctcgtaa 6687

<210> 3
<211> 22
<212> DNA
<213> Artificial sequence

<220>
<221> source
<223> /note= "Description of artificial sequence: An artificially synthesized primer sequence"

<400> 3
atcactgcta gatctgtgct gc 22

<210> 4
<211> 22
<212> DNA
<213> Artificial sequence

<220>
<221> source
<223> /note= "Description of artificial sequence: An artificially synthesized primer sequence"

<400> 4
ggactcctat acctcttgtc ct 22

<210> 5
<211> 37
<212> DNA
<213> Artificial sequence

<220>
<221> source
<223> /note= "Description of artificial sequence: An artificially synthesized primer sequence"

<400> 5
gctataagcc tccccttttt tggatcagcc actgatg 37

<210> 6
<211> 37
<212> DNA
<213> Artificial sequence

<220>
<221> source
<223> /note= "Description of artificial sequence: An artificially synthesized primer sequence"

<400> 6
catcagtggc tgatccaaaa aaggggattc ttatagc 37

<210> 7
<211> 35
<212> DNA
<213> Artificial sequence

<220>
<221> source

<223> /note= "Description of artificial sequence: An artificially synthesized primer sequence"

<400> 7
cagatgtggc cgacttgagg aggtcctctt tcttg 35

<210> 8
<211> 35
<212> DNA
<213> Artificial sequence

<220>
<221> source
<223> /note= "Description of artificial sequence: An artificially synthesized primer sequence"

<400> 8
caagaaagag gacctcctca agtcggccac atctg 35

<210> 9
<211> 21
<212> DNA
<213> Artificial sequence

<220>
<221> source
<223> /note= "Description of artificial sequence: An artificially synthesized primer sequence"

<400> 9
caagagaaaa aacatgtatg g 21

<210> 10
<211> 22
<212> DNA
<213> Artificial sequence

<220>
<221> source
<223> /note= "Description of artificial sequence: An artificially synthesized primer sequence"

<400> 10
agagtttaag agatatgtag cc 22

<210> 11
<211> 23
<212> DNA
<213> Artificial sequence

<220>
<221> source
<223> /note= "Description of artificial sequence: An artificially synthesized primer sequence"

<400> 11

cctccactaa tctatctcat agg 23

<210> 12
<211> 23
<212> DNA
<213> Artificial sequence

<220>
<221> source
<223> /note= "Description of artificial sequence: An artificially synthesized primer sequence"

<400> 12
ataacaatac ttggctgaac gtg 23

<210> 13
<211> 18
<212> DNA
<213> Artificial sequence

<220>
<221> source
<223> /note= "Description of artificial sequence: An artificially synthesized primer sequence"

<400> 13
cggattggcc tgaactgc 18

<210> 14
<211> 18
<212> DNA
<213> Artificial sequence

<220>
<221> source
<223> /note= "Description of artificial sequence: An artificially synthesized primer sequence"

<400> 14
aacaggcggc agtaaggc 18

SEQUENCE LISTING

<110> DNAVEC CORPORATION

<120> Attenuated negative strand RNA virus

<130> D4-A0701P

<150> JP 2007-027520
<151> 2007-02-07

<160> 14

<170> PatentIn version 3.3

<210> 1
<211> 2228
<212> PRT
<213> Sendai virus

<400> 1

```
Met Asp Gly Gln Glu Ser Ser Gln Asn Pro Ser Asp Ile Leu Tyr Pro
1               5                   10                  15

Glu Cys His Leu Asn Ser Pro Ile Val Arg Gly Lys Ile Ala Gln Leu
            20                  25                  30

His Val Leu Leu Asp Val Asn Gln Pro Tyr Arg Leu Lys Asp Asp Ser
        35                  40                  45

Ile Ile Asn Ile Thr Lys His Lys Ile Arg Asn Gly Gly Leu Ser Pro
    50                  55                  60

Arg Gln Ile Lys Ile Arg Ser Leu Gly Lys Ala Leu Gln Arg Thr Ile
65                  70                  75                  80

Lys Asp Leu Asp Arg Tyr Thr Phe Glu Pro Tyr Pro Thr Tyr Ser Gln
                85                  90                  95

Glu Leu Leu Arg Leu Asp Ile Pro Glu Ile Cys Asp Lys Ile Arg Ser
            100                 105                 110

Val Phe Ala Val Ser Asp Arg Leu Thr Arg Glu Leu Ser Ser Gly Phe
        115                 120                 125

Gln Asp Leu Trp Leu Asn Ile Phe Lys Gln Leu Gly Asn Ile Glu Gly
    130                 135                 140

Arg Glu Gly Tyr Asp Pro Leu Gln Asp Ile Gly Thr Ile Pro Glu Ile
145                 150                 155                 160

Thr Asp Lys Tyr Ser Arg Asn Arg Trp Tyr Arg Pro Phe Leu Thr Trp
                165                 170                 175

Phe Ser Ile Lys Tyr Asp Met Arg Trp Met Gln Lys Thr Arg Pro Gly
            180                 185                 190
```

Gly Pro Leu Asp Thr Ser Asn Ser His Asn Leu Leu Glu Cys Lys Ser
195 200 205

Tyr Thr Leu Val Thr Tyr Gly Asp Leu Val Met Ile Leu Asn Lys Leu
210 215 220

Thr Leu Thr Gly Tyr Ile Leu Thr Pro Glu Leu Val Leu Met Tyr Cys
225 230 235 240

Asp Val Val Glu Gly Arg Trp Asn Met Ser Ala Ala Gly His Leu Asp
245 250 255

Lys Lys Ser Ile Gly Ile Thr Ser Lys Gly Glu Glu Leu Trp Glu Leu
260 265 270

Val Asp Ser Leu Phe Ser Ser Leu Gly Glu Glu Ile Tyr Asn Val Ile
275 280 285

Ala Leu Leu Glu Pro Leu Ser Leu Ala Leu Ile Gln Leu Asn Asp Pro
290 295 300

Val Ile Pro Leu Arg Gly Ala Phe Met Arg His Val Leu Thr Glu Leu
305 310 315 320

Gln Thr Val Leu Thr Ser Arg Asp Val Tyr Thr Asp Ala Glu Ala Asp
325 330 335

Thr Ile Val Glu Ser Leu Leu Ala Ile Phe His Gly Thr Ser Ile Asp
340 345 350

Glu Lys Ala Glu Ile Phe Ser Phe Phe Arg Thr Phe Gly His Pro Ser
355 360 365

Leu Glu Ala Val Thr Ala Ala Asp Lys Val Arg Ala His Met Tyr Ala
370 375 380

Gln Lys Ala Ile Lys Leu Lys Thr Leu Tyr Glu Cys His Ala Val Phe
385 390 395 400

Cys Thr Ile Ile Ile Asn Gly Tyr Arg Glu Arg His Gly Gly Gln Trp
405 410 415

Pro Pro Cys Asp Phe Pro Asp His Val Cys Leu Glu Leu Arg Asn Ala
420 425 430

Gln Gly Ser Asn Thr Ala Ile Ser Tyr Glu Cys Ala Val Asp Asn Tyr
435 440 445

Thr Ser Phe Ile Gly Phe Lys Phe Arg Lys Phe Ile Glu Pro Gln Leu
450 455 460

Asp Glu Asp Leu Thr Ile Tyr Met Lys Asp Lys Ala Leu Ser Pro Arg
465 470 475 480

Lys Glu Ala Trp Asp Ser Val Tyr Pro Asp Ser Asn Leu Tyr Tyr Lys
485 490 495

Ala Pro Glu Ser Glu Glu Thr Arg Arg Leu Ile Glu Val Phe Ile Asn
500 505 510

Asp Glu Asn Phe Asn Pro Glu Glu Ile Ile Asn Tyr Val Glu Ser Gly
515 520 525

Asp Trp Leu Lys Asp Glu Glu Phe Asn Ile Ser Tyr Ser Leu Lys Glu
530 535 540

Lys Glu Ile Lys Gln Glu Gly Arg Leu Phe Ala Lys Met Thr Tyr Lys
545 550 555 560

Met Arg Ala Val Gln Val Leu Ala Glu Thr Leu Leu Ala Lys Gly Ile
565 570 575

Gly Glu Leu Phe Ser Glu Asn Gly Met Val Lys Gly Glu Ile Asp Leu
580 585 590

Leu Lys Arg Leu Thr Thr Leu Ser Val Ser Gly Val Pro Arg Thr Asp
595 600 605

Ser Val Tyr Asn Asn Ser Lys Ser Ser Glu Lys Arg Asn Glu Gly Met
610 615 620

Glu Asn Lys Asn Ser Gly Gly Tyr Trp Asp Glu Lys Lys Arg Ser Arg
625 630 635 640

His Glu Phe Lys Ala Thr Asp Ser Ser Thr Asp Gly Tyr Glu Thr Leu
645 650 655

Ser Cys Phe Leu Thr Thr Asp Leu Lys Lys Tyr Cys Leu Asn Trp Arg
660 665 670

Phe Glu Ser Thr Ala Leu Phe Gly Gln Arg Cys Asn Glu Ile Phe Gly
675 680 685

Phe Lys Thr Phe Phe Asn Trp Met His Pro Val Leu Glu Arg Cys Thr
690 695 700

Ile Tyr Val Gly Asp Pro Tyr Cys Pro Val Ala Asp Arg Met His Arg
705 710 715 720

Gln Leu Gln Asp His Ala Asp Ser Gly Ile Phe Ile His Asn Pro Arg
725 730 735

Gly Gly Ile Glu Gly Tyr Cys Gln Lys Leu Trp Thr Leu Ile Ser Ile

740 745 750

Ser Ala Ile His Leu Ala Ala Val Arg Val Gly Val Arg Val Ser Ala
755 760 765

Met Val Gln Gly Asp Asn Gln Ala Ile Ala Val Thr Ser Arg Val Pro
770 775 780

Val Ala Gln Thr Tyr Lys Gln Lys Lys Asn His Val Tyr Glu Glu Ile
785 790 795 800

Thr Lys Tyr Phe Gly Ala Leu Arg His Val Met Phe Asp Val Gly His
805 810 815

Glu Leu Lys Leu Asn Glu Thr Ile Ile Ser Ser Lys Met Phe Val Tyr
820 825 830

Ser Lys Arg Ile Tyr Tyr Asp Gly Lys Ile Leu Pro Gln Cys Leu Lys
835 840 845

Ala Leu Thr Lys Cys Val Phe Trp Ser Glu Thr Leu Val Asp Glu Asn
850 855 860

Arg Ser Ala Cys Ser Asn Ile Ser Thr Ser Ile Ala Lys Ala Ile Glu
865 870 875 880

Asn Gly Tyr Ser Pro Ile Leu Gly Tyr Cys Ile Ala Leu Tyr Lys Thr
885 890 895

Cys Gln Gln Val Cys Ile Ser Leu Gly Met Thr Ile Asn Pro Thr Ile
900 905 910

Ser Pro Thr Val Arg Asp Gln Tyr Phe Lys Gly Lys Asn Trp Leu Arg
915 920 925

Cys Ala Val Leu Ile Pro Ala Asn Val Gly Gly Phe Asn Tyr Met Ser
930 935 940

Thr Ser Arg Cys Phe Val Arg Asn Ile Gly Asp Pro Ala Val Ala Ala
945 950 955 960

Leu Ala Asp Leu Lys Arg Phe Ile Arg Ala Asp Leu Leu Asp Lys Gln
965 970 975

Val Leu Tyr Arg Val Met Asn Gln Glu Pro Gly Asp Ser Ser Phe Leu
980 985 990

Asp Trp Ala Ser Asp Pro Tyr Ser Cys Asn Leu Pro His Ser Gln Ser
995 1000 1005

Ile Thr Thr Ile Ile Lys Asn Ile Thr Ala Arg Ser Val Leu Gln
1010 1015 1020

Glu Ser Pro Asn Pro Leu Leu Ser Gly Leu Phe Thr Glu Thr Ser
1025 1030 1035

Gly Glu Glu Asp Leu Asn Leu Ala Ser Phe Leu Met Asp Arg Lys
1040 1045 1050

Val Ile Leu Pro Arg Val Ala His Glu Ile Leu Gly Asn Ser Leu
1055 1060 1065

Thr Gly Val Arg Glu Ala Ile Ala Gly Met Leu Asp Thr Thr Lys
1070 1075 1080

Ser Leu Val Arg Ala Ser Val Arg Lys Gly Gly Leu Ser Tyr Gly
1085 1090 1095

Ile Leu Arg Arg Leu Val Asn Tyr Asp Leu Leu Gln Tyr Glu Thr
1100 1105 1110

Leu Thr Arg Thr Leu Arg Lys Pro Val Lys Asp Asn Ile Glu Tyr
1115 1120 1125

Glu Tyr Met Cys Ser Val Glu Leu Ala Val Gly Leu Arg Gln Lys
1130 1135 1140

Met Trp Ile His Leu Thr Tyr Gly Arg Pro Ile His Gly Leu Glu
1145 1150 1155

Thr Pro Asp Pro Leu Glu Leu Leu Arg Gly Ile Phe Ile Glu Gly
1160 1165 1170

Ser Glu Val Cys Lys Leu Cys Arg Ser Glu Gly Ala Asp Pro Ile
1175 1180 1185

Tyr Thr Trp Phe Tyr Leu Pro Asp Asn Ile Asp Leu Asp Thr Leu
1190 1195 1200

Thr Asn Gly Cys Pro Ala Ile Arg Ile Pro Tyr Phe Gly Ser Ala
1205 1210 1215

Thr Asp Glu Arg Ser Glu Ala Gln Leu Gly Tyr Val Arg Asn Leu
1220 1225 1230

Ser Lys Pro Ala Lys Ala Ala Ile Arg Ile Ala Met Val Tyr Thr
1235 1240 1245

Trp Ala Tyr Gly Thr Asp Glu Ile Ser Trp Met Glu Ala Ala Leu
1250 1255 1260

Ile Ala Gln Thr Arg Ala Asn Leu Ser Leu Glu Asn Leu Lys Leu
1265 1270 1275

Leu Thr Pro Val Ser Thr Ser Thr Asn Leu Ser His Arg Leu Lys
1280 1285 1290

Asp Thr Ala Thr Gln Met Lys Phe Ser Ser Ala Thr Leu Val Arg
1295 1300 1305

Ala Ser Arg Phe Ile Thr Ile Ser Asn Asp Asn Met Ala Leu Lys
1310 1315 1320

Glu Ala Gly Glu Ser Lys Asp Thr Asn Leu Val Tyr Gln Gln Ile
1325 1330 1335

Met Leu Thr Gly Leu Ser Leu Phe Glu Phe Asn Met Arg Tyr Lys
1340 1345 1350

Lys Gly Ser Leu Gly Lys Pro Leu Ile Leu His Leu His Leu Asn
1355 1360 1365

Asn Gly Cys Cys Ile Met Glu Ser Pro Gln Glu Ala Asn Ile Pro
1370 1375 1380

Pro Arg Ser Thr Leu Asp Leu Glu Ile Thr Gln Glu Asn Asn Lys
1385 1390 1395

Leu Ile Tyr Asp Pro Asp Pro Leu Lys Asp Val Asp Leu Glu Leu
1400 1405 1410

Phe Ser Lys Val Arg Asp Val Val His Thr Val Asp Met Thr Tyr
1415 1420 1425

Trp Ser Asp Asp Glu Val Ile Arg Ala Thr Ser Ile Cys Thr Ala
1430 1435 1440

Met Thr Ile Ala Asp Thr Met Ser Gln Leu Asp Arg Asp Asn Leu
1445 1450 1455

Lys Glu Met Ile Ala Leu Val Asn Asp Asp Asp Val Asn Ser Leu
1460 1465 1470

Ile Thr Glu Phe Met Val Ile Asp Val Pro Leu Phe Cys Ser Thr
1475 1480 1485

Phe Gly Gly Ile Leu Val Asn Gln Phe Ala Tyr Ser Leu Tyr Gly
1490 1495 1500

Leu Asn Ile Arg Gly Arg Glu Glu Ile Trp Gly His Val Val Arg
1505 1510 1515

Ile Leu Lys Asp Thr Ser His Ala Val Leu Lys Val Leu Ser Asn
1520 1525 1530

Ala Leu Ser His Pro Lys Ile Phe Lys Arg Phe Trp Asn Ala Gly
1535 1540 1545

Val Val Glu Pro Val Tyr Gly Pro Asn Leu Ser Asn Gln Asp Lys
1550 1555 1560

Ile Leu Leu Ala Leu Ser Val Cys Glu Tyr Ser Val Asp Leu Phe
1565 1570 1575

Met His Asp Trp Gln Gly Gly Val Pro Leu Glu Ile Phe Ile Cys
1580 1585 1590

Asp Asn Asp Pro Asp Val Ala Asp Met Arg Arg Ser Ser Phe Leu
1595 1600 1605

Ala Arg His Leu Ala Tyr Leu Cys Ser Leu Ala Glu Ile Ser Arg
1610 1615 1620

Asp Gly Pro Arg Leu Glu Ser Met Asn Ser Leu Glu Arg Leu Glu
1625 1630 1635

Ser Leu Lys Ser Tyr Leu Glu Leu Thr Phe Leu Asp Asp Pro Val
1640 1645 1650

Leu Arg Tyr Ser Gln Leu Thr Gly Leu Val Ile Lys Val Phe Pro
1655 1660 1665

Ser Thr Leu Thr Tyr Ile Arg Lys Ser Ser Ile Lys Val Leu Arg
1670 1675 1680

Thr Arg Gly Ile Gly Val Pro Glu Val Leu Glu Asp Trp Asp Pro
1685 1690 1695

Glu Ala Asp Asn Ala Leu Leu Asp Gly Ile Ala Ala Glu Ile Gln
1700 1705 1710

Gln Asn Ile Pro Leu Gly His Gln Thr Arg Ala Pro Phe Trp Gly
1715 1720 1725

Leu Arg Val Ser Lys Ser Gln Val Leu Arg Leu Arg Gly Tyr Lys
1730 1735 1740

Glu Ile Thr Arg Gly Glu Ile Gly Arg Ser Gly Val Gly Leu Thr
1745 1750 1755

Leu Pro Phe Asp Gly Arg Tyr Leu Ser His Gln Leu Arg Leu Phe
1760 1765 1770

Gly Ile Asn Ser Thr Ser Cys Leu Lys Ala Leu Glu Leu Thr Tyr
1775 1780 1785

Leu Leu Ser Pro Leu Val Asp Lys Asp Lys Asp Arg Leu Tyr Leu

1790 1795 1800

Gly Glu Gly Ala Gly Ala Met Leu Ser Cys Tyr Asp Ala Thr Leu
1805 1810 1815

Gly Pro Cys Ile Asn Tyr Tyr Asn Ser Gly Val Tyr Ser Cys Asp
1820 1825 1830

Val Asn Gly Gln Arg Glu Leu Asn Ile Tyr Pro Ala Glu Val Ala
1835 1840 1845

Leu Val Gly Lys Lys Leu Asn Asn Val Thr Ser Leu Gly Gln Arg
1850 1855 1860

Val Lys Val Leu Phe Asn Gly Asn Pro Gly Ser Thr Trp Ile Gly
1865 1870 1875

Asn Asp Glu Cys Glu Ala Leu Ile Trp Asn Glu Leu Gln Asn Ser
1880 1885 1890

Ser Ile Gly Leu Val His Cys Asp Met Glu Gly Gly Asp His Lys
1895 1900 1905

Asp Asp Gln Val Val Leu His Glu His Tyr Ser Val Ile Arg Ile
1910 1915 1920

Ala Tyr Leu Val Gly Asp Arg Asp Val Val Leu Ile Ser Lys Ile
1925 1930 1935

Ala Pro Arg Leu Gly Thr Asp Trp Thr Arg Gln Leu Ser Leu Tyr
1940 1945 1950

Leu Arg Tyr Trp Asp Glu Val Asn Leu Ile Val Leu Lys Thr Ser
1955 1960 1965

Asn Pro Ala Ser Thr Glu Met Tyr Leu Leu Ser Arg His Pro Lys
1970 1975 1980

Ser Asp Ile Ile Glu Asp Ser Lys Thr Val Leu Ala Ser Leu Leu
1985 1990 1995

Pro Leu Ser Lys Glu Asp Ser Ile Lys Ile Glu Lys Trp Ile Leu
2000 2005 2010

Ile Glu Lys Ala Lys Ala His Glu Trp Val Thr Arg Glu Leu Arg
2015 2020 2025

Glu Gly Ser Ser Ser Ser Gly Met Leu Arg Pro Tyr His Gln Ala
2030 2035 2040

Leu Gln Thr Phe Gly Phe Glu Pro Asn Leu Tyr Lys Leu Ser Arg
2045 2050 2055

Asp Phe Leu Ser Thr Met Asn Ile Ala Asp Thr His Asn Cys Met
2060 2065 2070

Ile Ala Phe Asn Arg Val Leu Lys Asp Thr Ile Phe Glu Trp Ala
2075 2080 2085

Arg Ile Thr Glu Ser Asp Lys Arg Leu Lys Leu Thr Gly Lys Tyr
2090 2095 2100

Asp Leu Tyr Pro Val Arg Asp Ser Gly Lys Leu Lys Thr Ile Ser
2105 2110 2115

Arg Arg Leu Val Leu Ser Trp Ile Ser Leu Ser Met Ser Thr Arg
2120 2125 2130

Leu Val Thr Gly Ser Phe Pro Asp Gln Lys Phe Glu Ala Arg Leu
2135 2140 2145

Gln Leu Gly Ile Val Ser Leu Ser Ser Arg Glu Ile Arg Asn Leu
2150 2155 2160

Arg Val Ile Thr Lys Thr Leu Leu Asp Arg Phe Glu Asp Ile Ile
2165 2170 2175

His Ser Ile Thr Tyr Arg Phe Leu Thr Lys Glu Ile Lys Ile Leu
2180 2185 2190

Met Lys Ile Leu Gly Ala Val Lys Met Phe Gly Ala Arg Gln Asn
2195 2200 2205

Glu Tyr Thr Thr Val Ile Asp Asp Gly Ser Leu Gly Asp Ile Glu
2210 2215 2220

Pro Tyr Asp Ser Ser
2225

<210> 2
<211> 6687
<212> DNA
<213> Sendai virus

<400> 2
atggatgggc aggagtcctc ccaaaaccct tctgacatac tctatccaga atgccacctg 60

aactctccca tagtcagggg gaagatagca cagttgcacg tcttgttaga tgtgaaccag 120

ccctacagac tgaaggacga cagcataata aatattacaa agcacaaaat taggaacgga 180

ggattgtccc cccgtcaaat taagatcagg tctctgggta aggctcttca acgcacaata 240

aaggatttag accgatacac gtttgaaccg tacccaacct actctcagga attacttagg 300

cttgatatac cagagatatg tgacaaaatc cgatccgtct tcgcggtctc ggatcggctg 360

accagggagt tatctagtgg gttccaggat ctttggttga atatcttcaa gcaactaggc 420

aatatagaag gaagagaggg gtacgatccg ttgcaggata tcggcaccat cccggagata 480
actgataagt acagcaggaa tagatggtat aggccattcc taacttggtt cagcatcaaa 540
tatgacatgc ggtggatgca gaagaccaga ccggggggac ccctcgatac ctctaattca 600
cataacctcc tagaatgcaa atcatacact ctagtaacat acggagatct tgtcatgata 660
ctgaacaagt tgacattgac agggtatatc ctaacccctg agctggtctt gatgtattgt 720
gatgttgtag aaggaaggtg gaatatgtct gctgcagggc atctagataa gaagtccatt 780
gggataacaa gcaaaggtga ggaattatgg gaactagtgg attccctctt ctcaagtctt 840
ggagaggaaa tatacaatgt catcgcacta ttggagcccc tatcacttgc tctcatacaa 900
ctaaatgatc ctgttatacc tctacgtggg gcatttatga ggcatgtgtt gacagagcta 960
cagactgttt taacaagtag agacgtgtac acagatgctg aagcagacac tattgtggag 1020
tcgttactcg ccattttcca tggaacctct attgatgaga aagcagagat cttttccttc 1080
tttaggacat ttggccaccc cagcttagag gctgtcactg ccgccgacaa ggtaagggcc 1140
catatgtatg cacaaaaggc aataaagctt aagaccctat acgagtgtca tgcagttttt 1200
tgcactatca tcataaatgg gtatagagag aggcatggcg gacagtggcc cccctgtgac 1260
ttccctgatc acgtgtgtct agaactaagg aacgctcaag ggtccaatac ggcaatctct 1320
tatgaatgtg ctgtagacaa ctatacaagt ttcataggct tcaagtttcg gaagtttata 1380
gaaccacaac tagatgaaga tctcacaata tatatgaaag acaaagcact atcccccagg 1440
aaggaggcat gggactctgt atacccggat agtaatctgt actataaagc cccagagtct 1500
gaagagaccc ggcggcttat tgaagtgttc ataaatgatg agaatttcaa cccagaagaa 1560
attatcaatt atgtggagtc aggagattgg ttgaaagacg aggagttcaa catctcgtac 1620
agtctcaaag agaaagagat caagcaagag ggtcgtctat tcgcaaaaat gacttataag 1680
atgcgagccg tacaggtgct ggcagagaca ctactggcta aaggaatagg agagctattc 1740
agcgaaaatg ggatggttaa aggagagata gacctactta aaagattgac tactctttct 1800
gtctcaggcg tccccaggac tgattcagtg tacaataact ctaaatcatc agagaagaga 1860
aacgaaggca tggaaaataa gaactctggg gggtactggg acgaaaagaa gaggtccaga 1920
catgaattca aggcaacaga ttcatcaaca gacggctatg aaacgttaag ttgcttcctc 1980
acaacagacc tcaagaaata ctgcttaaac tggagatttg agagtactgc attgtttggt 2040
cagagatgca acgagatatt tggcttcaag accttcttta actggatgca tccagtcctt 2100
gaaaggtgta caatatatgt tggagatcct tactgtccag tcgccgaccg gatgcatcga 2160
caactccagg atcatgcaga ctctggcatt ttcatacata atcctagggg gggcatagaa 2220
ggttactgcc agaagctgtg gaccttaatc tcaatcagtg caatccacct agcagctgtg 2280
agagtgggtg tcagggtctc tgcaatggtt cagggtgaca atcaagctat agccgtgaca 2340
tcaagagtac ctgtagctca gacttacaag cagaagaaaa atcatgtcta tgaggagatc 2400
accaaatatt tcggtgctct aagacacgtc atgtttgatg tagggcacga gctaaaattg 2460

aacgagacca tcattagtag caagatgttt gtctatagta aaaggatata ctatgatggg 2520
aagattttac cacagtgcct gaaagccttg accaagtgtg tattctggtc cgagacactg 2580
gtagatgaaa acagatctgc ttgttcgaac atctcaacat ccatagcaaa agctatcgaa 2640
aatgggtatt ctcctatact aggctactgc attgcgttgt ataagacctg tcagcaggtg 2700
tgcatatcac tagggatgac tataaatcca actatcagcc cgaccgtaag agatcaatac 2760
tttaagggta agaattggct gagatgtgca gtgttgattc cagcaaatgt tggaggattc 2820
aactacatgt ctacatctag atgctttgtt agaaatattg gagaccccgc agtagcagcc 2880
ctagctgatc tcaaaagatt catcagagcg gatctgttag acaagcaggt attatacagg 2940
gtcatgaatc aagaacccgg tgactctagt tttctagatt gggcttcaga cccttattcg 3000
tgtaacctcc cgcattctca gagtataact acgattataa agaatatcac tgctagatct 3060
gtgctgcagg aatccccgaa tcctctactg tctggtctct tcaccgagac tagtggagaa 3120
gaggatctca acctggcctc gttccttatg gaccggaaag tcatcctgcc gagagtggct 3180
catgagatcc tgggtaattc cttaactgga gttagggagg cgattgcagg gatgcttgat 3240
acgaccaagt ctctagtgag agccagcgtt aggaaaggag gattatcata tgggatattg 3300
aggaggcttg tcaattatga tctattgcag tacgagacac tgactagaac tctcaggaaa 3360
ccggtgaaag acaacatcga atatgagtat atgtgttcag ttgagctagc tgtcggtcta 3420
aggcagaaaa tgtggatcca cctgacttac gggagaccca tacatgggct agaaacacca 3480
gaccctttag agctcttgag gggaatattt atcgaaggtt cagaggtgtg caagctttgc 3540
aggtctgaag gagcagaccc catctataca tggttctatc ttcctgacaa tatagacctg 3600
gacacgctta caaacggatg tccggctata agaatcccct attttggatc agccactgat 3660
gaaaggtcgg aagcccaact cgggtatgta agaaatctaa gcaaacccgc aaaggcggcc 3720
atccggatag ctatggtgta tacgtgggcc tacgggactg atgagatatc gtggatggaa 3780
gccgctctta tagcccaaac aagagctaat ctgagcttag agaatctaaa gctgctgact 3840
cctgtttcaa cctccactaa tctatctcat aggttgaaag atacggcaac ccagatgaag 3900
ttctctagtg caacactagt ccgtgcaagt cggttcataa caatatcaaa tgataacatg 3960
gcactcaaag aagcagggga gtcgaaggat actaatctcg tgtatcagca gattatgcta 4020
actgggctaa gcttgttcga gttcaatatg agatataaga aaggttcctt agggaagcca 4080
ctgatattgc acttacatct taataacggg tgctgtataa tggagtcccc acaggaggcg 4140
aatatccccc caaggtccac attagattta gagattacac aagagaacaa taaattgatc 4200
tatgatcctg atccactcaa ggatgtggac cttgagctat ttagcaaggt cagagatgtt 4260
gtacacacag ttgacatgac ttattggtca gatgatgaag ttatcagagc aaccagtatc 4320
tgtactgcaa tgacgatagc tgatacaatg tctcaattag atagagacaa cttaaaagag 4380
atgatcgcac tagtaaatga cgatgatgtc aacagcttga ttactgagtt tatggtgatt 4440
gatgttcctt tattttgctc aacgttcggg ggtattctag tcaatcagtt tgcatactca 4500
ctctacggct taaacatcag aggaagggaa gaaatatggg gacatgtagt ccggattctt 4560

aaagat acct cccacgcagt t t t aaaagt c t t at ct aat g ct ct at ct ca t cccaaaat c 4620
t t caaacgat t ct ggaat gc aggt gt cgt g gaacct gt gt at gggcct aa cct ct caaat 4680
caggat aaga t act ct t ggc cct ct ct gt c t gt gaat at t ct gt ggat ct at t cat gcac 4740
gat t ggcaag ggggt gt acc gct t gagat c t t t at ct gt g acaat gaccc agat gt ggcc 4800
gacat gagga ggt cct ct t t ct t ggcaaga cat ct t gcat acct at gcag ct t ggcagag 4860
at at ct aggg at gggccaag at t agaat ca at gaact ct c t agagaggct cgagt cact a 4920
aagagt t acc t ggaact cac at t t ct t gat gacccggt ac t gaggt acag t cagt t gact 4980
ggcct agt ca t caaagt at t cccat ct act t t gacct at a t ccggaagt c at ct at aaaa 5040
gt gt t aagga caagaggt at aggagt ccct gaagt ct t ag aagat t ggga t cccgaggca 5100
gat aat gcac t gt t agat gg t at cgcggca gaaat acaac agaat at t cc t t t gggacat 5160
cagact agag cccct t t t t g ggggt t gaga gt at ccaagt cacaggt act gcgt ct ccgg 5220
gggt acaagg agat cacaag aggt gagat a ggcagat cag gt gt t ggt ct gacgt t acca 5280
t t cgat ggaa gat at ct at c t caccagct g aggct ct t t g gcat caacag t act agct gc 5340
t t gaaagcac t t gaact t ac ct acct at t g agcccct t ag t t gacaagga t aaagat agg 5400
ct at at t t ag gggaaggagc t ggggccat g ct t t cct gt t at gacgct ac t ct t ggccca 5460
t gcat caact at t at aact c aggggt at ac t ct t gt gat g t caat gggca gagagagt t a 5520
aat at at at c ct gct gaggt ggcact agt g ggaaagaaat t aaacaat gt t act agt ct g 5580
ggt caaagag t t aaagt gt t at t caacggg aat cct ggct cgacat ggat t gggaat gat 5640
gagt gt gagg ct t t gat t t g gaat gaat t a cagaat agct cgat aggcct agt ccact gt 5700
gacat ggagg gaggagat ca t aaggat gat caagt t gt ac t gcat gagca t t acagt gt a 5760
at ccggat cg cgt at ct ggt gggggat cga gacgt t gt gc t t at aagcaa gat t gct ccc 5820
aggct gggca cggat t ggac caggcagct c agcct at at c t gagat act g ggacgaggt t 5880
aacct aat ag t gct t aaaac at ct aaccct gct t ccacag agat gt at ct cct at cgagg 5940
caccccaaat ct gacat t at agaggacagc aagacagt gt t agct agt ct cct ccct t t g 6000
t caaaagaag at agcat caa gat agaaaag t ggat ct t aa t agagaaggc aaaggct cac 6060
gaat gggt t a ct cgggaat t gagagaagga agct ct t cat cagggat gct t agacct t ac 6120
cat caagcac t gcagacgt t t ggct t t gaa ccaaact t gt at aaat t gag cagagat t t c 6180
t t gt ccacca t gaacat agc t gat acacac aact gcat ga t agct t t caa cagggt t t t g 6240
aaggat acaa t ct t cgaat g ggct agaat a act gagt cag at aaaaggct t aaact aact 6300
ggt aagt at g acct gt at cc t gt gagagat t caggcaagt t gaagacaat t t ct agaaga 6360
ct t gt gct at ct t ggat at c t t t at ct at g t ccacaagat t ggt aact gg gt cat t ccct 6420
gaccagaagt t t gaagcaag act t caat t g ggaat agt t t cat t at cat c ccgt gaaat c 6480
aggaacct ga gggt t at cac aaaaact t t a t t agacaggt t t gaggat at t at acat agt 6540
at aacgt at a gat t cct cac caaagaaat a aagat t t t ga t gaagat t t t aggggcagt c 6600

aagatgttcg gggccaggca aaatgaatac acgaccgtga ttgatgatgg atcactaggt 6660

gatatcgagc catatgacag ctcgtaa 6687

<210> 3
<211> 22
<212> DNA
<213> Artificial

<220>
<223> An artificially synthesized primer sequence

<400> 3
atcactgcta gatctgtgct gc 22

<210> 4
<211> 22
<212> DNA
<213> Artificial

<220>
<223> An artificially synthesized primer sequence

<400> 4
ggactcctat acctcttgtc ct 22

<210> 5
<211> 37
<212> DNA
<213> Artificial

<220>
<223> An artificially synthesized primer sequence

<400> 5
gctataagcc tcccctttt tggatcagcc actgatg 37

<210> 6
<211> 37
<212> DNA
<213> Artificial

<220>
<223> An artificially synthesized primer sequence

<400> 6
catcagtggc tgatccaaaa aaggggattc ttatagc 37

<210> 7
<211> 35
<212> DNA
<213> Artificial

<220>
<223> An artificially synthesized primer sequence

<400> 7
cagatgtggc cgacttgagg aggtcctctt tcttg 35

<210> 8
<211> 35
<212> DNA
<213> Artificial

<220>
<223> An artificially synthesized primer sequence

<400> 8
caagaaagag gacctcctca agtcggccac atctg 35

<210> 9
<211> 21
<212> DNA
<213> Artificial

<220>
<223> An artificially synthesized primer sequence

<400> 9
caagagaaaa aacatgtatg g 21

<210> 10
<211> 22
<212> DNA
<213> Artificial

<220>
<223> An artificially synthesized primer sequence

<400> 10
agagtttaag agatatgtag cc 22

<210> 11
<211> 23
<212> DNA
<213> Artificial

<220>
<223> An artificially synthesized primer sequence

<400> 11
cctccactaa tctatctcat agg 23

<210> 12
<211> 23
<212> DNA
<213> Artificial

<220>
<223> An artificially synthesized primer sequence

<400> 12
ataacaatac ttggctgaac gtg 23

<210> 13
<211> 18
<212> DNA
<213> Artificial

<220>
<223> An artificially synthesized primer sequence

<400> 13
cggattggcc tgaactgc 18

<210> 14

```
<211>  18
<212>  DNA
<213>  Artificial

<220>
<223>  An artificially synthesized primer sequence

<400>  14
aacaggcggc agtaaggc                                            18
```